(19) Europäisches Patentamt
European Patent Office
Office européen des brevets

(11) Veröffentlichungsnummer: **0 192 255**
**B1**

(12) **EUROPÄISCHE PATENTSCHRIFT**

(45) Veröffentlichungstag der Patentschrift: **08.08.90**

(21) Anmeldenummer: **86102155.8**

(22) Anmeldetag: **19.02.86**

(51) Int. Cl.[5]: **C 07 D 207/273,**
C 07 D 207/38, A 61 K 31/40

(54) **Bi-2H-pyrroli(di)ndione.**

(30) Priorität: **22.02.85 CH 828/85**

(43) Veröffentlichungstag der Anmeldung:
**27.08.86 Patentblatt 86/35**

(45) Bekanntmachung des Hinweises auf die Patenterteilung:
**08.08.90 Patentblatt 90/32**

(84) Benannte Vertragsstaaten:
**AT BE CH DE FR GB IT LI LU NL SE**

(56) Entgegenhaltungen:
**FR-A-1 061 755**

(73) Patentinhaber: **CIBA-GEIGY AG**
**Klybeckstrasse 141**
**CH-4002 Basel (CH)**

(72) Erfinder: **Paioni, Romeo, Dr.**
**Wettsteinstrasse 6**
**CH-4125 Riehen (CH)**

(74) Vertreter: **Zumstein, Fritz, Dr. et al**
**Bräuhausstrasse 4**
**D-8000 München 2 (DE)**

**EP 0 192 255 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue substituierte Tetrahydro-, Hexahydro-bzw. Octahydro-[3,4'-bi-2H-pyrrol]-2,2'-dione der Formel

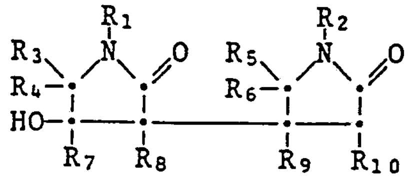

(I),

worin $R_1$ und $R_2$ unabhängig voneinander Carboxy-$C_1$—$C_4$-alkyl, Carbamyl-$C_1$—$C_4$-alkyl, N-$C_1$—$C_7$- oder N,N-di-$C_1$—$C_4$-alkylcarbamyl-$C_1$—$C_4$-alkyl, die Hydroxy-, Alkoxy-, Amino-, Alkylamino-, Dialkylamino-, Alkylen- bzw. Alkenylen- oder Aza-, Oxa- oder Thiaalkylylenaminogruppe(n) in höherer als der α-Stellung zum Carbamyl-Stickstoffatom tragendes N-Mono- oder N,N-Di(hydroxy-$C_2$—$C_4$-alkyl)carbamyl-$C_1$—$C_4$-alkyl, N-(—$C_1$—$C_4$-Alkoxy-$C_2$—$C_4$-alkyl)carbamyl-$C_1$—$C_4$-alkyl, N-Mono oder N,N-Di(amino-$C_2$—$C_4$-alkyl)-carbamyl-$C_1$—$C_4$-alkyl, N-(—$C_1$—$C_7$-Alkylamino-$C_2$—$C_4$-alkyl)- oder N-(Di-$C_2$—$C_4$-alkylamino-$C_2$—$C_4$-alkyl)-carbamyl-$C_1$—$C_4$-alkyl, N-(Alkylenamino-$C_2$—$C_4$-alkyl)carbamyl-$C_1$—$C_4$-alkyl, N-(Alkenylenamino-$C_2$—$C_4$-alkyl)carbamyl-$C_1$—$C_4$-alkyl, N-[(Aza)alkylenamino-$C_2$—$C_4$-alkyl]carbamyl-$C_1$—$C_4$-alkyl, N-[(Oxa)-alkylen-amino-$C_2$—$C_4$-alkyl]-carbamyl-$C_1$—$C_4$-alkyl bzw. N-[(Thia)alkylenamino-$C_2$—$C_4$-alkyl]-carbamyl-$C_1$—$C_4$-alkyl, wobei der Alkylenamino-, Alkenylenamino-, (Aza)alkylenamino-, (Oxa)alkylenamino- bzw. (Thia)alkylenaminorest einschliesslich der Aminogruppe 4 bis 8 Ringglieder enthält und Alkylenamino durch $C_1$—$C_4$-Alkoxygruppe sowie durch Oxo oder Hydroxy, Alkenylamino durch $C_1$—$C_4$-Alkoxycarbonyl und gegebenenfalls zusätzlich durch Hydroxy C-substituiert und (Aza)alkylenamino durch $C_1$—$C_4$-Alkyl C- oder N'- und/oder durch $C_1$—$C_7$-Alkanoyl N'-substituiert sein kann, N-(Carbamyl-$C_1$—$C_4$-alkyl)carbamyl-$C_1$—$C_4$-alkyl, N-(N'-$C_1$—$C_7$- oder N',N'-Di-$C_1$—$C_4$-alkylcarbamyl-$C_2$—$C_4$-alkyl)carbamyl-$C_1$—$C_4$-alkyl, 3-N-$C_3$—$C_8$-Cycloalkyl- oder N,N-Di-$C_3$—$C_8$-cycloalkylcarbamyl-$C_1$—$C_4$-alkyl, N-$C_7$—$C_9$-Bicycloalkylcarbamyl-$C_1$—$C_4$-alkyl, N-$C_8$—$C_{12}$-Tricycloalkylcarbamyl-$C_1$—$C_4$-alkyl, unsubstituiertes oder im Phenylteil durch $C_1$—$C_4$-Alkyl, $C_1$—$C_4$-Alkoxy, Halogen und/oder Trifluormethyl substituiertes N-Mono- oder N,N-Di(phenyl-$C_1$—$C_4$-alkyl)carbamyl-$C_1$—$C_4$-alkyl, N,N-Alkylencarbamyl-$C_1$—$C_4$-alkyl, N,N-Alkenylencarbamyl-$C_2$—$C_4$-alkyl, N,N-(Aza)alkylencarbamyl-$C_1$—$C_4$-alkyl oder N,N-(Oxa-oder Thia)alkylencarbamyl-$C_1$—$C_4$-alkyl bedeuten, wobei der Alkylenamino-, Alenylenamino-, (Aza)alkylenamino-, (Oxa)alkylenamino- bzw. (Thia)alkylenaminorest einschliesslich der Aminogruppe 4 bis 8 Ringglieder enthält und Alkyenamino durch $C_1$—$C_4$-Alkoxycarbonyl sowie durch Oxo oder Hydroxy, Alkenylenamino durch $C_1$—$C_4$-Alkoxy-carbonyl und gegebenenfalls zusätzlich durch Hydroxy C-substituiert und (Aza)alkylenamino durch $C_1$—$C_4$-Alkyl C- oder N'-und/oder durch $C_1$—$C_7$-Alkanoyl N'-substituiert sein kann, und $R_3$, $R_4$, $R_5$ und $R_6$ jeweils Wasserstoff oder $C_1$—$C_7$-Alkyl bedeuten oder $R_3$ und $R_4$ und/oder $R_5$ und $R_6$ gemeinsam einen $C_4$—$C_8$-Alkylenrest darstellen un d$R_7$, $R_8$, $R_9$ und $R_{10}$ jeweils für Wasserstoff stehen oder $R_7$ gemeinsam mit $R_8$ und/oder $R_9$ gemeinsam mit $R_{10}$ jeweils eine zusätzliche Bindung bedeuten, und ihre Salze.

Vor- und nachstehend sind unter niederen Resten beispielsweise solche zu verstehen, die bis und mit 7, insbesondere bis und mit 4, Kohlenstoffatome (C-Atome) aufweisen. Ferner gilt:

$C_1$—$C_7$-Alkyl ist beispielsweise $C_1$—$C_4$-Alkyl, wie Methyl, Ethyl, Propyl, Butyl, Isobutyl, Sekundärbutyl oder Tertiärbutyl, kann aber auch $C_4$—$C_7$-Alkyl, z.B. eine Phenyl-, Hexyl- oder Heptylgruppe sein.

Carboxy-$C_1$—$C_4$-alkyl und bedeutet z.B. Carboxymethyl, 2-Carboxyethyl, 3-Carboxypropyl oder 4-Carboxybutyl, kann aber auch 2-Carboxypropyl, 2-Carboxy-2-methyl-propyl oder 1-Carboxyethyl sein.

$C_1$—$C_4$-alkyl bedeutet z.B. Carbamylmethyl, 2-Carbamylethyl, 3-Carbamylpropyl oder 4-Carbamylbutyl, kann aber auch 2-Carbamylpropyl, 2-Carbamyl-2-methyl-propyl oder 1-Carbamylethyl sein.

Hydroxy-$C_2$—$C_4$-alkyl trägt die Hydroxygruppe insbesondere in höherer als der α-Stellung und bedeutet beispielsweise 2-Hydroxyethyl, 3- oder auch 2-Hydroxypropyl oder 4-Hydroxybutyl. Durch Hydroxyniederalkyl substituiertes Carbamylniederalkyl ist z.B. N-(2-Hydroxyethyl)carbamylmethyl.

$C_1$—$C_4$-Alkoxy-$C_2$—$C_4$-alkyl trägt die Alkoxygruppe insbesondere in höherer als der α-Stellung und bedeutet beispielsweise 2-Methoxyethyl, 3-Methoxypropyl, ferner 2-Ethoxyethyl.

Amino-$C_2$—$C_4$-alkyl bzw. N-$C_1$—$C_7$- oder N,N-Di-$C_1$—$C_4$-alkylaminoniederalkyl trägt die Amino- bzw. N-Mono- oder N,N-Dialkylaminogruppe insbesondere in höherer als der α-Stellung und bedeutet beispielsweise 2-Aminoethyl, 3-Aminopropyl, 2-(N-Isopropylamino)ethyl, 2-(N-Tertiärbutylamino)ethyl, 2-(N,N-Dimethylamino)ethyl, 2-(N,N-Diethylamino)ethyl oder insbesondere 2-(N,N-Diisopropylamino)ethyl.

N,N-Alkyen- bzw. N,N-Alkenylen- oder N,N-(Aza-, Oxa- oder Thia)alkylenaminoniederalkyl trägt die N,N-disubstituierte Aminogruppe insbesondere in höherer als der α-Stellung und bedeutet beispielsweise gegebenfalls durch $C_1$—$C_4$-Alkoxycarbonyl, z.B. Methoxy- oder Ethoxycarbonyl, und Oxo oder Hydroxy substituiertes 4- bis 6-gliedriges N,N-Alkylen-amino-$C_2$—$C_4$-alkyl bzw. gegebenenfalls durch $C_1$—$C_4$-Alkoxycarbonyl, z.B. Methoxy- oder Ethoxycarbonyl, und gegebenenfalls zusätzlich durch Hydroxy substituiertes N,N-Alkenylenamino-$C_2$—$C_4$-alkyl oder unsubstituertes N,N-(3-Aza)-, N,N-(3-Oxa)- oder N,N-(3-Thia)-alkenylenamino-$C_2$—$C_4$-alkyl, wie 2-(3,3-Dimethylazetidino)ethyl, 2-Pyrrolidinoethyl, 2-Piperidinoethyl, 2-(3-Ethoxycarbonyl-4-oxo-piperidino)ethyl, 2-(3-Methoxycarbonyl-4-oxo-piperidino)-ethyl), 2-(3-Ethoxycarbonyl-4-hydroxy-piperidino)ethyl, 2-(3-Methoxycarbonyl-4-hydroxy-piperidino)ethyl,

2-(3-Methoxycarbonyl-1,2,5,6-tetrahydropyridino)ethyl, 2-(2,6-Dimethyliperidino)ethyl, 2-Morpholinoethyl, 2-(4-Methylpiperazino)ethyl oder 2-(4-Acetylpiperazino)ethyl.

N-$C_1$—$C_7$- oder N,N-Di-$C_1$—$C_4$-alkyl-$C_1$—$C_4$-alkylcarbamyl ist beispielsweise N,N-Dimethylcarbamylmethyl, N-Ethylcarbamylmethyl, N,N-diethylcarbamylmethyl, N-(2,2-Dimethylpropyl)carbamylmethyl, N,N-Diisopropylcarbamylmethyl, 2-(N,N-Dimethylcarbamyl)ethyl oder 2-(N,N-Diispropylcarbamyl)ethyl.

$C_3$—$C_8$-Cycloalkyl, $C_7$—$C_9$-Bicycloalkyl bzw. $C_8$—$C_{12}$-Tricycloalkyl ist beispielsweise Cyclopropyl, Cyclobutyl, Cyclopentyl oder Cyclohexyl, ferner Cycloheptyl oder Cyclooctyl, Bicyclo-[2,2,2]-octyl, oder Adamantyl.

$C_1$—$C_4$-Alkoxy ist beispielsweise, wie Methoxy, Ethoxy, Propyloxy, Isopropyloxy, Butyloxy, Isobutyloxy, Sekundärbutyloxy oder Tertiärbutyloxy.

Halogen ist beispielsweise Halogen der Atomnummer bis und mit 35, wie Fluor, Chlor oder Brom.

Phenyl-$C_1$—$C_4$-alkyl ist beispielsweise Benzyl, 2-Phenylethyl, 2-(3,4-Dimethoxyphenyl)ethyl, oder 3-Phenylpropyl.

3- bis 7-gliedriges Alkylen bedeutet beispielsweise gegebenenfalls durch $C_1$—$C_4$-Alkoxycarbonyl, z.B. Methoxy- oder Ethoxycarbonyl, und Oxo oder Hydroxy substituiertes 3- bis 7-gliedriges Alkylen, wie 1,3-Propylen, 1,3-(2,2-Dimethyl)propylen, 1,4-Butylen, 1,5-Pentylen, 1,5-(2-Methoxy- oder Ethoxycarbonyl-3-oxo)pentylen, 1,5-(2-Methoxy- oder 2-Ethoxycarbonyl-3-hydroxy)pentylen oder 1,6-Heptylen. Durch 3- bis 7-gliedriges Alkylen substituiertes Carbamylniederalkyl bedeutet somit z.B. Piperidinocarbonylniederalkyl oder (3-Methoxycarbonyl-4-oxo-piperidinocarbonyl)methyl, kann aber auch Pyrrolidinocarbonylmethyl sein.

3- bis 7-gliedriges Alkenylen bedeutet beispielsweise 1,5-(2-$C_1$—$C_4$-Alkoxycarbonyl)pent-2-enylen oder 1,5-(2-$C_1$—$C_4$-Alkoxycarbonyl-3-hydroxy)pent-2-enylen, entsprechend substituiertes Carbamyl-$C_1$—$C_4$-alkyl- z.B. 3-$C_1$—$C_4$-Alkoxycarbonyl-, wie 3-Methoxycarbonyl-1,2,5,6-tetrahydropyridylinocarbonylmethyl- oder 3-$C_1$—$C_4$-Alkoxycarbonyl-, wie 3-Methoxycarbonyl-4-hydroxy-1,2,5,6-tetrahydro-pyridinocarbonylmethyl.

3- bis 7-gliedriges Aza-, Oxa- oder Thiaalkylen weist insbesondere 5 oder 6 Kettenglieder auf bedeutet beispielsweise 1,5-(3-Aza)pentylen, 1,5-(3-Methyl-3-aza)pentylen, 1,5-(3-Oxa)pentylen oder 1,5-(3-Thia)pentylen. Durch 3- bis 7-gliedriges Aza-, Oxa- oder Thiaalkylen substituiertes Carbamyl-$C_1$—$C_4$-alkyl bedeutet z.B. Morpholino-, (4-Methyl)piperazino-, (4-Acetyl)piperazino-, oder (cis-2,6-Dimethyl)piperazinocarbonylmethyl.

Die Verbindungen der Formel I können je nach Anzahl asymmetrischer C-Atome als Stereoisomere, wie Enantiomere oder Diastereomere, oder als Stereoisomerengemische, wie Enantiomerengemische, Diastereomerengemische, Racemate oder Racematgemische auftreten. Diese entsprechen sämtlich der Formel I und sind dementsprechend in der Erfindung inbegriffen.

Salze von Verbindungen der Formel I sind beispielsweise deren pharmazeutisch verwendbare Salze mit Basen, ebenso innere Salze und pharmazeutisch verwendbare Säureaditionssalze von Verbindungen der Formel I, in denen $R_1$ und/oder $R_2$ basichen Charakter besitzen, d.h. in denen die Carbamylgruppe durch basische Reste, wie Aminoniederalkyl, N-Mono- bzw. N,N-Diniederalkylaminoniederalkyl oder N,N-Alkylen- bzw. N,N-(Aza-, Oxa- oder Thia)alkylenaminoniederalkyl, substituiert ist.

Pharmazeutisch verwendbare Salze von Verbindungen der Formel I mit Basen sind beispielsweise Metall- oder Ammoniumsalze, wie Alkalimetall- und Erdalkalimetall-, z.B. Natrium-, Kalium-, Magnesium- oder Kalziumsalze, ebenso Ammoniumsalze mit Ammoniak oder geeigneten organischen Aminen, wie Salze mit Niederalkyl und/oder Hydroxyniederalkyl aufweisenden aliphatischen Aminen, wie N-Hydroxyniederalkylaminen, z.B. Ethanolamin, N-Hydroxyniederalkyl-N,N-di(niederalkyl)-aminen, z.B. 2,2-Dimethylaminoethanol, N,N-Di(hydroxyniederalkyl)aminen, z.B. Diethanolamin, oder N,N,N-Tri(hydroxyniederalkyl)aminen, z.B. Triethanolamin, ebenso Salze mit N-Tri(hydroxymethyl)-methylamin, ferner Salze mit basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylamin, z.B. N,N'-Dibenzylethylendiamin, Dibenzylamin oder N-Benzyl-β-phenylethylamin.

Pharmazeutisch verwendbare Säureadditionssalze sind beispielsweise Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten organischen Carbon- oder Sulfonsäuren, wie gegebenenfalls hydroxylierten aliphatischen Mono- oder Dicarbonsäuren, z.B. Acetate, Oxalate, Succinate, Fumarate, Maleinate, Maleate, Ascorbinate oder Citrate, der aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate.

Zur Isolierung oder Reinigung können auch pharmazeutisch ungegeignete Salze Verwendung finden. Zur therapeutischen Anwendung gelangen nur die pharmazeutisch verwendbaren, nicht-toxischen Salze, die deshalb bevorzugt sind.

Die Verbindung der Formel I und ihre pharmazeutisch verwendbaren Salze besitzen wertvolle pharmakologische, insbesondere nootrope Eigenschaften. So vermindern sie an der Maus in Dosen ab etwa 0,1 mg/kg i.p. sowie p.o. die amnesiogene Wirkung eines Elektroschocks in mindestens gleichem Ausmass wie nach Verabfolgung einer nootrop wirksamen Dosis von Piracetam (100 mg/kg i.p.). Zum Nachweis der nootropen Wirkung kann beispielsweise der Two-Compartment-Test herangezogen werden.

Als Literatur über pharmakologische Modelle dieser Art seien z.B. genannt: S. J. Sara und D. Lefevre, Psychopharmacologia *25*, 32—40 (1972), Hypoxia-induced amnesia in one-trial learning and pharmacological protection by piracetam. Boggan, W. O., und Schlesinger, K., in Behavioral Biology *12*, 127—134 (1974).

Im weiteren zeigen die Verbindungen der Formel I eine starke gedächnisverbessernde Wirkung im Step-down Passive Avoidance Test nach Mondadori und Wasser, Psychopharmacology *63*, 297—300 (1979). Die Substanzen sind wirksam bei intraperitonelaer Gabe 30 Minuten vor dem Lernversuch (wirksame Dosen 0,1, 1,0, 10 mg/kg). Eine deutliche Wirkung war auch feststellbar bei peroraler Applikation 60 Minuten vor dem Lernversuch (wirksame Dosen 0,1, 1, 10 mg/kg) sowie bei intraperitonealer Applikation unmittelbar nach dem Lernversuch (wirksame Dosen 0,1, 1, 10 mg/kg).

Die Verbindungen der Formel I und ihre pharmazeutisch verwendbaren Salze können dementsprechend als Nootropika, beispeilsweise zur Behandlung zerebraler Leistungsinsuffizienz, insbesondere von Gedächnisstörungen unterschiedlicher Genese, wie senile Demenz oder Demenz von Alzheimer-Typ, ferner, Folgezuständen von Hirntraumata und Apoplexien, Verwendung finden.

Die Erfindung betrifft vor allem Verbindungen der Formel I, worin $R_1$ und $R_2$ unabhängig voneinander Carboxy-$C_1$—$C_4$-alkyl, wie Carboxymethyl oder 2-Carboxyethyl, Carbamyl-$C_1$—$C_4$-alkyl, wie Carbamylmethyl oder 2-Carbamylethyl, N-Mono- oder N,N-Di-$C_1$—$C_4$-alkylcarbamyl-$C_1$—$C_4$-alkyl, wie N-Mono- oder N,N-Dimethylcarbamylmethyl oder N-Mono- oder N,N-Diisopropylcarbamylmethyl, N-Mono-oder N,N-Di(ω-hydroxy-$C_2$—$C_4$-alkyl)carbamyl-$C_1$—$C_4$-alkyl, wie N-Mono- oder N,N-Di(2-hydroxyethyl)-carbamylmethyl, oder N-(ω-Amino-$C_2$—$C_4$-alkyl)carbamyl-$C_1$—$C_4$-alkyl, wie N-(2-Aminoethyl)-carbamylmethyl, N-[ω-(N',N'-Di-$C_1$—$C_4$-alkylamino)-$C_2$—$C_4$-alkyl]carbamyl-$C_1$—$C_4$-alkyl, wie N-(2-Dimethylaminoethyl)carbamylmethyl oder N-(2-Diisopropylethyl)carbamylmethyl, N-(ω-Alkylenamino-$C_2$—$C_4$-alkyl)carbamyl-$C_1$—$C_4$-alkyl mit 4 bis und mit 7 Ringgliedern, wie N-[2-(3,3-Dimethylazetidino)ethyl]-carbamylmethyl, N-(2-Pyrrolidinoethyl)carbamylmethyl, N-(2-Piperidinoethyl)carbamylmethyl oder N-[2-(2,6-Dimethylpiperidino)ethyl]carbamylmethyl, N-[ω-(3-Azaalkylenamino)-$C_2$—$C_4$-alkyl]carbamyl-$C_1$—$C_4$-alkyl mit 4 bis und mit 7 Ringgliedern, wie N-(2-Piperazinoethyl)-carbamylmethyl oder N-[2-(4-Methyl-piperazino)ethyl]carbamylmethyl, N-[ω-(3-Oxaalkylenamino)-$C_2$—$C_4$-alkyl]carbamyl-$C_1$—$C_4$-alkyl mit 4 bis und mit 7 Ringgliedern, wie N-(2-Morpholinoethyl)carbamylmethl, N-(Phenyl-$C_1$—$C_4$-alkyl)-carbamyl-$C_1$—$C_4$-alkyl, wobei der Phenylteil durch $C_1$—$C_4$-Alkyl, wie Methyl, $C_1$—$C_4$-Alkoxy, wie Methoxy, Halogen der Atomnummer bis und mit 35, wie Chlor, und/oder Trifluormethyl mono- oder disubstituiert sein kann, wie N-(2-Phenylethyl)- oder N-[2-(3,4-Dimethoxyphenyl)ethyl]carbamylmethyl, N-(α-Carbamyl-$C_1$—$C_4$-alkyl)carbamyl-$C_1$—$C_4$-alkyl, wie N-(Carbamylmethyl)carbamylmethyl, N,N-Alkylencarbamyl-$C_1$—$C_4$-alkyl mit 4 bis und mit 7 Ringgliedern, wie (3,3-Dimethylazetidino)carbonylethyl, Pyrrolidonocarbonylmethyl, Piperidinocarbonylmethyl oder (2,6-Dimethylpiperidino)carbonylmethyl, N,N-(Azaalkylen)carbamyl-$C_1$—$C_4$-alkyl mit 4 bis und mit 7 Ringglierdern, wie Piperazinocarbonylmethyl oder (4-Methylpiperazino)-carbonylmethyl, N,N-(3-Oxaalkylen)carbamyl-$C_1$—$C_4$-alkyl mit 4 bis und mit 7 Ringgliedern, wie Morpholinocarbonylmethyl, oder N,N-(3-Thiaalkylen)carbamyl-$C_1$—$C_4$-alkyl mit 4 bis und mit 7 Ringglierdern, wie Thiomorpholinocarbonylmethyl, bedeutet, $R_3$, $R_4$, $R_5$ und $R_6$ für Wasserstoff stehen oder $R_3$ und $R_5$ gleiche $C_1$—$C_4$-Alkylreste, wie Methyl, und $R_4$ und $R_6$ Wasserstoff oder gleiche $C_1$—$C_4$-Alkylreste, wie Methy, oder $R_3$ und $R_4$ sowie $R_5$ und $R_6$ gemeinsam jeweils gleiche $C_2$—$C_6$-Alkylenreste, wie Ethylen, 1,3-Propylen, 1,4-Butylen, 1,5-Pentylen oder 1,3-(2,2-Dimethyl)propylen, darstellen und $R_7$ gemeinsam mit $R_8$ und/oder $R_9$ gemeinsam mit $R_{10}$ geweils eine zusätzliche Bindung bedeuten, insbesondere solche, in denen $R_1$ und $R_2$ gleich sind und $R_3$, $R_4$, $R_5$ und $R_6$ Wasserstoff darstellen, und ihre Salze insbesondere pharmazeutisch verwendbaren Salze.

Die Erfindung betrifft vorzugsweise Verbindungen der Formel I, worin $R_1$ und $R_2$ gleich sind und Carboxy-$C_1$—$C_4$-alkyl, wie Carboxymethyl oder 2-Carboxyethyl, Carbamyl-$C_1$—$C_4$-alkyl, wie Carbamylmethyl oder 2-Carbamylethyl, N-Mono-$C_1$—$C_7$- oder N,N-Di-$C_1$—$C_4$-alkylcarbamyl-$C_1$—$C_4$-alkyl, wie N-Ethyl-, N-(2,2-Dimethylpropyl)- oder N,N-Diisopropylcarbamylmethyl, N-(ω-Di-$C_1$—$C_4$-alkylamino-$C_2$—$C_4$-alkyl)carbamyl-$C_1$—$C_4$-alkyl, wie N-(2-Diisopropylaminoethyl)carbamylmethyl, N-(ω-Hydroxy-$C_2$—$C_4$-alkyl)carbamyl-$C_1$—$C_4$-alkyl, wie N-(2-Hydroxyethyl)carbamylmethyl, N-(ω-$C_1$—$C_4$-Alkoxy-$C_2$—$C_4$-alkyl)carbamyl-$C_1$—$C_4$-alkyl, wie N-(3-Methoxypropyl)carbamylmethyl, N-$C_5$—$C_6$-Cycloalkyl-, N-$C_8$—$C_9$-Bicycloalkyl- oder N-$C_8$—$C_{10}$-Tricycloalkylcarbamyl-$C_1$—$C_4$-alkyl, wie N-Cyclohexyl-, N-Bicyclo[2,2,2]-octyl- oder Adamantylcarbamylmethyl, 5- oder 6-gliedriges, gegebenenfalls durch $C_1$—$C_4$-Alkoxycarbonyl, wie Methoxy- oder Ethoxycarbonyl, 3- und Oxo- oder Hydroxy 4-substituiertes N,N-Alkylenaminocarbonyl-$C_1$—$C_4$-alkyl, wie Pipieridinocarbonylmethyl, 3-Methoxycarbonyl-4-hydroxy-piperidinocarbonylmethyl, 3-Ethoxycarbonyl-4-hydroxy-piperidinocarbonylmethyl, 3-Ethoxycarbonyl-4-oxo-piperidinocarbonylmethyl oder 3-Methoxycarbonyl-4-oxo-piperidinocarbonylmethyl, 5- oder 6-gliedriges, durch $C_1$—$C_4$-Alkoxycarbonyl, wie Methoxy- oder Ethoxycarbonyl, 3-substituiertes und gegebenen falls zusätzlich durch Hydroxy 4-substituiertes Alkenylaminocarbonyl-$C_1$—$C_4$-Alkyl, wie N-(3-Methoxycarbonyl-1,2,5,6-tetrahydropyridino)- oder N-(4-Hydroxy-3-ethoxycarbonyl-1,2,5,6-tetrahydropyridino)carbonylmethyl, 5- bis 6-gliedriges, gegebenenfalls durch $C_1$—$C_4$-Alkyl oder $C_1$—$C_4$-Alkanoyl N'-substituiertes N,N-(Aza)alkylenaminocarbonyl-$C_1$—$C_4$-alkyl, wie (N'-Methyl)piperazino- oder (N'-Acetyl)piperazinocarbonylmethyl, 5- bis 6-gliedriges N,N-(Oxa)alkylenaminocarbonyl-$C_1$—$C_4$-alkyl, wie Morpholinocarbonylmethyl, oder im Phenylteil gegebenenfalls durch $C_1$—$C_4$-Alkoxy, wie Methoxy, mono- oder disubstituiertes N-Phenyl-$C_1$—$C_4$-alkylcarbamyl-$C_1$—$C_4$-alkyl, wie N-(2-Phenylethyl)carbamylmethyl oder N-[2-(3,4-Dimethoxyphenyl)ethyl]carbamylmethyl, bedeuten, $R_3$, $R_4$, $R_5$ und $R_6$ Wasserstoff bedeuten und $R_7$, $R_8$, $R_9$ und $R_{10}$ jeweils für Wasserstoff stehen oder insbesondere $R_7$ gemeinsam mit $R_8$ sowie $R_9$ gemeinsam mit $R_{10}$ je eine zusätzliche Bindung darstellen, insbesondere solche, in denen $R_1$ und $R_2$ gleich sind und $R_3$, $R_4$, $R_5$ und $R_6$ Wasserstoff darstellen, und deren Salze, insbesondere pharmazeutisch verwendbare Salze.

Die Erfindung betrifft in allererster Linie Verbindungen der Formel I, worin $R_1$ und $R_2$ gleiche Carboxy-$C_1$—$C_4$-alkyl- bzw. Carbamyl-$C_1$—$C_4$-alkylreste, wie Carboxymethyl bzw. Carbamylmethyl, oder durch ω-N,N-Di-$C_1$—$C_4$-alkylamino-$C_2$—$C_4$-alkyl, wie 2-Diisopropylaminoethyl, oder Carbamylmethyl N-monosubstituierte, durch ω-Hydroxy-$C_2$—$C_4$-alkyl, wie 2-Hydroxyethyl, N-mono- oder N,N-disubstituierte oder durch 4- bis 6-gliedriges (3-$C_1$—$C_4$-Alkyl-3-aza)alkylen, worin $C_1$—$C_4$-Alkyl z.B. Methyl bedeutet, N,N-disubtituierte Carbamylmethylreste darstellen und $R_3$, $R_4$, $R_5$ und $R_6$ für Wasserstoff stehen und $R_7$ und $R_8$ sowie $R_9$ und $R_{10}$ gemeinsam je eine zusätzliche Bindung darstellen, und deren Salze, insbesondere pharmazeutisch verwendaren Salze.

Die Erfindung betrifft ganz besonders Verbindungen der Formel I, worin $R_1$ und $R_2$ gleiche Carboxy-$C_1$—$C_4$-alkyl- bzw. Carbamyl-$C_1$—$C_4$-alkylreste, wie Carboxymethyl bzw. Carbamylmethyl, darstellen und $R_3$, $R_4$, $R_5$ und $R_6$ für Wasserstoff stehen und $R_7$ und $R_8$ sowie $R_9$ und $R_{10}$ gemeinsam je eine zusätzliche Bindung darstellen, und deren Salze, insbesondere pharmazeutisch verwendbaren Salze.

Die Erfindung betrifft die Verbindungen der Formel I jeweils vorzugsweise in Form ihrer pharmazeutisch verwendbaren Alkalimetall-, Erdalkalimetall- oder Ammoniumsalze mit Ammoniak oder pharmazeutisch verwendbaren Niederalkyl und/oder Hydroxyniederalkyl aufweisenden aliphatischen Aminen, vorzugsweise in Form ihrer Natrium-, Kalium-, Ammonium-, Diethylammonium-, Bis(2-hydroxyethyl)ammonium-, Tris(2-hydroxyethyl)ammonium-, Tris(hydroxymethyl)methyl-ammonium oder N,N-Dimethyl-N-(2-hydroxyethyl)ammoniumsalze.

Die Erfindung betrifft namentlich die in den Beispielen genannten Verbindungen der formel I und ihre pharmazeutisch verwendbaren Salze.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man

a) eine Verbindung der Formel

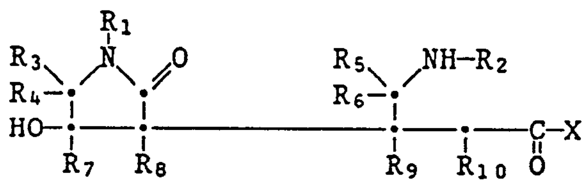

(II)

worin X gegebenenfalls verestertes oder verethertes Hydroxy bedeutet, oder ein Salz davon cyclisiert oder

b) in einer Verbindung der Formel

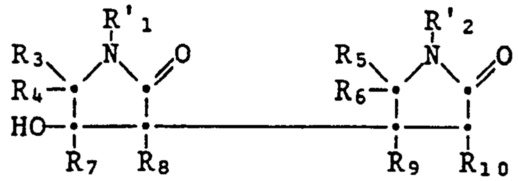

(III)

worin $R'_1$ eine in einen Rest $R_1$ überführbare Gruppe und $R'_2$ einen Rest $R_2$ oder eine in diesen überführbare Gruppe bedeutet, oder ein einem Salz davon $R'_1$ in eine Gruppe $R_1$ und gegebenenfalls einen in $R_2$ überführbaren Rest $R'_2$ in eine Gruppe $R_2$ überführt oder

c) in einer Verbindung der Formel

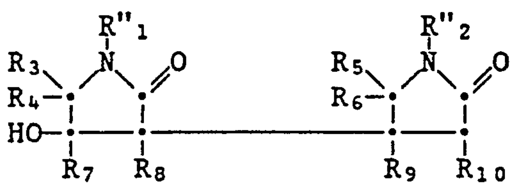

(IV)

worin $R''_1$ Wasserstoff und $R''_2$ Wasserstoff oder einen Rest $R_2$ bedeutet, oder in einem Salz davon das Wasserstoffatom $R''_1$ durch einen Rest $R_1$ und gegebenenfalls das Wasserstoffatom $R''_2$ durch einen Rest $R_2$ ersetzt oder

d) eine Verbindung der Formel

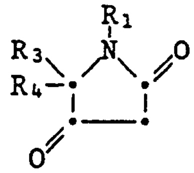

(V)

oder ein Tautomeres und/oder Salz davon mit einer Verbindung der Formel

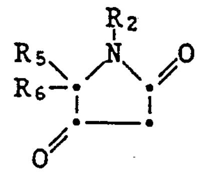

(VI)

oder einem Tautomeren davon kondensiert und gewünschtenfalls eine verfahrensgemäss erhältliche Verbindung in eine andere Verbindung der Formel I überführt, ein verfahrensgemäss erhältliches Isomerengemisch in die Isomeren auftrennt und/oder eine verfahrengemäss erhältliche freie Verbindung in ein Salz oder ein verfahrensgemäss erhältliches Salz in die freie Verbindung oder in ein anderes Salz überführt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

In Ausgangsstoffen der Formel II für die Cyclisierung gemäss der Verfahrensvariante a) ist verestertes Hydroxy X beispielsweise mit einer Halogenwasserstoffsäure, wie Chlorwasserstoffsäure, oder mit einer Niederalkansäure verestert und verethertes Hydroxy beispielsweise mit einem Niederalkanol oder einem gegebenenfalls, z.B. durch Niederalkyl, Niederalkoxy, Halogen und/oder Nitro, substituierten Phenol verethert. X bedeutet somit beispielsweise Hydroxy, Halogen, z.B. Chlor, Niederalkanoyloxy, z.B. Acetoxy, Niederalkoxy, z.B. Methoxy oder Ethoxy, Phenyloxy oder p-Nitrophenyloxy.

Salze von Verbindungen der Formel II sind beispielsweise Alkalimetallsalze, z.B. Natrium- oder Kaliumsalze, oder innere Salze derselben.

Die intramolekulare Kondensation erfolgt in üblicher Weise, ausgehend von Verbindungen der Formel II, worin X Hydroxy bedeutet, beispielsweise durch Dehydratisierung, d.h. Entfernung des gebildeten Reaktionswassers, z.B. mit Hilfe eines wasserbindenden Mittels, wie Phosphorpentoxid oder eines organischen Carbodiimides, z.B. von Dicyclohexylcarbodiimid, oder durch Destillation, insbesondere azeotrope Destillation, ausgehend von Verbindungen der Formel II, worin X verestertes Hydroxy ist, bespielsweise in Gegenwart einer Base, wie eines Alkalimetall- oder Erdalkalimetallhydroxides oder -carbonates, z.B. von Natrium- oder Kaliumhydroxid oder Kaliumcarbonat, oder eines tertiären organischen Amins, wie eines Triniederalkylamins, z.B. von Triethylamin, oder von Pyridin, und ausgehend von Verbindungen der Formel II, worin X verethertes Hydroxy ist, erforderlichenfalls unter Entfernung des gebildeten Alkohols, beispielsweise durch Destillation oder azeotrope Destillation.

Die Ausgangsstoffe der Formel II werden vorzugsweise *in situ* hergestellt und ohne Isolierung cyclisiert, beispielsweise indem man eine Verbindung der Formel

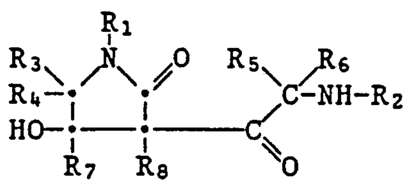

(VII)

in Gegenwart einer Base, insbesondere einer Metallbase, wie eines Alkalimetallhydrides, -amides oder -alkoholates, z.B. von Natriumhydrid, Natriumamid, Lithiumiisopropylamid oder eines Natrium- oder Kaliumniederalkanolates, vor allem von Natriummethanolat, mit einer Verbindung der Formel $CH_3—C(=O)—X_1$ (VIII) kondensiert, worin $X_1$ insbesondere verethertes Hydroxy, wie Niederalkoxy, bedeutet. Gewünschtenfalls kann man in der erhaltenen Verbindung der Formel II, worin $R_9$ und $R_{10}$ gemeinsam eine zusätzliche Bindung darstellen, die Doppelbindung zu einer Einfachbindung absättigt, beispielsweise mittels Wasserstoff in Gegenwart von Palladiumkohle oder eines anderen geeigneten Hydrierungskatalysators.

Zwischenprodukte der Formel VII ihrerseits können beispielsweise hergestellt werden, indem man eine Verbindung der Formel

$$R_2—NH—C(R_5)(R_6)—C(=O)—CH_2—C(=O)—X_1 \quad (IXb)$$

worin $X_1$ verethertes Hydroxy, z.B. Niederalkoxy ist, in Gegenwart einer Base, insbesondere einer Metallbase, wie eines Alkalimetallhydrides, -amides oder -alkoholates, z.B. von Natriumhydrid, Natriumamid, Lithiumdiisopropylamid oder eines Natrium- oder Kaliumniederalkanolates, vor allem von Natriummethanolat, mit einer Verbindung der Formel

$$R_1—NH—C(R_3)(R_4)—C(=O)—X \quad (Xa)$$

worin X gegebenenfalls verestertes oder verethertes Hydroxy, insbesondere Chlor, Niederalkoxy oder gegebenenfalls substituiertes Phenyloxy, z.B. p-Nitrophenyloxy, ist, umsetzt und in dem Reaktionsprodukt VII, worin $R_7$ und $R_8$ gemeinsam für eine zusätzliche Bindung stehen, gewünschtenfalls die Doppelbindung zu einer Einfachbindung absättigt, beispielsweise mittels Wasserstoff in Gegenwart von Palladiumkohle oder eines anderen geeigneten Hydrierungskatalysators.

Verbindungen der Formel VII können weiterhin erhalten werden, indem man eine Verbindung der Formeln

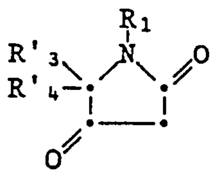 (XIa) 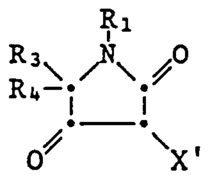 (XIb) oder 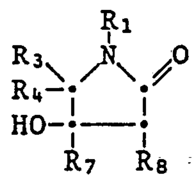 (XIc),

worin $R'_3$ und $R'_4$ von Wasserstoff verschieden sind und X' Ethoxycarbonyl bedeutet, oder ein Tautomeres, z.B. jeweils das $\Delta_{3,4}$-Enol, einer Verbindung XIa bzw. XIb in Gegenwart einer Base, insbesondere einer Metallbase, wie eines Alkalimetallhydrides, -amides oder -alkoholates, z.B. von Natriumhydrid, Natriumamid, Lithiumdiisopropylamid oder eines Natrium- oder Kaliumniederalkanolates, vor allem von Natriummethanolat, mit einer Verbindung der Formel

$$R_2—NH—C(R_5)(R_6)—C(=O)—X \quad (Xb)$$

umsetzt und in ausgehend von Verbindungen XIa bzw. XIb erhaltenen Verbindungen VII, worin $R_7$ und $R_8$ gemeinsam eine zusätzliche Bindung darstellen, gewünschtenfalls die Doppelbindung zu einer Einfachbindung absättigt, beispielsweise mittels Wasserstoff in Gegenwart von Palladiumkohle oder eines anderen geeigneten Hydrierungskatalysators.

Gemäss der Verfahrenvariaten b) in Reste $R_1$ und/oder $R_2$ überführbare Gruppen $R'_1$ bzw. $R'_2$ sind beispielsweise von gegebenenfalls substituiertem Carbamylniederalkyl $R_1$ bzw. $R_2$ verschiedene funktionell abgewandelte Carboxyniederalkylreste, wie veresterte oder anhydridisierte Carboxyniederalkylreste, ferner Cyanoniederalkylreste. Veresterte Carboxyniederalkylreste sind dabei beispielsweise aliphatisch oder aromatisch veresterte Carboxyniederalkylreste, wie Niederalkoxycarbonylniederalkyl oder, z.B. durch Niederalkyl, Niederalkoxy, Halogen und/oder Nitro, substituierte Phenyloxycarbonylniederalkylreste, z.B. Phenyloxy-, p-Nitrophenyloxy- oder 2,4-Dinitrophenyloxycarbonylniederalkylreste. Anhydridisierte Carboxyniederalkylreste sind beispielsweise Halogen-, insbesondere Chlorcarbonylniederalkylreste. Vorzugsweise bedeuten entweder beide Reste $R'_1$ und $R'_2$ gleiche in identische Reste $R_1$ und $R_2$ überführbare Gruppen oder stellt $R'_2$ eine Gruppe $R_2$ und $R'_1$ einen in eine Gruppe $R_1$ überführbaren Rest dar.

Die Ueberführung funktionnel abgewandelt Carboxyniederalkylreste $R'_1$ und/oder $R'_2$ in Carboxyniederalkyl $R_1$ und/oder $R_2$ erfolgt beispielsweise durch Hydrolyse, die Ueberführung veresterter oder anhydridisierter Carboxyniederalkylreste in gegebenenfalls substituierte Carbamylniederalkylreste $R_1$ bzw. $R_2$ bespielsweise durch Umsetzung mit Ammoniak oder einem entsprechenden Amin, z.B. einem Mono- oder Diniederalkylamin, Mono- oder Di-(hydroxyniederalkyl)amin, jeweils 4- bis 8-gliedrigen, gegebenenfalls durch Niederalkoxycarbonyl und Oxo oder Hydroxy substituiertes N-(Alkylenaminoniederalkyl)amin bzw. gegebenenfalls durch Niederalkoxycarbonyl und gegebenenfalls zusätzlich durch Hydroxy substituiertes N-(Alkenylenaminoniederalkyl)amin, gegebenenfalls durch Niederalkyl C- und/oder N'-substituiertes oder durch Niederalkanoyl N'-substituiertes N-[(Aza)alkylen-aminoniederalkyl]amin, oder N-[(Oxa- oder Thia)alkylenminoniederalkyl]amin, jeweils 3- bis 8-gliedrigen Mono- oder Di(cycloalkylaminoniederalkyl)amin, (Bicycloalkylaminoniederalkyl)amin, (Tricyclo-alkylaminoniederalkyl)amin, gegebenenfalls im Phenylteil durch Niederalkyl, Niederalkoxy, Halogen und/oder Trifluormethyl substituierten Mono- oder Di-(phenylniederalkyl)amin oder jeweils 4- bis 8-gliedrigen gegebenenfalls durch Niederalkoxycarbonyl und Hydroxy oder Oxo substituierten Alkylenamin, gegebenegalls durch Niederalkoxycarbonyl und gegebenenfalls zusätzlich durch Hydroxy substituierten Alkenylamin, gegebenenfalls durch Niederalkyl und/oder Niederalkanoyl substituierten Azaalkylenamin oder Oxa- oder Thiaalkylenamin. Die Hydrolyse erfolgt in üblicher Weise, beispielsweise in Gegenwart eines sauren oder basischen Mittels. Die Umsetzung von veresterten oder anhydrisierten Carboxyniederalkylresten mit Ammoniak erfolgt, erforderlichenfalls unter Dehydratisierung primär gebildeter Ammoniumsalz, beispielsweise durch Erhitzen, Behandlung mit einem wasserbindenden Mittel, wie Phosphorpentoxid oder einem organischen Carbodiimid, z.B. mit Dicyclohexylcarbodiimid, oder eines säurebindenen Mittels, wie einer tertiären organischen Stickstoffbase, wie von Pyridin oder eines Triniederalkylamins, z.B. von Triethylamin, bzw. unter Entfernung des gebildeten Alkohols durch Destillation oder azeotrope Destillation. Ausgehend von Verbindungen der Formel III, worin $R'_1$ und/oder $R'_2$ für verestertes Carboxyniederalkyl steht, wird erforderlichenfalls der gebildete Alkohol destillativ oder azeotrop-destillativ aus dem Reaktionssystem entfernt. Ausgehend von Verbindungen der Formel III, worin

R'$_1$ und/oder R'$_2$ anhydrisiertes Carboxyniederalkyl bedeutet, arbeitet man vorteilhaft in Gegenwart einer Base, wie eines Alkalimetalle- oder Erdalkalimetallhydroxides oder -carbonates, z.B. von Natrium- oder Kaliumhydroxide oder Kaliumcarbonat, oder eines tertiären organischen Amins, beispielsweise eines Triniederalkylamins, z.B. von Triethylamin, oder von Pyridin. Cyanoniederalkyl R'$_1$ und/oder R'$_2$ kann zu Carboxyniederalkyl oder zu Carbamylneideralkyl hydrolysiert oder durch Umsetzung mit einem sauren Mittel und anschliessend einem primären oder sekundären der genannten Amine zunächst in eine Amidinogruppierung überführt werden, die dann zum entsprechende N-mono- oder N,N-disubstituierten Carbamylneideralkylrest hydrolysiert werden kann.

Die Hydrolyse kann in beiden Fälle in Gegenwart eines sauren Mittels, in erstgenanntem Fall auch in Gegenwart eines basischen Mittels in Gegenwart von Wasserstoffperoxid durchgeführt werden. Geeignete saure Mittels sind beispielsweise Mineralsäuren, wie Halogenwasserstoffsäuren, z.B. Chlor- oder Bromwasserstoffsäure, oder Sauerstoffsäuren des Schwefels oder Phosphors, z.B. Schwefel-, Phosphor- oder Polyphosphorsäure. Basische Mittel sind beispielsweise Alkalimetall- oder Erdalkalimetallhydroxide oder Carbonate, z.B. Natrium-, Kalium- oder Bariumhydroxid oder Kaliumcarbonat.

Die Ausgangsstoffe der Formel III können beispielsweise hergestellt werden, indem man eine Verbindung der Formel

$$R'_2—NH—C(R_5)(R_6)—C(=O)—CH_2—C(=O)—X_1 \qquad \text{(IXc)}$$

worin X$_1$ verethertes Hydroxy, z.B. Niederalkoxy, bedeutet, in Gegenwart einer Base, insbesondere einer Metallbase, wie eines Alkalimetallhydrides, -amides oder -alkoholates, z.B. von Natriumhydrid, Natriumamid, Lithiumdiisopropylamid oder eines Natrium- oder Kaliumniederalkanolates, vor allem von Natriummethanolat, mit einer Verbindung der Formel

$$R'_1—NH—C(R_3)(R_4)—C(O=)—X \qquad \text{(Xb)}$$

worin X gegebenenfalls verestertes oder verethertes Hydroxy, insbesondere Chlor, Niederalkoxy oder gegebenenfalls substitutiertes Phenyloxy, z.B. p-Nitrophenyloxy, ist, umsetzt und in dem Reaktionsprodukt der Formel

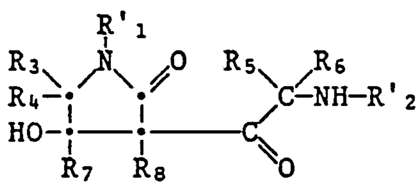

(VIIa),

worin R$_7$ und R$_8$ gemeinsam für eine zusätzliche Bindung stehen, gewünschtenfalls die Doppelbindung zu einer Einfachbindung absättigt, beispielsweise mittels Wasserstoff in Gegenwart von Palladiumkohle oder eines anderen geeigneten Hydrierungskatalysators, die Verbindung VIIa in Gegenwart einer Base, insbesondere einer Metallbase, wie eines Alkalimetallhydrides, -amides oder -alkoholates, z.B. von Natriumhydrid, Natriumamid, Lithiumdiisopropylamid oder eines Natrium- oder Kaliumniederalkanolates, vor allem von Natriummethanolat, mit einer Verbindung der Formel

$$CH_3—C(=O)—X_1 \qquad \text{(VIII)}$$

kondensiert, worin X$_1$ insbesondere verethertes Hydroxy, wie Niederalkoxy, bedeutet, und das Reaktionsprodukt der Formel

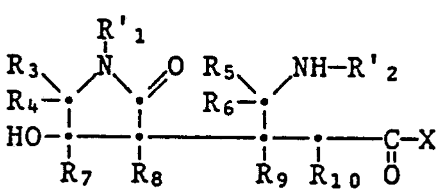

(IIa),

worin R$_9$ und R$_{10}$ gemeinsam eine zusätzliche Bindung darstellt, in üblicher Weise, z.B. wie für die Verfahrensvariante a) angegeben, cyclisiert und gewünschtenfalls vorgängig oder nachfolgend die durch R$_9$ und R$_{10}$ gemeinsam dargestellte Doppelbinding zur Einfachbindung hydriert, beispielsweise mittels Wasserstoff in Gegenwart von Palladiumkohle oder eines anderen geeigneten Hydrierungskatalysators.

Ein besonders elegantes Verfahren zur Herstellung von Verbindungen der Formel III, worin R'$_1$ und R'$_2$ gleich sind und beispielsweise Niederalkoxycarbonylniederalkyl, insbesondere Methoxycarbonylniederalkyl, bedeuten und R$_3$, R$_4$, R$_5$ und R$_6$ für Wasserstoff stehen, besteht darin, dass man eine Verbindung der Formel

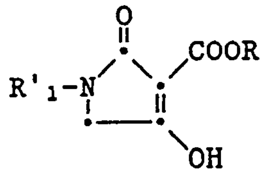

(XII),

worin COOR Niederalkoxycarbonyl, insbesondere Methoxycarbonyl, bedeutet, oder ein Tautomeres, z.B. das entsprechende $\Delta_{3,4}$-Enol, davon in wässrigem Acetonitril auf etwa 50°C bis 82°C erwärmt, worin unter Abspaltung der ROOC-Gruppe Dimerisierung zur entsprechenden Verbindung der Formel

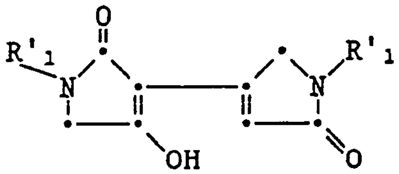

(III')

erfolgt, die in einer besonders vorteilhaften Ausführungsform des erfindungsgemässen Verfahrens durch Umsetzung mit Ammoniak oder einem geeigneten Amin zum entsprechenden Ammoniumsalze der entsprechenden Verbindung der Formel

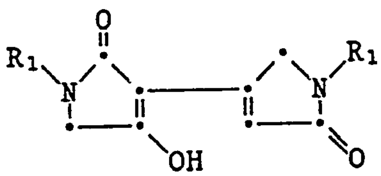

(I')

aminolysiert werden kann, aus dem die freie Verbindung der Formel I' dann durch Säurebehandlung freigesetzt werden kann.

Der Ersatz von Wasserstoff durch einen Rest $R_1$ und/oder $R_2$ gemäss der Verfahrensvariante c) erfolgt beispielsweise durch Umsetzung mit einer Verbindung der Formeln

$$R_1—X \quad (XIIIa)$$

und/oder

$$R_2—X \quad (XIIIb)$$

worin X jeweils reaktionsfähig verestertes Hydroxy, wie mit einer Halogenwasserstoffsäure oder einer Sulfonsäure verestertes Hydroxy, beispielsweise Chlor, Brom oder Jod oder Alkan- bzw. gegebenenfalls substituiertes Benzolsulfonyloxy, z.B. Methan-, Aethan-, Benzol-, p-Toluol- oder p-Brombenzolsulfonyloxy, bedeutet. Auch in diesem Falle stehen vorzugsweise entweder $R''_1$ und $R_2$ beide für Wasserstoff oder ist $R''_2$ ein Rest $R_2$ und $R''_1$ Wasserstoff.

Die Umsetzung erfolgt in üblicher Weise, vorteilhaft in Gegenwart eines basischen Kondensationsmittels, wie einer Metall- oder quaternären Ammoniumbase, oder eines tertiären Amins, beispielsweise eines Hydroxides, Carbonates, Alkoholates, Amides oder Hydrides eines Alkalimetalls oder Erdalkimetalles, z.B. von Natrium- oder Kaliumhydroxid, Kaliumcarbonat, Natriummethanolat, Kaliumtertiärbutanolat, Natriumamid, Lithiumdiisopropylamid oder Natriumhydrid, eines quaternären Ammoniumhydroxides, z.B. von Tetrabutylammoniumhydroxid oder Benzyl-trimethylammoniumhydroxid, oder eines Triniederalkylamins oder einer tertiären heteroaromatischen Base, z.B. von Triethylamin oder Pyridin.

Die Ausgangsstoffe der Formel IV können beispielsweise hergestellt werden, indem man eine Verbindung der Formel

$$R''_2—NH—C(R_5)(R_6)—C(=O)—CH_2—C(=O)—X_1 \quad (IXd)$$

worin $X_1$ veretheretes Hydroxy, z.B. Niederalkoxy darstellt in Gegenwart einer Base, inbesondere einer Metallbase, wie eines Alkalimetallhydrides, -amides oder -alkoholates, z.B. von Natriumhydrid, Natriumamid, Lithiumdiisopropylamid oder eines Natrium- oder Kaliumniederalkanolates, vor allem von Natriuummethanolat, mit einer Verbindung der Formel

$$R''_1—NH—C(R_3)(R_4)—C(=O)—X \quad (Xc)$$

worin X gegebenenfalls verestertes oder verethertes Hydroxy, insbesondere Chlor, Niederalkoxy oder

gegebenenfalls substituiertes Phenyloxy, z.B. p-Nitrophenyloxy, ist, umsetzt und in dem Reaktionsprodukt der Formel

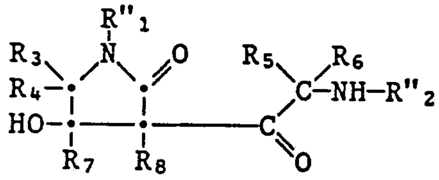

(VIIb),

worin $R_7$ und $R_8$ gemeinsam eine zusätzliche Bindung darstellen, gewünschtenfalls die Doppelbindung zu einer Einfachbindung absättigt, beispielsweise mittels Wasserstoff in Gegenwart von Palladiumkohle oder eines anderen geeigneten Hydrierungskatalysators, die Verbindung VIIb in Gegenwart einer Base, insbesondere einer Metallbase, wie eines Alkalimetallhydrides, -amides oder -alkololates, z.B. von Natriumhydrid, Natriumamid, Lithiumdiisopropylamid oder eines Natrium- oder Kaliumniederalkanolates, vor allem von Natriummethanolat, mit einer Verbindung der Formel

$$CH_3—C(=O)—X_1 \qquad (VIII)$$

kondensiert, worin $X_1$ insbesondere verethertes Hydroxy, wie Niederalkoxy, bedeutert, und das Reaktionprodukt der Formel

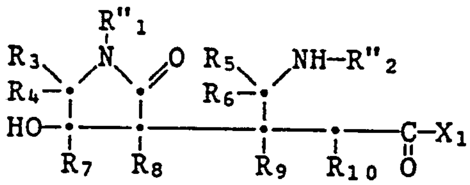

(IIb)

worin $R_9$ und $R_{10}$ gemeinsam eine zusätzliche Bindung darstellt, in überlicher Weise, z.B. wie für die Verfahrenvariante a) angegeben, cyclisiert und gewünschtenfalls vorgängig oder nachfolgend die durch $R_9$ und $R_{10}$ gemeinsam dargestellte Doppelbindung zur Einfachbindung hydriert.

Verbindungen der Formel IV, worin $R''_1$, $R''_2$, $R_3$, $R_4$, $R_5$ und $R_6$ Wasserstoff bedeuten, können ferner hergestellt werden, indem man ein gegebenenfalls durch Niederalkyl, Niederalkoxy oder Halogen p-substituierte Benzoylazid unter Bestrahlung mit einer Hochdruckquecksilberdampflampe mit Diketen umsetzt und das Reaktionsprudukt der Formel

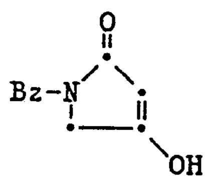

(XIV),

worin Bz gegebenenfalls durch Niederalkyl, Niederalkoxy oder Halogen p-substituiertes Benzoyl bedeutet, mit einer Base, insbesondere einem Alkalimetall- oder Erdalkalimetallhydroxid, behandelt.

Verbindungen der Formel V, worin $R''_1$ und $R''_2$ Wasserstoff darstellen, und worin $R_5$ und $R_6$ die gleichen Bedeutungen haben wie $R_3$ und $R_4$, können ferner hergestellt werden, indem man eine Verbindung der Formel

$$X_1—C(=O)—C(R_3)(R_4)—NH—C(=O)—CH_2—C(=O)—X_1 \qquad (XV)$$

worin die Gruppe $X_1$ verethertes Hydroxy, z.B. Niederalkoxy, bedeutet, mit einer Metallbase, z.B. mit Natriummethanolat oder -ethanolat, umsetzt und die erhaltene Verbindung der Formel

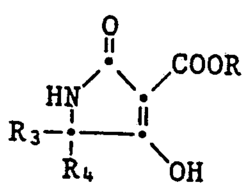

(XVI)

mit einer Base, beispielsweise einem Alkalimetallhydroxid, wie Natriumhydroxid in Wasser, behandelt.

Die Kondensation von Verbindungen der Formeln V und VI gemäss der Verfahrensvariante d) erfolgt im allgemeinen spontan, wird aber durch Erwärmen und Gegenwart einer Base, wie einem Alkalimetall- oder Erdalkalimetallhydroxid, z.B. durch Natrium-, Kalium oder Bariumhydroxid, oder einer Säure, wie

einer Halogenwasserstoffsäure, z.B. von Chlorwasserstoffsäure, einer Sauerstoffsäure des Schwefels oder Phosphors, z.B. von Schwefel- oder Phosphorsäure, oder einer organischen Carbonsäure, z.B. von Essigsäure beschleunigt.

In einer besonders zur Herstellung von Verbindungen der Formel I, worin $R_1$ und $R_2$ gleich sind, und worin $R_5$ und $R_6$ die gleichen Bedeutungen haben wie $R_3$ und $R_4$, geeigneten Ausführungsform der Verfahrensvariante d) können die Ausgangsstoffe beispielsweise *in situ* hergestellt und ohne Isolierung der Autokondensation unterworfen werden. Dabei geht man vorzugsweise von einer Verbindung der Formel

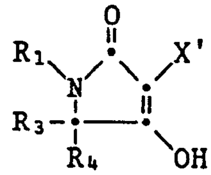

(XVII)

aus, worin X' einen abspalteren Rest, beispielsweise eine Acylgruppe, insbesondere eine von einer organischen Carbonsäure oder einem Halbester der Kohlensäure abgeleitete Acylgruppe, wie Niederalkanoly, z.B. Acetyl, bzw. gegebenenfalls, z.B. durch Niederalkyl, Niederalkoxy und/oder Halogen, substituiertes Benzoyl, oder Niederalkoxycarbonyl, z.B. Methoxycarbonyl bedeutet, und spaltet die Acylgruppe X' durch Erwärmen und/oder Base- oder Säureeinwirkung ab, wobei die intermediär gebildete Verbindung der Formel VI in der gewünschten Weise Autokondensation zu der entsprechenden Verbindung der Formel

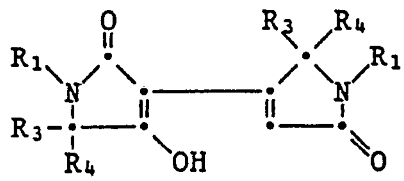

(I")

eingeht.

Zwischenprodukte XVII, worin X' eine von einem Halbester der Kohlensäure abgeleitete Acylgruppe bedeutet, werden beispielsweise hergestellt, indem man eine durch Umsetzung von Verbindungen der Formeln

$$H_2N—C(R_3)(R_4)—C(=O)—X_1 \qquad \text{(XVIIIa)}$$

und

$$R_1—Y \qquad \text{(XIXa)}$$

worin X verethertes Hydroxy, insbesondere Niederalkoxy, und Y Halogen, insbesondere Chlor oder Brom, bedeutet, erhältliche Verbindung der Formel

$$R_1NH—C(R_3)(R_4)—C(=O)—X_1 \qquad \text{(Xa)}$$

mit einer Verbindung der Formel

$$X_2—C(=O)—CH_2—X' \qquad \text{(XX)}$$

worin $X_2$ gegebenenfalls verestertes Hydroxy, insbesondere Hydroxy oder Chlor, darstellt, umsetzt und das Kondensationsprodukt der Formel

$$X'—CH_2—C(=O)—N(R_1)—C(R_3)(R_4)—C(=O)—X \qquad \text{(XXI)}$$

cyclisiert. Die Umsetzung von Verbindungen (XVIIIa) und (XIXa) sowie (Xa) und (XX; X = Chlor) erfolgt beispielsweise in Gegenwart einer Base, wie von Natriumhydroxid oder eines Triniederalkylamins, z.B. von Triethylamin, die Umsetzung von Verbindungen (Xa) und (XX; X = Hydroxy) beispielsweise in Gegenwart eines wasserbindenden Mittels, wie eines N,N'-Dialkyl- oder N,N'-Dicycloalkylcarbodiimides, z.B. von N,N'-Diisopropyl- oder N,N'-Dicyclohexylcarbodiimid.

Man kann abere auch in einer Verbindung der Formel

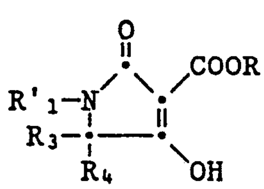

(XII),

worin COOR Niederalkoxycarbonyl, z.B. Methoxycarbonyl und $R'_1$ Niederalkoxy-, z.B.

Methoxycarbonylniederalkyl darstellt und die beispielsweise durch dem vorstehenden Reaktionscyklus analoge Umsetzung von Verbindungen (XVIIIa) und $R'_1$—Y (XXI; Y = Halogen) zur entsprechende Verbindung

$$R'_1—NH—C(R_3)(R_4)—C(=O)—X_1 \qquad (XXII)$$

Kondensation derselben mit einer Verbindung (XX) und Cyclisierung des Kondensationsproduktes gut zugänglich ist, die Gruppe $R'_1$ in üblicher Weise, beispielsweise wie unter der Verfahrensvariante c) angegeben, insbesondere durch Behandeln mit Ammoniak oder einem entsprechenden Amin, in eine Gruppe R überführen.

Verbindungen XVII, worin X' Acetyl bedeutet, können beispielsweise durch Umsetzung der entsprechende Verbindung (Xa) mit Diketen in Gegenwart einer Base, z.B. von Natriumethanolat in Ethanol, bequem erhalten werden.

In einer anderen bevorzugten Ausführungsform der Verfahrensvariante d), die auch die Herstellung von Verbindungen der Formel I, worin $R_1$ und $R_2$, $R_3$ und $R_5$ und/oder $R_4$ und $R_6$ verschieden sind, gestattet, setzt man aus einem annähernd aquimolekularen Gemisch von Verbindungen der Formeln

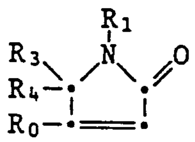 (XXIIIa) und 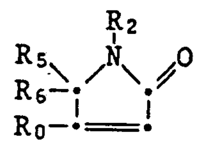 (XXIIIb),

worin die Gruppen $R_0$ gleiche oder verschiedene in Hydroxy überführbare Reste bedeuten, die entsprechenden Zwischenprodukte V und VI frei, die unter den Bedinungen ihrer Bildung verfahrensgemäss miteinander kondensieren. Sind $R_1$ und $R_2$, $R_3$ und $R_5$ und/oder $R_4$ und $R_6$ verschieden werden dabei neben unsymmetrischen Verbindungen I ($R_1 \neq R_2$, $R_3 \neq R_5$ und/oder $R_4 \neq R_6$) auch symmetrische Verbindungen I, z.B. Verbindungen der Formeln

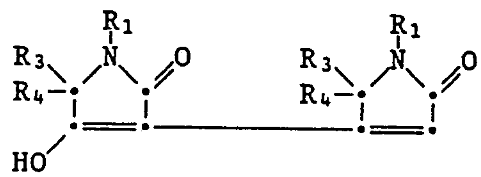

(Ia) bzw.

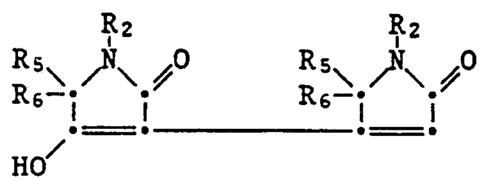

(Ib),

erhalten, die, sofern unerwünscht, aus dem Reaktionsgemisch abgetrennt werden müssen.

Die Freisetzung der Reaktionskomponenten V und VI d.h. Ueberführung von $R_0$ in Hydroxy, erfolgt in üblicher Weise beispielsweise durch Säurebehandlung. Verbindungen (XXIIIa) bzw. (XXIIIb) werden beispielsweise hergestellt, indem man eine Verbindung der Formel

$$R_3—CH(R_4)—C(=O)—CH_2—C(=O)—X_1 \qquad (XXIVa)$$

bzw.

$$R_5—CH(R_6)—C(=O)—CH_2—C(=O)—X_1 \qquad (XXIVb)$$

worin $X_1$ verethertes Hydroxy, z.B. Niederalkoxy, bedeutet durch Umsetzung von einem reaktiven Ester, z.B. einem Halogenwasserstoffester, eines Alkohols der Formel

$$R_0—H \qquad (XXV)$$

in Gegenwart eines Alkalimetallhydrides, in den Enolether der Formel

$$R_3—CH(R_4)—C(R_0)=CH—C(=O)—X \qquad (XXVIa)$$

bzw.

$$R_5—CH(R_6)—C(R_0)=CH—C(=O)—X \qquad (XXVIb)$$

überführt, diesen, beispielsweise mittels N-Bromsuccinimid halogeniert und das Reaktionsprodukt der Formel

$$R_3-\underset{\text{Hal}}{C}(R_4)-C(R_0)=CH-C(=O)-X \quad \text{(XXVIIa) bzw.}$$

$$R_5-\underset{\text{Hal}}{C}(R_4)-C(R_0)=CH-C(=O)-X \quad \text{(XXVIIb)}$$

worin Hal Halogen, z.B. Brom, bedeutet, mit der entsprechenden Aminoverbindung der Formel

$$H_2N—R_1 \quad \text{(XXVIIIa)}$$

bzw.

$$H_2N—R_2 \quad \text{(XXVIIIb)}$$

umsetzt.

Als Unwandlungsreaktionen erfindungsgemäss erhältlicher Verbindungen in andere Verbindungen der Formel I sind insbesondere Einführungs-, Umwandlungs- und Abspaltungsreaktionen an den Resten $R_1$ und $R_2$ sowie die Absättigung von Doppelbindungen, dargestellt durch $R_7$ und $R_8$ und/oder $R_9$ und $R_{10}$ gemeinsam zu erwähnen.

So kann man beispielsweise in verfahrensgemäss erhältlichen Verbindungen der Formel I, worin $R_7$ gemeinsam mit $R_8$ und/oder $R_9$ gemeinsam mit $R_{10}$ eine oder gegebenenfalls zwei zusätzliche Bindungen darstellen, die Doppelbindung(en) zu(r) Einfachbindung(en) absättigen, beispielsweise mittels Wasserstoff in Gegenwart eins Hydrierungskatalysators, z.B. von Palladiumkohle.

Ferner kann man unsubstituierte Carbamylniederalkylreste $R_1$ und/oder $R_2$ durch Umsetzung mit einem reaktionsfähigen Ester, wie einem Halogenwasserstoffsäure-, z.B. Chlor-, Brom- oder Jodwasserstoffsäureester, oder Sulfonsäure-, wie p-Toluolsulfonsäureester, eines Niederalkanols, Niederalkandiols, Aminoniederalkanols, N-Mono- oder N,N-Diniederalkylaminoalkanols, N,N-Alkylenamino- bzw. N,N-(Aza-, Oxa- oder Thia)alkylenaminoniederalkanols, Cycloalkanols, oder gegebenenfalls substituierten Phenylniederalkanols durch Niederalkyl, Hydroxyniederalkyl, Aminoniederalkyl, N-Mono- oder N,N-Diniederalkylaminoniederalkyl, 4-bis 8-gliedriges N,N-Alkylenamino- bzw. N,N-(Aza-, Oxa- oder Thia)alkylenaminoniederalkyl, Carbamylniederalkyl, N-Mono- oder N,N-Diniederalkylcarbamoylniederalkyl, 3- bis 8-gliedriges Cycloalkyl, unsubstituiertes oder im Phenylteil durch Niederalkyl, Niederalkoxy, Halogen und/oder Trifluormethyl substituiertes Phenylniederalkyl N-Mono- oder N,N-disubstituieren. In analoger Weise kann man auch einen durch einen der genannten Reste N-monosubstituierten Carbamylniederalkylrest N,N-disubstituieren oder einen unsubstituierten Carbamylniederalkylrest durch Umsetzung mit einem reaktiven Diester eines Alkandiols bzw. (Aza-, Oxa- oder Thia)alkandiols in den entsprechenden durch 3-bis 7-gliedriges Alkylen bzw. Aza-, Oxa- oder Thiaalkylen N,N-disubstituiertes Carbamylniederalkylrest überführen.

Ferner kann man gegebenenfalls substituierte Carbamylniederalkylreste $R_1$ und/oder $R_2$ in üblicher Weise zu Carboxyniederalkyl hydrolysieren, beispielsweise in Gegenwart eines sauren oder basischen Mittels. Geeignete saure Mittel sind beispielsweise Mineralsäuren, wie Halongenwasserstoffsäuren, z.B. Chlor- oder Bromwasserstoffsäure, oder Sauerstoffsäuren des Schwefels oder Phosphors, z.B. Schwefel-, Phosphor- oder Polyphosphorsäure. Basische Mittel sind beispielsweise Alkalimetall- oder Erdalkalimetallhydroxide oder Carbonate, z.B. Natrium-, Kalium- oder Bariumhydroxid oder Kaliumcarbonat.

Umgekehrt kann man freie Carboxyniederalkylreste $R_1$ und/oder $R_2$ beispielsweise durch Umsetzung mit Ammoniak oder einem entsprechenden Amin, d.h. einem Mono- oder Diniederalkylamin, Mono- oder Di-(hydroxyniederalkyl)amin, 4- bis 8-gliedrigen Mono- oder Di-(N',N'-Alkylenaminiederalkyl)-, Mono- oder Di-[N',N'-(Aza-, Oxa- oder Thia)-alkylenaminoniederalkyl]amin, 3- bis 8-gliedrigen Mono- oder Di-(cycloalkylaminoniederalkyl)amin, gegebenenfalls im Phenylteil durch Niederalkyl, Niederalkoxy, Halogen und/oder Trifluormethyl substituierten Mono- oder Di-(phenylniederalkyl)amin oder 4- bis 8-gliedrigen Alkylenamin bzw. Aza-, Oxa- oder Thiaalkylenamin, erforderlichenfalls unter Dehydratisierung primär gebildeter Ammoniumsalze, beispielsweise durch Erhitzen, Behandlung mit einem wasserbindenden Mittel, wie Phosphorpentoxid oder einem organischen Carbodiimid, z.B. mit Dicyclohexylcarbodiimid, oder unter Entfernung des gebildeten Wassers durch Destillation oder azeotrope Destillation in gegebenenfalls substituiertes Carbamylniederalkyl überführen.

Zur erwähnten Auftrennung eines erfindungsgemäss erhältlichen Isomerengemisches bzw. zur Isolierung der gewünschten Komponente aus einem solchen ist insbesondere folgende zu bemerken:

Die Verbindungen der Formel I mit mindestens einem asymmetrischen C-Atom können je nach Anzahl derselben Stereoisomere, wie Enantiomere, Diastereomere bzw. Epimere bilden und in form eines reinen Stereoisomeren oder eines Stereoisomerengemisches anfallen.

Diastereomerengemische können auf Grund ihrer unterschiedlichen physikalischen Eigenschaften nach den üblichen chemischen und physikalischchemischen Trennverfahren, z.B. durch fraktionierte Kristallisation, Destillation, Chromatographie und andere Phasenverteilungsverfahren, aufgetrennt werden.

Enantiomerengemische können nach üblichen Racematspaltungsverfahren, z.B. Kristallisation aus einem optisch aktiven Lösungsmittel, Chromatographie an einer optisch aktiven stationären oder mobilen Phase, oder intermediäre Bildung einer diastereomeren Hilfsverbindung, z.B. eines diastereomeren Säureadditionssalzes, mit einer optisch aktiven Carbon- oder Sulfonsäure, z.B. mit der D- oder L-Form von Weinsäure, Di-o-toluylweinsäure, Aepfelsäure, Mandelsäure, Chinasäure oder einer Camphersulfonsäure, Trennung der Diastereomeren und Freisetzung der enantiomeren Verbindung I aufgetrennt werden. Vorzugsweise isoliert man jeweils wirksamere Enantiomere.

Die gegenseitige Ueberführung freier Verbindungen und ihrer Salze ineinander erfolgt beispielsweise, indem man eine freie Verbindung mit einer Säure bzw. ein Salz mit einer Base oder einem Salz einer anderen Säure umsetzt. Salze der neuen Verbindungen können auch zur Reinigung derselben sowie, wie erwähnt, zur Enantiomerentrennung dienen, indem man eine verfahrensgemäss erhältliche Verbindung in ein Salze überführt, dieses reinigt bzw. in die Diastereomeren auftrent und aus dem Salz dann die freie Verbindung wieder freisetzt und gewünschtenfalls in ein anderes Salz überführt.

So können erhaltene freie Verbindungen in an sich bekannter Weise in Basesalze überführt werden, z.B. durch Umsetzen einer Lösung der freien Verbindung in einem geeigneten Lösungsmittel oder Lösungsmittelgemisch mit einer der vorgenannten Basen oder mit einer Lösung davon, oder mit einem geeigneten Anionenaustauscher.

Erhaltene Basesalze können in an sich bekannter Weise in die freien Verbindungen umgewandelt werden, z.B. durch Behandeln mit einer Säure, wie einer Mineralsäure, wie Chlorwasserstoff- oder Schwefelsäure.

Amphotere Salze von basischen Verbindungen der Formel I können durch Säurebehandlung in Säureadditionssalze bzw. durch Basebehandlung in Basesalze überführt werden. Ebenso kann eine amphotere basische Verbindung aus einem ihrer Säureadditions- bzw. Basesalze durch Base- bzw. Säurebehandlung freigesetzt werden.

Die neuen Verbindungen, einschliesslich ihrer Salze können auch in Form der Hydrate erhalten werden oder das zur Kristallisation verwendete Lösungmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man einer auf beliebiger Verfahrensstufe als Zwischenprodukt erhältlichen Verbindung ausgeht und die fehlenden Verfahrenschritte ausführt oder einen Ausgangsstoff unter den Reaktionsbedingungen bildet oder in Form eines Derivats davon, gegebenenfalls eines Salzes, verwendet.

Beim Verfahren der vorliegenden Erfindung werden vorzugsweise solche Ausgangsstoffe verwendet, welche zu den eingangs als besonders wertvoll geschilderten Verbindungen führen. Neue Ausgangstoffe sowie Verfahren zu ihrer Herstellung bilden ebenfalls einen Gegenstand der vorliegenden Erfindung.

Die Erfindung betrifft ebenfalls pharmazeutische Präparite, welche Verbindungen der Formel I bzw. pharmazeutisch verwendbare Salze davon enthalten. Bei diesen handelt es sich um solche zur enteralen wie oralen oder rektalen, und parenteralen Verabreichung an Warmblüter, welche den pharmakoligischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 10—100 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungsgemäss pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten doer Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weisen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzüen, Lösungen von

geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoreingrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die vorliegende Erfindung betrifft ebenfalls die Verwendung der Verbindungen der Formeln I sowie der Salze solcher Verbindungen mit salzbildenden Eigenschaften, vorzugsweise zur Behandlung von cerebraler Leistungsinsuffizienz.

Die nachfolgenden Beispiele illustrieren die vorstehend beschriebene Erfindung; sie sollen jedoch diese in ihrem Umfang in keiner Weise einschränken. Temperaturen sind in Celsiusgraden, Drucke in mbar angegeben.

Beispiel 1

9,7 g (0,03 Mol) 1,1',5,5'-Tetrahydro-4-hydroxy-2,2'-dioxo-[3,4'-bi-2H-pyrrol]-1,1'-diessigsäuredimethylester werden in 100 ml einer konzentrierten wässrigen Ammoniaklösung gelöst und 12 Stunden bei Raumtemperatur gerührt. Anschliessend wird die rotbraune Lösung am Wasserstrahlvakuum zur Trockne eingedampf. Der erhaltene Rückstand wird in 200 ml Wasser aufgenommen und bis zu stark sauerer Reaktion mit einer konzentrierten wässrigen Lösung von Chlorwasserstoffsäure versetzt. Der ausgefallene farblose Niederschlag wird abfiltriert, mit Wasser und Aceton gewaschen und im Hochvakuum bei 50° getrocknet. Man erhält das 1,1',5,5'-Tetrahydro-4-hydroxy-2,2'-dioxo[3,4'-bi-2H-pyrrol]-1,1'-diessigsäurediamid in Form weisser Mikrokristalle von Smp. 298—300° (Zers).

Der als Ausgangsmaterial dienende 1,1',5,5'-Tetrahydro-4-hydroxy-2,2'-dioxo[3,4'-bi-2H-pyrrol]-1,1'-diessigsäuredimethylester kann z.B. wie folgt hergestellt werden:

a) 197,6 (1 Mol) Iminodiessigsäuredimethylester-hydrochlorid werden in 1000 ml Chloroform suspendiert und auf 0° gekühlt. Bei dieser Temperatur wird zuerst innerhalb von 10 Minuten 222,6 g (2,2 Mol) Triethylamin und dann innerhalb von 90 Minuten eine Lösung 150,2 g (1.1 Mol) Malonsäuremonomethylester-chlorid in 120 ml Chloroform zugetropft. Das Reaktionsgemisch wird anschliessend 4 Stunden bei Raumtemperatur gerührt und dann mit 500 ml Wasser versetzt. Die organische Phase wird abgetrennt, nacheinander mit je 500 ml 2n-Salzsäure, Wasser, gesättigter Natriumbicarbonat-Lösung und nochmals Wasser gewaschen, über Magnesiumsulfat getrocknet, filtriert und im Vakuum zur Trockne eingedampt. Der ölige Rückstand wird durch Zugabe von 400 ml Hexan unter Rühren kristallisiert. Nach dem Filtrieren, Waschen mit wenig Hexan und Trocknen im Vakuum erhält man farblose Kristalle, Smp 61—62°, von N-(2-Methoxycarbonylacetyl)-iminodiessigsäuredimethylester.

b) Zu einer Lösung von 17,1 g Natrium (0,75 Mol) in 900 ml Methanol werden 196,5 g (0,75 Mol) N-(2-Methoxycarbonylacetyl)-iminodiessigsäuredimethylester, gelöst in 200 ml Methanol, zugetropft. Das Reaktionsgemisch wird anschliessend 12 Stunden zum Rückfluss erhitzt, auf Raumtemperatur gekühlt und im Wasserstrahlvakuum zur Trockne eingedampt. Der gelbliche Rückstand wird in 500 ml Wasser gelöst, über eine Schicht Diatomerenerde filtriert und mit 160 ml halbkonzentrierter wässriger Chlorwasserstoffsäure versetzt. Die ausgefallenen Kristalle werden nach einer Stunde weiteren Rührens abfiltriert, mit Aceton gewaschen und getrocknet. Man erhält das 2,5-Dihydro-4-hydroxy-3-methoxycarbonyl-2-oxo-1H-pyrrol-1-essigsäuremethylester vom Smp. 192° (Zers.).

c) 75,5 g (0,165 Mol) des vorstehend beschriebenen Pyrrolderivaten werden in 75 ml Wasser und 750 ml Acetonitril suspendiert. Beim Aufheizen unter Rühren auf 65—70° setzt die Kohlendioxidabspaltung ein und es bildet sich eine klare Lösung, die nach 30 Minuten im Wasserstrahl-Vakuum eingedampft wird. Der ölige Rückstand wird in 500 ml Wasser aufgenommen und auf 90—95° erwärmt. Das ausgefallene Produkt wird abfiltriert, mit Wasser gewaschen und im Hochvakuum getrocknet. Man erhält das 1,1',5,5'-Tetrahydro-4-hydroxy-2,2'-dioxo[3,4'-bi-2H-pyrrol]-1,1'-diessigsäuredimethylester in Form farbloser Kristalle vom Smp. 228° (Zers.).

Beispiel 2

6.5 g (0,02 Mol) 1,1',5,5'-Tetrahydro-4-hydroxy-2,2'-dioxo[3,4'-bi-2H-pyrrol]-1,1'-diessigsäuredimethylester werden in 30 ml 2-Phenylethylamin 12 Stunden bei 110° gerührt. Anschliessend wird die allmählich gebildete Suspension auf Raumtemperatur abgekühlt, auf 250 ml Wasser gegossen, mit einer konzentrierten wässrigen Chlorwasserstofflösung stark sauer gestellt und abgesaugt. Das Nutschgut wird mehrmals mit Wasser gewaschen, in 150 ml Aceton kurz bei Siedetemperatur gerührt, abgekühlt, abfiltriert und im Hochvakuum getrocknet. Man erhält das 1,1',5,5'-Tetrahydro-4-hydroxy-2,2'-dioxo[3,4'-bi-2H-pyrrol]-1,1'-diessigsäuredi-N-(2-phenylethyl)mit in Form weisser Mikrokristalle vom Smp. 250° (Zers.).

Beispiel 3

6.5 g (0,02 Mol) 1,1',5,5'-Tetrahydro-4-hydroxy-2,2'-dioxo-[3,4'-bi-2H-pyrrol]-1,1'-diessigsäuredimethylester werden in 100 ml Wasser und 20 ml konzentrierter wässriger Natrolauge 12 Stunden bei 90° gerührt. Anschliessend wird die Lösung unter Kühlung mit einem Eiswasserbad mit konzentrierter wässriger Chlorwasserstoffsaurelösung stark sauer gestellt. Die gebildete Suspension wird noch 2 Stunden ausgerührt, abfiltriert, mit Wasser und Aceton gewaschen und im Hochvakuum getrocknet. Man erhält die 1,1',5,5'-Tetrahydro-4-hydroxy-2,2'-dioxo[3,4'-bi-2H-pyrrol]-1,1'-diessigsäure in Form weisser Mikrokristalle vom Smp. 268° (Zer.).

Beispiel 4

4.9 g (0.015 Mol) 1,1',5,5'-Tetrahydro-4-hydroxy-2,2'-dioxo[3,4'-bi-2H-pyrrol]-1,1'-diessigsäuredimethylester werden in 30 ml 2-Diisopropylaminoethylamin 12 Stunden auf 105° erhitzt. Das Reaktionsgemisch wird abgekühlt und im Wasserstrahlvakuum eingeengt. Der ölige Rückstand wird in 150 ml Diethylether aufgenommen und mit einem Ueberschuss einer etherischen Lösung von Chlorwasserstoffsäure (ca. 4n) stark sauer gestellt. Die Lösung, in der sich ein harziger Niederschlag bildet, wird erneut im Wasserstrahlvakuum eingeengt. Der hygroskopische Rückstand wird in 100 ml Wasser gelöst, die Lösung mit 2n-Natronlauge alkalisch gestellt und mit 100 ml Diethylether versetzt. Nach dem Abtrennen der wässrigen Phase scheiden sich in der etherischen Phase weise Kristalle ab, die abfiltriert und im Vakuum getrocknet werden. Man erhält das 2-Diisopropylaminoethylammonium-Salz des 1,1',5,5'-Tetrahydro-4-hydroxy-2,2'-dioxo[3,4'-bi-2H-pyrrol]-1,1'-diessigsäuredi-N-(2-diisopropylaminoethyl)amid in Form farblosser Kristalle von Smp. 163—164°.

Beispiel 5

Analog zu der in Beispiel 2 beschriebenen Verfahrensweise erhält man aus 4,8 g (0,015 Mol) 1,1',5,5'-Tetrahydro-4-hydroxy-2,2'-dioxo[3,4'-bi-2H-pyrrol]-1,1'-diessigsäuredimethylester und 25 ml Piperidin das 1,1',5,5'-Tetrahydro-4-hydroxy-2,2'-dioxo-[3,4'-bi-2H-pyrrol]-1,1'-diessigsäuredipiperidid in Form blassgelber Mikrokristalle vom Smp. 150—155° (Zers.).

Beispiel 6

Analog zu der in Beispiel 2 beschriebenen Verfahrensweise erhält man aus 4,8 g (0,015 Mol) 1,1',5,5'-Tetrahydro-4-hydroxy-2,2'-dioxo-[3,4'-bi-2H-pyrrol]-1,1'-diessigsäuredimethylester und 25 ml Cyclohexylamin das 1,1',5,5'-Tetrahydro-4-hydroxy-2,2'-dioxo-[3,4'-bi-2H-pyrrol]-1,1'-diessigsäuredi-N-cyclohexylamid in Form weisser Kristalle vom Smp. 257° (Zers.).

Beispiel 7

Analog zu der in Beispiel 2 beschriebenen Verfahrensweise erhält man aus 4,8 g (0,015 Mol) 1,1',5,5'-Tetrahydro-4-hydroxy-2,2'-dioxo-[3,4'-bi-2H-pyrrol]-1,1'-diessigsäuredimethylester und 25 ml Morpholin das 1,1',5,5'-Tetrahydro-4-hydroxy-2,2'-dioxo-[3,4'-bi-2H-pyrrol]-1,1'-diessigsäuredimorpholid in Form blassgelber Kristalle vom Smp. 250° (Zers.).

Beispiel 8

Analog zu der in Beispiel 2 beschriebenen Verfahrensweise erhält man aus 6,4 g (0,02 Mol) 1,1',5,5'-Tetrahydro-4-hydroxy-2,2'-dioxo-[3,4'-bi-2H-pyrrol]-1,1'-diessigsäuredimethylester und 50 ml einer 70%-igen wässrigen Ethylaminolösung nach 48-stündigem Rühren bei Raumtemperatur das 1,1',5,5'-Tetrahydro-4-hydroxy-2,2'-dioxo-[3,4'-bi-2H-pyrrol]-1,1'-diessigsäuredi-N-ethyl-amid in Form blassgelber Kristalle vom Smp. 190° (Zers.).

Beispiel 9

Analog zu der in Beispiel 2 beschriebenen Verfahrensweise erhält man aus 4,8 g (0,015 Mol) 1,1',5,5'-Tetrahydro-4-hydroxy-2,2'-dioxo-[3,4'-bi-2H-pyrrol]-1,1'-diessigsäuredimethylester und 20 ml 2-(3,4-Dimethoxyphenyl)ethylamin das 1,1',5,5'-Tetrahydro-4-hydroxy-2,2'-dioxo-[3,4'-bi-2H-pyrrol]-1,1'-diessigsäuredi-N-[2-(3,4-dimethoxyphenyl)ethyl]amid in Form weisser Mikrokristalle vom Smp. 199° (Zers.).

Beispiel 10

Analog zu der in Beispiel 2 beschriebenen Verfahrensweise erhält man aus 4,8 g (0,015 Mol) 1,1',5,5'-Tetrahydro-4-hydroxy-2,2'-dioxo-[3,4'-bi-2H-pyrrol]-1,1'-diessigsäuredimethyl-ester und 12 ml 2,2-Dimethylpropylamin das 1,1',5,5'-Tetrahydro-4-hydroxy-2,2'-dioxo-[3,4'-bi-2H-pyrrol]-1,1'-diessigsäuredi-N-(2,2-dimethylpropyl)amid in Form weisser Mikrokristalle vom Smp. 242° (Zers.).

Beispiel 11

Analog zu der in Beispiel 2 beschriebenen Verfahrensweise erhält man aus 4,8 g (0,015 Mol) 1,1',5,5'-Tetrahydro-4-hydroxy-2,2'-dioxo-[3,4'-bi-2H-pyrrol]-1,1'-diessigsäurediethyl-ester und 40 ml einer 70%-igen wässrigen Lösung von 3-Methoxypropylamin nach 48-stündigem Rühren bei Raumtemperatur das 1,1',5,5'-Tetrahydro-4-hydroxy-2,2'-dioxo-[3,4'-bi-2H-pyrrol]-1,1'-diessigsäuredi-N-(3-methoxypropyl)amid in Form blass gelblicher Mikrokristalle vom Smp. 214° (Zers.).

Beispiel 12

4,9 g (0,015 Mol) 1,1',5,5'-Tetrahydro-4-hydroxy-2,2'-dioxo[3,4'-bi-2H-pyrrol]-1,1'-diessigsäure-dimethylester werden in 40 ml einer 70%-igen wässrigen Lösung von 2-(N-Methylamino)ethanol 48 Stunden bei Raumtemperatur gerührt. Das Reaktionsgemisch wird im Hochvakuum eingedampft, der ölige Rückstand in 20 ml Methanol aufgenommen und mit 5 ml einer 30%-igen Natriummethylatlöslung versetzt. Durch Zugabe von 200 ml Aceton wird das Natriumsalz von 1,1',5,5'-Tetrahydro-4-hydroxy-2,2'-dioxo-[3,4'-bi-2H-pyrrol]-1,1'-diessigsäuredi-N-methyl-(2-hydroxyethyl)-amid zur Kristallisation gebracht. Nach dem Abfiltrieren, Waschen und Trocknen im Hochvakuum erhält man dieses in Form blass hellbrauner Mikrokristalle vom Smp. 158° (Zers.).

Beispiel 13

2,9 g (0,01 Mol) 1,1',5,5'-Tetrahydro-4-hydroxy-2,2'-dioxo-[3,4'-bi-2H-pyrrol]-1,1'-diessigsäurediamid werden in 50 ml Wasser suspendiert und bei Raumtemperatur 2 ml einer konzentrierten wässrigen Ammoniaklösung versetzt. Die entstandene gelbliche Lösung wird im Wasserstrahlvakuum zur Trockne eingedampft, der amorphe Rückstand in 30 ml Aceton aufgenommen, filtriert, das Nutschgut mit Aceton nachgewaschen und im Hochvakuum getrocknet. Man erhält das Ammoniumsalz von 1,1',5,5'-Tetrahydro-4-hydroxy-2,2'-dioxo-[3,4'-bi-2H-pyrrol]-1,1'-diessigsäurediamid in Form eines blass gelblichen Pulvers; Smp 133° (Zers.).

Beispiel 14

In analoger Weise wie in Beispiel 13 beschrieben erhält man aus 2,9 g (0,01 Mol) 1,1',5,5'-Tetrahydro-4-hydroxy-2,2'-dioxo-[3,4'-bi-2H-pyrrol]-1,1'-diessigsäurediamid und 0,1-n wässriger Natronlauge (bis pH 6,4 zugegeben) das Natriumsalz von 1,1',5,5'-Tetrahydro-4-hydroxy-2,2'-dioxo-[3,4'-bi-2H-pyrrol]-1,1'-diessigsäurediamid in Form eines weissen Pulvers; Smp. 150° (Zers.).

Beispiel 15

In analoger Weise wie in Beispiel 13 erhält man aus 2,9 g (0,01 Mol) 1,1',5,5'-Tetrahydro-4-hydroxy-2,2'-dioxo-[3,4'-bi-2H-pyrrol]-1,1'-diessigsäurediamid und einer wässrigen Diethanolaminlösung (bis pH 6,5 zugegeben) das Diethanolammoniumsalz von 1,1',5,5'-Tetrahydro-4-hydroxy-2,2'-dioxo-[3,4'-bi-2H-pyrrol]-1,1'-diessigsäurediamid in Form eines weissen Pulvers; Smp. 130° (Zers.).

Beispiel 16

In analoger Weise wie in Beispiel 13 beschrieben erhält man aus 2,9 g (0,01 Mol) 1,1',5,5'-Tetrahydro-4-hydroxy-2,2'-dioxo-[3,4'-bi-2H-pyrrol]-1,1'-diessigsäurediamid und 5 ml 2-Dimethylaminoethanol, das N,N-Dimethyl-N-ethanol-ammoniumsalz von 1,1',5,5'-Tetrahydro-4-hydroxy-2,2'-dioxo-[3,4'-bi-2H-pyrrol]-1,1'-diessigsäurediamid in Form eines weissen Pulvers; Smp. 170° (Zers.).

Beispiel 17

2,1 g (0,007 Mol) 1,1',5,5'-Tetrahydro-4-hydroxy-2,2'-dioxo-[3,4'-bi-2H-pyrrol]-1,1'-diessigsäure werden in 30 ml Methanol suspendiert und mit 6 ml einer 30%-igen Natriummethanolatlösung in Methanol bei Raumtemperatur versetzt. Nach 3 Stunden Rühren wird von wenig Ungelösten abfiltriert und das Filtrat mit 50 ml Diethylether versetzt. Die ausgefallenen Kristalle werden abfiltriert, mit einem Ether/Methanolgemisch (2:1) kurz gewaschen und im Vakuum getrocknet. Man erhält das Trinatriumsalz von 1,1',5,5'-Tetrahydro-4-hydroxy-2,2'-dioxo-[3,4'-bi-2H-pyrrol]-1,1'-diessigsäure in Form weisser Mikrokristalle; Smp. >300° (Zers.).

Beispiel 18

Analog zu der in Beispiel 1 beschriebenen Verfahrensweise erhält man aus 25,7 g (0,1 Mol) 3-Ethoxycarbonyl-2,5-dihydro-4-hydroxy-2,2'-dioxo-1H-pyrrol-1-essigsäureethylester durch Behandeln in Wasser und Acetonitril den 1,1',5,5'-Tetrahydro-4-hydroxy-2,2'-dioxo-[3,4'-bi-2H-pyrrol]-1,1'-diessigsäurediethylester in Form blassgelber Kristalle vom Smp. 192—194°.

Dieser kann in analoger Weise wie in Bespiel 1 beschrieben in das 1,1',5,5'-Tetrahydro-4-hydroxy-2,2'-dioxo-[3,4'-bi-2H-pyrrol]-1,1'-diessigsäurediamid von Smp. 298—300° (Zers.) oder in analogue Weise wie in Beispiel 3 beschrieben in die 1,1',5,5'-Tetrahydro-4-hydroxy-2,2'-dioxo-[3,4'-bi-2H-pyrrol]-1,1'-diessigsäure vom Smp 268° (Zers.) überführt werden.

Beispiel 19

Analog zu der in Beispiel 1 beschriebenen Verfahrensweise erhält man aus 7,7 g (0,022 Mol) 1,1',5,5'-Tetrahydro-4-hydroxy-2,2'-dioxo-[3,4'-bi-2H-pyrrol]-1,1'-di(3-propionsäure)dimethylester durch Behandlung in 70 ml konzentrierter wässriger Ammoniaklösung das 1,1',5,5'-Tetrahydro-4-hydroxy-2,2'-dioxo-[3,4'-bi-2H-pyrrol]-1,1'-di(3-propionsäure)diamid in Form weisser Kristalle vom Smp. 230° (Zers.).

Der als Ausgangsmaterial dienende 1,1',5,5'-Tetrahydro-4-hydroxy-2,2'-dioxo-[3,4'-bi-2H-pyrrol]-1,1'-di(3-propionsäure)dimethylester kann, ebenfalls in Analogie zur Verfahrensweise von Beispiel 1, wie folgt hergestellt werden:

a) 98,2 g (0,77 Mol) N-(2-Cyanoethyl)glycin werden in 550 ml Methanol suspendiert und in einem Eis/Wasser-Bad auf 5° gekühlt. Bei dieser Temperatur wird unter Kühlung 3 Stunden lang ein Strom Chlorwasserstoff eingeleitet. Anschliessend wird das Reaktionsgemisch 12 Stunden bei Raumtemperatur gerührt und noch 1 Stunde zum Rückfluss erhitzt. Die farblose Suspension wird auf etwa 2/3 eingeengt und filtriert, der Rückstand wird mit wenig kaltem Methanol nachgewaschen, das Filtrat mit Natriumbicarbonat neutralisiert und zur Trockne eingedampft. Der ölige Rückstand wird in 250 ml Chloroform aufgenommen und mit Wasser ausgeschüttelt. Die organische Phase wird über Magnesiumsulfat getrocknet, zur Trockne eingedampft und der erhaltene Rückstand unter stark vermindertem Druck destilliert. Man erhält den N-(Methoxycarbonylmethyl)-β-aminopropionsäuremethylester als farbloses Oel, Sdp. 73—75° (0.005 mm Hg = $6,25 \times 10^{-6}$Bar).

b) Analog zu der in Beispiel 1a) beschriebenen Verfahrensweise erhält man aus 35.0 g (0,2 Mol) N-Methoxycarbonylmethyl)-β-amino-propionsäuremethylester, 33 ml Triethylamin (0,24 Mol) und 36.2 g (0,24 Mol) Malonsäuremonomethylester-chlorid in 60 ml Methylenchlorid N-Methoxycarbonylacetyl-N-methoxycarbonylmethyl-β-amino-propionsäuremethylester als gelbliches Oel.

c) Analog zu der in Beispiel 1b) beschriebenen Verfahrensweise erhält man aus 53.2 g (0,19 Mol) N-Methoxycarbonylacetyl-N-methoxycarbonyl-methyl-β-amino-propionsäuremethylester in 55 ml Methanol und 4,4 g Natrium in 240 ml Methanol 3-[2,5-Dihydro-4-hydroxy-3-methoxycarbonyl-2-oxo-1H-pyrrol]-propionsäuremethylester von Smp. 170—171°.

d) Analog zu der in Beispiel 1c) beschriebenen Verfahrensweise erhält man aus 24.3 g (0,1 Mol) des vorstehend beschriebenen Pyrroliderivaten in 24 ml Wasser und 240 ml Acetonitril 1,1',5,5'-Tetrahydro-4-hydroxy-2,2'-dioxo-[3,4'-bi-2H-pyrrol]-1,1'-di(3-propionsäure)dimethylester in Form weisser Kristalle vom Smp. 183—184°.

Beispiel 20

Analog zu der in Beispiel 3 beschriebenen Verfahrensweise erhält man aus 7.8 g (0,022 Mol) 1,1',5,5'-Tetrahydro-4-hydroxy-2,2'-dioxo-[3,4'-bi-2H-pyrrol]-1,1'-di(3-propionsäure)dimethylester in 100 ml Wasser und 20 ml konzentrierter wässriger Natronlauge das 1,1',5,5'-Tetrahydro-4-hydroxy-2,2'-dioxo-[3,4'-bi-2H-pyrrol]-1,1'-di(3-propionsäure) in Form farbloser Kristalle vom Smp. 227° (Zers.).

Beispiel 21

4.4 g (0,015 Mol) 1,1',5,5'-Tetrahydro-4-hydroxy-2,2'-dioxo-[3,4'-bi-2H-pyrrol]-1,1'-diessigsäure werden zusammen mit 3,3 g (0.030 Mol) Glycinamid-Hydrochlorid in 80 ml Dimethylformamid suspendiert. Anschliessend werden bei Raumtemperatur zuerst 6,8 ml (0.05 Mol) Triethylamin und dann 8,1 ml (0.03 Mol) Triphenylphosphit zugetropft; die resultierende leicht gelbe, dünnflüssige Suspension wird 3 Stunden bei 85—90° gerührt. Die entstandene braune Lösung wird auf 5° gekühlt und mit 160 ml Diethylether versetzt. Der gebildete Niederschlag wird abfiltriert, mit Diethylether gewaschen, in 80 ml Ethanol suspendiert und mit einem Ueberschuss einer etwa 4n-Chlorwasserstoffsäurelösung in Diethylether angesäuert. Nach gutem Durchrühren wird erneut filtriert und das Nutschgut 12 Stunden bei Raumtemperatur mit 90 ml Wasser und 9 ml 4n-Salzsäure verrührt. Die Suspension wird filtriert, der Filtrationsrückstand mit Wasser und Aceton gewaschen und bei 60° im Hochvakuum getrocknet. Man erhält das 1,1',5,5'-Tetrahydro-4-hydroxy-2,2'-dioxo-[3,4'-bi-2H-pyrrol]-1,1'-diessigsäuredi-N-(carbamylmethyl)amid in Form weisser Kristalle von Smp. 215° (Zers.).

Beispiel 22

Analog zu der in Beispiel 21 beschriebenen Verfahrensweise erhält man aus 4,4 g (0.015 Mol) 1,1',5,5'-Tetrahydro-4-hydroxy-2,2'-dioxo-[3,4'-bi-2H-pyrrol]-1,1'-diessigsäure, 6,6 g (0.03 Mol) 1,2,5,6-Tetrahydro-pyridin-3-carbonsäuremethylester-hydrobromid, 6,8 ml Triethylamin und 9,3 g Triphenylphosphit in 80 ml Dimethylformamid das 1,1',5,5'-Tetrahydro-4-hydroxy-2,2'-dioxo-[3,4'-bi-2H-pyrrol]-1,1'-diessigsäuredi-N,N-[1,5-(2-methoxycarbonyl)pent-2-enylen]amid in Form weisser Kristalle. Smp. 208° (Zers).

Beispiel 23

Analog zu der in Beispiel 21 beschriebenen Verfahrensweise erhält man aus 3,3 g (0.011 Mol) 1,1',5,5'-Tetrahydro-4-hydroxy-2,2'-dioxo-[3,4'-bi-2H-pyrrol]-1,1'-diessigsäure, 4,1 g Adamantylaminohydrochlorid (0.022 Mol), 5,1 ml Triethylamin und 6,8 g Triphenylphosphit in 80 ml Dimethylformamid das 1,1',5,5'-Tetrahydro-4-hydroxy-2,2'-dioxo-[3,4'-bi-2H-pyrrol]-1,1'-diessigsäuredi-N-adamantylamid in Form weisser Kristalle von Smp. 220° (Zers.).

Beispiel 24

Analog zu der in Beispiel 21 beschriebenen Verfahrensweise erhält man aus 3,8 g (0.013 Mol) 1,1',5,5'-Tetrahydro-4-hydroxy-2,2'-dioxo-[3,4'-bi-2H-pyrrol]-1,1'-diessigsäure, 3,3 g (0.026 Mol) N-Acetylpiperazin, 1,8 ml Triethylamin und 8,1 g Triphenylphosphit in 80 ml Dimethylformamid das 1,1',5,5'-Tetrahydro-4-hydroxy-2,2'-dioxo-[3,4'-bi-2H-pyrrol]-1,1'-diessigsäuredi-(4-acetyl)piperazid in Form weisser Kristalle von Smp. 220° (Zers.).

Beispiel 25

Analog zu der in Beispiel 21 beschriebenen Verfahrensweise erhält man aus 4,5 g (0.015 Mol) 1,1',5,5'-Tetrahydro-4-hydroxy-2,2'-dioxo-[3,4'-bi-2H-pyrrol]-1,1'-diessigsäure, 5,0 g (0.05 Mol) N-Methylpiperazin und 9,3 g Triphenylphosphit in 80 ml Dimethylformamid das 1,1',5,5'-Tetrahydro-4-hydroxy-2,2'-dioxo-[3,4'-bi-2H-pyrrol]-1,1'-diessigsäuredi-(4-methyl)piperazid-dihydrochlorid in Form weisser Kristalle von Smp. 263° (Zers.).

Beispiel 26

3,0 g (0.010 Mol) 1,1',5,5'-Tetrahydro-4-hydroxy-2,2'-dioxo-[3,4'-bi-2H-pyrrol]-1,1'-diessigsäurediamid werden in 70 ml Methanol suspendiert, mit 2,4 g Tris(hydroxymethyl)-amino-methan versetzt und 12 Stunden bei Raumtemperatur gerührt. Die freie Säure geht dabei in Lösung und fällt als Salz wieder aus. Der gebildete Niederschlag wird abfiltriert, mit kaltem Methanol gewaschen und unter stark vermindertem Druck bei 60° getrocknet. Man erhält das Tris(hydroxymethyl)methylammoniumsalz von 1,1',5,5'-Tetrahydro-4-hydroxy-2,2'-dioxo-[3,4'-bi-2H-pyrrol]-1,1'-diessigsäurediamid in Form weisser Kristalle von Smp. 203° (Zers.).

Beispiel 27

4,9 g (0.015 Mol) 1,1',5,5'-Tetrahydro-4-hydroxy-2,2'-dioxo-[3,4'-bi-2H-pyrrol]-1,1'-diessigsäure-dimethylester werden in 40 ml einer 70%-igen wässrigen Lösung von 2-Diisopropylaminoethylamin 3 Tage bei Raumtemperatur gerührt. Der nach dem Eindampfen im Hochvakuum erhaltene ölige Rückstand wird in 100 ml Aceton gelöst und mit 6 ml einer 30%-igen Lösung von Natriummethylat in Methanol versetzt. Das ausgefallene Produkt wird abfiltriert mit Aceton gewaschen und bei 70° unter stark vermindertem Druck getrocknet. Man erhält das Natriumsalz von 1,1',5,5'-Tetrahydro-4-hydroxy-2,2'-dioxo-[3,4'-bi-2H-pyrrol]-1,1'-diessigsäuredi-N-(2-di-isopropylaminoethyl)amid in Form eines blass gelbbraunen Pulvers, Smp. 215° (Zers.).

Beispiel 28

4,3 g 2,5-Dihydro-4-hydroxy-3-methoxycarbonyl-2-oxo-1H-pyrrol-1-acetamid werden in 50 ml Acetonitril und 30 ml Wasser suspendiert. Beim Aufheizen auf 75° setzt die Kohlendioxidabspaltung ein; das Reaktionsgemisch wird 12 Stunden bei dieser Temperatur gerührt. Nach dem Abkühlen wird das ausgefallene Produkt abfiltriert, mit Wasser und Aceton gewaschen und unter vermindertem Druck getrocknet. Man erhält das 1,1',5,5'-Tetrahydro-4-hydroxy-2,2'-dioxo-[3,4'-bi-2H-pyrrol]-1,1'-diessigsäurediamid in Form weisser Kristalle vom Smp. 300° (Zers.). Das Produkt ist mit dem im Beispiel 1 beschriebenen identisch.

Das Ausgangsmaterial kann z.B. folgendermassen hergestellt werden:

23 g (0.1 Mol) des im Beispiel 1 beschriebenen 2,5-Dihydro-4-hydroxy-3-methoxycarbonyl-2-oxo-1H-pyrrol-1-essigsäuremethylester werden in 200 ml einer konzentrierter wässriger Ammoniaklösung gelöst und 12 Stunden bei Raumtemperatur gerührt. Anschliessend wird die gelbliche Lösung im Wasserstrahlvakuum zur Trockne eingedampft. Der erhaltene feste Rückstand wird in 200 ml Wasser gelöst und bis zur stark sauren Reaktion mit einer konzentrierten wässrigen Lösung von Chlorwasserstoffsäure versetzt. Der ausgefallene, farblose Niederschlag wird abfiltriert, mit Wasser und Aceton gewaschen und unter vermindertem Druck bei 50° getrocknet. Man erhält das 2,5-Dihydro-4-hydroxy-3-methoxycarbonyl-2-oxo-1H-pyrrol-1-acetamid in Form weisser Kristalle vom Smp. 202° (Zers.).

Beispiel 29

3,2 g (0,01 Mol) 1,1',5,5'-Tetrahydro-4-hydroxy-2,2'-dioxo-[3,4'-bi-2H-pyrrol]-1,1'-diessigsäurediamid werden in 60 ml Methanol in Anwesenheit von 0,3 g Plantinoxid bei Raumtemperatur und unter Normaldruck hydriert. Das Reaktionsgemisch der nach Aufnahme der theoretischen Menge Wasserstoff zum Stillstand gekommenen Hydrierung wird vom Katalysator abfiltriert, zu Trockne eingedampft, der erhaltene Rückstand wird in 40 ml Ethanol kurz aufgekocht, filtriert und das Filtrat erneut zur Trockne eingedampft. Man erhält Octahydro-4-hydroxy-2,2'-dioxo-[3,4'-bi-2H-pyrrol]-diessigsäurediamid in Form eines farblosen, amorphen Pulvers vom Smp. ab ~120° (Zers.).

Beispiel 30

Analog zu der in Beispiel 29 beschriebenen Verfahrensweise erhält man aus 6 g (0,02 Mol) 1,1',5,5'-Tetrahydro-4-hydroxy-2,2'-dioxo-[3,4'-bi-2H-pyrrol]-1,1'-diessigsäure Octahydro-4-hydroxy-2,2'-dioxo-[3,4'-bi-2H-pyrrol]-1,1'-diessigsäure in Form eines farblosen, amorphen Pulvers.

Beispiel 31

In analoger Weise wie in den Beispielen 1—28 beschrieben kann man ferner 1,1',5,5'-Tetrahydro-4-hydroxy-2,2'-dioxo-[3,4'-bi-2H-pyrrol]-1,1'-diessigsäuredi(*cis*-2,6-dimethyl)-piperazid, 1,1',5,5'-Tetrahydro-

4-hydroxy-2,2'-dioxo-[3,4'-bi-2H-pyrrol]-1,1'-diessigsäuredi(2-tetramethylen)piperidid, 1,1',5,5'-Tetrahydro-4-hydroxy-2,2'-dioxo-[3,4'-bi-2H-pyrrol-1,1'-diessigsäuredi(3-methoxycarbonyl-4-oxo)-piperidid und 1,1',5,5'-Tetrahydro-4-hydroxy-5,5'-di(tetramethylen)-2,2'-dioxo-[3,4'-bi-2H-pyrrol-1-1'-diessigsäurediamid herstellen.

Beispiel 32

Tabletten, enthaltend 25 mg Wirkstoff, z.B. 1,1',5,5'-Tetrahydro-4-hydroxy-2,2'-dioxo-[3,4'-bi-2H-pyrrol]-1,1'-diessigsäurediamid oder ein Salz, z.B. das Natriumsalz, davon, können folgendermassen hergestellt werden:

Bestandteile (für 1000 Tabletten)

| | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb von 0,6 mm Maschenweite getrieben. Dann wird der Wirkstoff, die Lactose, des Talkum, des Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 33

Tabletten, enthaltend 10 mg Wirkstoff, z.B. 1,1',5,5'-Tetrahydro-4-hydroxy-2,2'-dioxo-[3,4'-bi-2H-pyrrol]-1,1'-diessigsäurediamid oder ein Salz, z.B. das Natriumsalz, davon, können folgendermassen hergestellt werden:

Bestandteile (für 1000 Tabletten)

| | |
|---|---|
| Wirkstoff | 10,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 34

Tabletten, enthaltend 5 mg Wirkstoff, z.B. 1,1',5,5'-Tetrahydro-4-hydroxy-2,2'-dioxo-[3,4'-bi-2H-pyrrol]-1,1'-diessigsäurediamid oder ein Salz, z.B. das Natriumsalz, davon, können folgendermassen hergestellt werden:

Bestandteile (für 1000 Tabletten)

| | |
|---|---|
| Wirkstoff | 5,0 g |
| Lactose | 150,7 g |
| Weizenstärke | 27,5 g |
| Polyethylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer

siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 34

In analoger Weise wie in den Beispielen 32—34 beschrieben können auch pharmazeutische Präparate, enthaltend eine andere Verbindung der Formel I gemäss den Beispielen 1—31 oder ein Salz davon, hergestellt werden.

**Patentansprüche für die Vertragsstaaten: BE CH DE FR GB IT LI LU NL SE**

1. Neue substituierte Tetrahydro-, Hexahydro,-bzw. Octahydro-[3,4'-bi-2H-pyrrol]-2,2'-dione der Formel

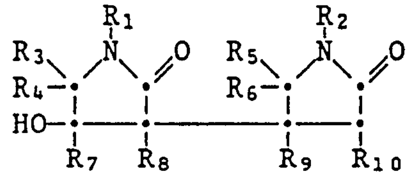

(I),

worin $R_1$ und $R_2$ unabhängig voneinander Carboxy-$C_1$—$C_4$-alkyl, Carbamyl-$C_1$—$C_4$-alkyl, N-$C_1$—$C_7$- oder N,N-di-$C_1$—$C_4$-alkylcarbamyl-$C_1$—$C_4$-alkyl, die Hydroxy-, Alkoxy-, Amino-, Alkylamino-, Dialkylamino-, Alkylen- bzw. Alkenylen- oder Aza-, Oxa- oder Thiaalkylenaminogruppe(n) in höherer als der α-Stellung zum Carbamyl-Stickstoffatom tragendes N-Mono- oder N,N-Di(hydroxy-$C_2$—$C_4$-alkyl)carbamyl-$C_1$—$C_4$-alkyl, N-(—$C_1$—$C_4$-Alkoxy-$C_2$—$C_4$-alkyl)carbamyl-$C_1$—$C_4$-alkyl, N-Mono oder N,N-Di(amino-$C_2$—$C_4$-alkyl)-carbamyl-$C_1$—$C_4$-alkyl, N-(—$C_1$—$C_7$-Alkylamino-$C_2$—$C_4$-alkyl)- oder N-(Di-$C_1$—$C_4$-alkylamino-$C_2$—$C_4$-alkyl)-carbamyl-$C_1$—$C_4$-alkyl, N-(Alkylenamino-$C_2$—$C_4$-alkyl)carbamyl-$C_1$—$C_4$-alkyl, N-(Alkenylenamino-$C_2$—$C_4$-alkyl)carbamyl-$C_1$—$C_4$-alkyl, N-[(Aza)alkylenamino-$C_2$—$C_4$-alkyl]carbamyl-$C_1$—$C_4$-alkyl, N-[(Oxa)-alkylenamino-$C_2$—$C_4$-alkyl]-carbamyl-$C_1$—$C_4$-alkyl bzw. N-[(Thia)alkylenamino-$C_2$—$C_4$-alkyl]-carbamyl-$C_1$—$C_4$-alkyl, wobei der Alkylenamino-, Alkenylenamino-, (Aza)alkylenamino-, (Oxa)alkylenamino- bzw. (Thia)alkylenaminorest einschliesslich der Aminogruppe 4 bis 8 Ringglieder enthält und Alkylenamino durch $C_1$—$C_4$-Alkoxycarbonyl sowie durch Oxo oder Hydroxy, Alkenylenamino durch $C_1$—$C_4$-Alkoxycarbonyl und gegebenenfalls zusätzlich durch Hydroxy C-substituiert und (Aza)alkylenamino durch $C_1$—$C_4$-Alkyl C- oder N'- und/oder durch $C_1$—$C_7$-Alkanoyl N'-substituiert sein kann, N-(Carbamyl-$C_1$—$C_4$-alkyl)-carbamyl-$C_1$—$C_4$-alkyl, N-(N'-$C_1$—$C_7$- oder N',N'-Di-$C_1$—$C_4$-alkylcarbamyl-$C_2$—$C_4$-alkyl)carbamyl-$C_1$—$C_4$-alkyl, 3-N-$C_3$—$C_8$-Cycloalkyl- oder N,N-Di-$C_3$—$C_8$-cycloalkylcarbamyl-$C_1$—$C_4$-alkyl, N-$C_7$—$C_9$-Bicycloalkylcarbamyl-$C_1$—$C_4$-alkyl, N-$C_8$—$C_{12}$-Tricycloalkylcarbamyl-$C_1$—$C_4$-alkyl, unsubstituiertes oder im Phenylteil durch $C_1$—$C_4$-Alkyl, $C_1$—$C_4$-Alkoxy, Halogen und/oder Trifluormethyl substituiertes N-Mono-oder N,N-Di(phenyl-$C_1$—$C_4$-alkyl)carbamyl-$C_1$—$C_4$-alkyl, N,N-Alkylencarbamyl-$C_1$—$C_4$-alkyl, N,N-Alkenylcarbamyl-$C_1$—$C_4$-alkyl, N,N-(Aza)alkylencarbamyl-$C_1$—$C_4$-alkyl oder N,N-(Oxa-oder Thia)alkylencarbamyl-$C_1$—$C_4$-alkyl bedeuten, wobei der Alkylenamino-, Alenylenamino-, (Aza)alkylenamino-, (Oxa)alkylenamino-bzw. (Thia)alkylenaminorest einschliesslich der Aminogruppe 4 bis 8 Ringglieder enthält und Alkylenamino durch $C_1$—$C_4$-Alkoxycarbonyl sowie durch Oxo oder Hydroxy, Alkenylenamino durch $C_1$—$C_4$-Alkoxycarbonyl und gegebenenfalls zusätzlich durch Hydroxy C-substituiert und (Aza)alkylenamino durch $C_1$—$C_4$-Alkyl C- oder N'-und/oder durch $C_1$—$C_7$-Alkanoyl N'-substituiert sein kann, und $R_3$, $R_4$, $R_5$ und $R_6$ jeweils Wasserstoff oder $C_1$—$C_7$-Alkyl bedeuten oder $R_3$ und $R_4$ und/oder $R_5$ und $R_6$ gemeinsam einen $C_4$—$C_8$-Alkylenrest darstellen und $R_7$, $R_8$, $R_9$ und $R_{10}$ jeweils für Wasserstoff stehen oder $R_7$ gemeinsam mit $R_8$ und/oder $R_9$ gemeinsam mit $R_{10}$ jeweils eine zusätzliche Bindung bedeuten, und ihre Salze, insbesondere ihre pharmazeutisch verwendbaren Salze.

2. Verbindungen der Formel I gemäss Anspruch 1, worin $R_1$ und $R_2$ unabhängig voneinander Carboxy-$C_1$—$C_4$-alkyl, Carbamyl-$C_1$—$C_4$-alkyl, N-Mono- oder N,N-Di-$C_1$—$C_4$-alkyl-Carbamyl-$C_1$—$C_4$-alkyl, N-Mono-oder N,N-Di(ω-hydroxy-$C_2$—$C_4$-alkyl)carbamyl-$C_1$—$C_4$-alkyl, N,N-(ω-Amino-$C_2$—$C_4$-alkyl)carbamyl-$C_1$—$C_4$-alkyl, N-[ω-(N',N'-Di-$C_1$—$C_4$-alkylamino)-$C_2$—$C_4$-alkyl]carbamyl-$C_1$—$C_4$-alkyl, N-(ω-Alkylenamino-$C_2$—$C_4$-alkyl)carbamyl-$C_1$—$C_4$-alkyl mit 4 bis 7 Ringgliedern, N-[ω-(3-Azaalkylenamino)-$C_2$—$C_4$-alkyl]carbamyl-$C_1$—$C_4$-alkyl mit 4 bis 7 Ringgliedern, N-[ω-(3-Oxaalkylenamino)-$C_2$—$C_4$-alkyl]carbamyl-$C_1$—$C_4$-alkyl mit 4 bis und mit 7 Ringgliedern, N-(Phenyl-$C_1$—$C_4$-alkyl)-carbamyl-$C_1$—$C_4$-alkyl, wobei der Phenylteil durch $C_1$—$C_4$-Alkyl, $C_1$—$C_4$-Alkoxy, Halogen der Atomnummer bis und mit 35 und/oder Trifluormethyl mono-oder disubstituiert sein kann, N-(α-Carbamyl-$C_1$—$C_4$-alkyl)carbamyl-$C_1$—$C_4$-alkyl, N,N-Alkylencarbamyl-$C_1$—$C_4$-alkyl mit 4 bis 7 Ringgliedern, N,N-(Azaalkylen)carbamyl-$C_1$—$C_4$-alkyl mit 4 bis 7 Ringgliedern N,N-(3-Oxaalkylen)carbamyl-$C_1$—$C_4$-alkyl mit 4 bis 7 Ringgliedern oder N,N-(3-Thiaalkylen)carbamyl-$C_1$—$C_4$-alkyl mit 4 bis 7 Ringglierdern bedeutet, $R_3$, $R_4$, $R_5$ und $R_6$ für Wasserstoff stehen oder $R_3$ und $R_5$ gleiche $C_1$—$C_4$-Alkylreste, und $R_4$ und $R_6$ Wasserstoff oder gleiche $C_1$—$C_4$-Alkylreste darstellen oder $R_3$ und $R_4$ sowie $R_5$ und $R_6$ gemeinsam jeweils gleiche $C_2$—$C_6$-Alkylenreste, bedeuten und $R_7$ gemeinsam mit $R_8$ und/oder $R_9$ gemeinsam mit $R_{10}$ jeweils eine zusätzliche Bindung bedeuten, und ihre Salze

3. Verbindungen der Formel I gemäss Anspruch 1, worin $R_1$ und $R_2$ gleich sind und Carboxy-$C_1$—$C_4$-

alkyl, Carbamyl-$C_1$—$C_4$-alkyl, N-Mono-$C_1$—$C_7$- oder N,N-Di-$C_1$—$C_4$-alkylcarbamyl-$C_1$—$C_4$-alkyl, N-(ω-Di-$C_1$—$C_4$-alkylamino-$C_2$—$C_4$-alkyl)carbamyl-$C_1$—$C_4$-alkyl, N-(ω-Hydroxy-$C_2$—$C_4$-alkyl)carbamyl-$C_1$—$C_4$-alkyl, N-(ω-$C_1$—$C_4$-Alkoxy-$C_2$—$C_4$-alkyl)carbamyl-$C_1$—$C_4$-alkyl, N-$C_5$—$C_6$-Cycloalkyl-, N-$C_8$—$C_9$-Bicycloalkyl- oder N-$C_8$—$C_{10}$-Tricycloalkylcarbamyl-$C_1$—$C_4$-alkyl, 5- oder 6-gliedriges, gegebenenfalls durch $C_1$—$C_4$-Alkoxycarbonyl, 3- und Oxo- oder Hydroxy 4-substituiertes N,N-Alkylenaminocarbonyl-$C_1$—$C_4$-alkyl, 5- oder 6-gliedriges, durch $C_1$—$C_4$-Alkoxycarbonyl, 3-substituiertes und gegebenenfalls zusätzlich durch Hydroxy 4-substituiertes Alkenylaminocarbonyl-$C_1$—$C_4$-alkyl, 5- bis 6-gliedriges, gegebenenfalls durch $C_1$—$C_4$-Alkyl oder $C_1$—$C_4$-Alkanoyl N'-substituiertes N,N-(Aza)alkylenaminocarbonyl-$C_1$—$C_4$-alkyl, 5- bis 6-gliedriges N,N-(Oxa)alkylenaminocarbonyl-$C_1$—$C_4$-alkyl, oder im Phenylteil gegebenenfalls durch $C_1$—$C_4$-Alkoxy mono- oder disubstituiertes N-Phenyl-$C_1$—$C_4$-alkylcarbamyl-$C_1$—$C_4$-alkyl, bedeuten, $R_3$, $R_4$, $R_5$ und $R_6$ Wasserstoff bedeuten und $R_7$, $R_8$, $R_9$ und $R_{10}$ jeweils für Wasserstoff stehen oder $R_7$ gemeinsam mit $R_8$ sowie $R_9$ gemeinsam mit $R_{10}$ je eine zusätzliche Bindung darstellen, und deren Salze.

4. Verbindungen der Formel I gemäss Anspruch 2, worin $R_1$ und $R_2$ gleiche Carboxy-$C_1$—$C_4$-alkyl- bzw. Carbamyl-$C_1$—$C_4$-alkylreste oder durch ω-N,N-Di-$C_1$—$C_4$-alkylamino-$C_2$—$C_4$-alkyl N-mono-substituierte, durch ω-Hydroxy-$C_2$—$C_4$-alkyl N-mono- oder N,N-disubstituierte oder durch 4-bis 6-gleidriges (3-$C_1$—$C_4$-Alkyl-3-aza)alkylen, N,N-disubstituierte Carbamylmethylreste darstellen und $R_3$, $R_4$, $R_5$ und $R_6$ für Wasserstoff stehen, und deren Salze.

5. Verbindungen gemäss einem der Ansprüche 1 und 2, worin $R_1$ und $R_2$ gleich sind, $R_3$, $R_4$, $R_5$ und $R_6$ für Wasserstoff stehen und $R_7$ gemeinsam mit $R_8$ und $R_9$ gemeinsam mit $R_{10}$ je eine zusätzliche Bindung darstellen.

6. 1,1',5,5' - Tetrahydro - 4 - hydroxy - 2,2' - dioxo-[3,4'-bi-2H-pyrrol]-1,1'-diessigsäurediamid gemäss Anspruch 1 oder ein Salz davon.

7. 1,1',5,5' - Tetrahydro - 4 - hydroxy - 2,2' - dioxo - [3,4'-bi-2H-pyrrol]-1,1'-diessigsäuredi-N-(2-phenylethyl)amid gemäss Anspruch 1 oder ein Salz davon,

8. 1,1',5,5' - Tetrahydro - 4 - hydroxy - 2,2' - dioxo - [3,4'-bi-2H-pyrrol]-1,1'-diessigsäuredi-N-(carbamylmethyl)amid gemäss Anspruch 1 oder ein Salz davon,

9. 1,1',5,5' - Tetrahydro - 4 - hydroxy - 2,2' - dioxo - [3,4'-bi-2H-pyrrol]-1,1'-diessigsäuredi-N,N-[1,5-(2-methoxycarbonyl)pent-2-enylen]amid gemäss Anspruch 1 oder ein Salz davon,

10. 1,1',5,5' - Tetrahydro - 4 - hydroxy - 2,2' - dioxo - [3,4'-bi-2H-pyrrol]-1,1'-di(3-propionsäure)diamid gemäss Anspruch 1 oder ein Salz davon,

11. 1,1',5,5'-Tetrahydro-4-hydroxy-2,2'-dioxo-[3,4'-bi-2H-pyrrol]-1,1'-diessigsäure gemäss Anspruch 1 oder ein Salz davon,

12. 1,1',5,5'-Tetrahydro-4-hydroxy-2,2'-dioxo-[3,4'-bi-2H-pyrrol]-1,1'-diessigsäuredi-N,(2-diisopropylaminoethyl)amid gemäss Anspruch 1 oder ein Salz davon,

13. 1,1',5,5'-Tetrahydro-4-hydroxy-2,2'-dioxo-[3,4'-bi-2H-pyrrol]-1,1'-diessigsäuredipiperidid gemäss Anspruch 1 oder ein Salz davon,

14. 1,1',5,5'-Tetrahydro-4-hydroxy-2,2'-dioxo-[3,4'-bi-2H-pyrrol]-1,1'-diessigsäuredi-N-cyclohexylamid gemäss Anspruch 1 oder ein Salz davon,

15. 1,1',5,5'-Tetrahydro-4-hydroxy-2,2'-dioxo-[3,4'-bi-2H-pyrrol]-1,1'-diessigsäuredimorpholid gemäss Anspruch 1 oder ein Salz davon,

16. 1,1',5,5' - Tetrahydro - 4 - hydroxy - 2,2' - dioxo - [3,4'-bi-2H-pyrrol]-1,1'-diessigsäuredi-N-ethylamid gemäss Anspruch 1 oder ein Salz davon,

17. 1,1',5,5' - Tetrahydro - 4 - hydroxy - 2,2' - dioxo -[3,4'-bi-2H-pyrrol]-1,1'-diessigsäuredi-N-[2-(3,4-dimethoxyphenyl)ethyl]amid gemäss Anspruch 1 oder ein Salz davon,

18. 1,1',5,5'-Tetrahydro-4-hydroxy-2,2'-dioxo-[3,4'-bi-2H-pyrrol]-1,1'-diessigsäuredi-N-(2,2-dimethylpropyl)amid gemäss Anspruch 1 oder ein Salz davon,

19. 1,1',5,5'-Tetrahydro-4-hydroxy-2,2'-dioxo-[3,4'-bi-2H-pyrrol]-1,1'-diessigsäuredi-N-(3-methoxypropyl)amid gemäss Anspruch 1 oder ein Salz davon, oder

20. 1,1',5,5'-Tetrahydro-4-hydroxy-2,2'-dioxo-[3,4'-bi-2H-pyrrol]-1,1'-diessigsäuredi-N-methyl-N-(2-hydroxyethyl)-amid gemäss Anspruch 1 oder ein Salz davon,

21. 9. 1,1',5,5'-Tetrahydro-4-hydroxy-2,2'-dioxo-[3,4'-bi-2H-pyrrol]-1,1'-di(3-propionsäure) gemäss Anspruch 1 oder ein Salz davon,

22. 1,1',5,5'-Tetrahydro-4-hydroxy-2,2'-dioxo-[3,4'-bi-2H-pyrrol]-1,1'-diessigsäuredi-N-adamantylamid gemäss Anspruch 1 oder ein Salz davon,

23. 1,1',5,5'-Tetrahydro-4-hydroxy-2,2'-dioxo-[3,4'-bi-2H-pyrrol]-1,1'-diessigsäuredi(4-acetyl)piperazid gemäss Anspruch 1 oder ein Salz davon,

24. Octahydro-4-hydroxy-2,2'-dioxo-[3,4'-bi-2H-pyrrol]-1,1'-diessigsäure gemäss Anspruch 1 oder ein Salz davon,

25. 1,1',5,5'-Tetrahydro-4-hydroxy-2,2'-dioxo-[3,4'-bi-2H-pyrrol]-1,1'-diessigsäuredi(4-methyl)piperazid gemäss Anspruch 1 oder ein Salz davon, oder

26. Octahydro-4-hydroxy-2,2'-dioxo-[3,4'-bi-2H-pyrrol]-1,1'-diessigsäurediamid gemäss Anspruch 1 oder ein Salz davon.

27. Das Natrium-, Kalium-, Ammonium-, Diethylammonium-, Bis(2-hydroxyethyl)ammonium-, Tris(2-hydroxyethyl)ammonium-, Tris(hydroxymethyl)methyl-ammonium- oder N,N-Dimethyl-N-(2-hydroxyethyl)-ammoniumsalz einer Verbindung gemäss einem der Ansprüche 1 bis 26.

28. Eine Verbindung gemäss der Ansprüche 1—27 in freier Form oder in Form eines pharmazeutisch verwendbaren Salzes zur Anwendung in einem Verfahren zur therapeutischen Behandlung des menschlichen oder tierischen Organismus.

29. Pharmazeutische Präparate, enthaltend eine Verbindung gemäss einem der Ansprüche 1—27 in freier Form oder in Form eines pharmazeutisch verwendbaren Salzes neben üblichen pharmazeutischen Hilfsstoffen.

30. Verfahren zur Herstellung neuer Tetrahydro-, Hexahydro- bzw. Octahydro-[3,4'-bi-2H-pyrrol]-2,2'-dione gemäss Anspruch 1, und ihrer Salze dadurch gekennzeichnet, dass man

a) eine Verbindung der Formel

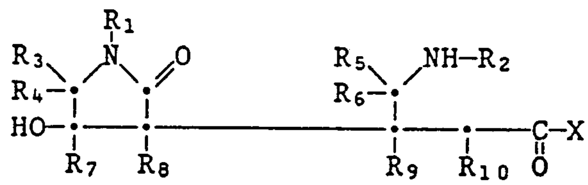

(II)

worin X gegebenenfalls verestertes oder verethertes Hydroxy bedeutet, oder ein Salz davon cyclisiert oder

b) in einer Verbindung der Formel

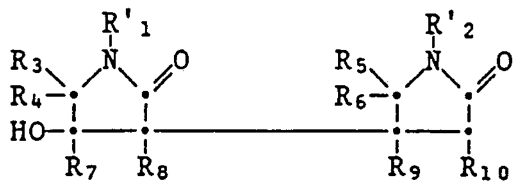

(III)

worin $R'_1$ eine in einen Rest $R_1$ überführbare Gruppe und $R'_2$ einen Rest $R_2$ oder eine in diesen überführbare Gruppe bedeutet, oder in einem Salz davon $R'_1$ in eine Gruppe $R_1$ und gegebenenfalls einem in $R_2$ überführbaren Rest $R'_2$ in eine Gruppe $R_2$ überführt oder

c) in einer Verbindung der Formel

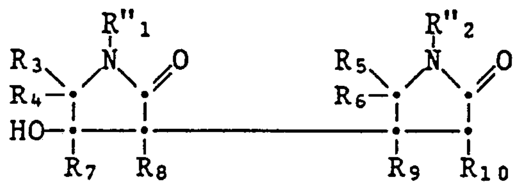

(IV)

worin $R''_1$ Wasserstoff und $R''_2$ Wasserstoff oder einen Rest $R_2$ bedeutet, oder in einem Salz davon das Wasserstoffatom $R''_1$ durch einem Rest $R_1$ und gegebenenfalls das Wasserstoffatom $R''_2$ durch einen Rest $R_2$ ersetzt oder

d) eine Verbindung der Formel

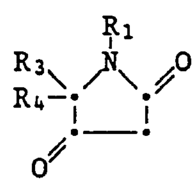

(V)

oder ein Tautomeres und/oder Salz davon mit einer Verbindung der Formel

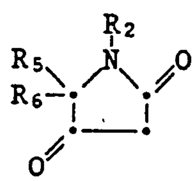

(VI)

oder einem Tautomeren davon kondensiert und jeweils gewünschtenfalls eine verfahrensgemäss erhältliche Verbindung in eine andere Verbindung der Formel I überführt, ein verfahrensgemäss erhältliches Isomerengemisch in die Isomeren auftrennt und/oder eine verfahrensgemäss erhältliche freie Verbindung in ein Salz oder ein verfahrensgemäss erhältliches Salz in die freie Verbindung oder in anderes Salz überführt.

31. Verwendung einer Verbindung gemäss einem der Ansprüche 1 bis 28 zur Herstellung eines Nootropikums.

**Patentansprüche für den Vertragsstaat: AT**

1. Verfahren zur Herstellung neuer Tetrahydro-, Hexahydro,-bzw. Octahydro-[3,4'-bi-2H-pyrrol]-2,2'-dione der Formel

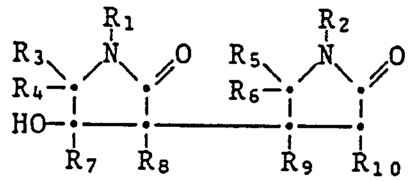

(I),

worin $R_1$ und $R_2$ unabhängig voneinander Carboxy-$C_1$—$C_4$-alkyl, Carbamyl-$C_1$—$C_4$-alkyl, N-$C_1$—$C_7$- oder N,N-di-$C_1$—$C_4$-alkylcarbamyl-$C_1$—$C_4$-alkyl, die Hydroxy-, Alkoxy-, Amino-, Alkylamino-, Dialkylamino-, Alkylen- bzw. Alkenylen- oder Aza-, Oxa- oder Thiaalkylenaminogruppe(n) in höherer als der α-Stellung zum Carbamyl-Stickstoffatom tragendes N-Mono- oder N,N-Di(hydroxy-$C_2$—$C_4$-alkyl)carbamyl-$C_1$—$C_4$-alkyl, N-(—$C_1$—$C_4$-Alkoxy-$C_2$—$C_4$-alkyl)carbamyl-$C_1$—$C_4$-alkyl, N-Mono oder N,N-Di(amino-$C_2$—$C_4$-alkyl)-carbamyl-$C_1$—$C_4$-alkyl, N-(—$C_1$—$C_7$-Alkylamino-$C_2$—$C_4$-alkyl)- oder N-(Di-$C_1$—$C_4$-alkylamino-$C_2$—$C_4$-alkyl)-carbamyl-$C_1$—$C_4$-alkyl, N-(Alkylenamino-$C_2$—$C_4$-alkyl)carbamyl-$C_1$—$C_4$-alkyl, N-(Alkenylenamino-$C_2$—$C_4$-alkyl)carbamyl-$C_1$—$C_4$-alkyl, N-(Alkenylenamino-$C_2$—$C_4$-alkyl)carbamyl-$C_1$—$C_4$-alkyl, N-[(Aza)alkylen-amino-$C_2$—$C_4$-alkyl]carbamyl-$C_1$—$C_4$-alkyl, N-[(Oxa)-alkylenamino-$C_2$—$C_4$-alkyl]-carbamyl-$C_1$—$C_4$-alkyl bzw. N-[(Thia)alkylenamino-$C_2$—$C_4$-alkyl]-carbamyl-$C_1$—$C_4$-alkyl, wobei der Alkylenamino-, Alkenylenamino-, (Aza)alkylenamino-, (Oxa)alkylenamino- bzw. (Thia)alkylenaminorest einschliesslich der Aminogruppe 4 bis 8 Ringglieder enthält und Alkylenamino durch $C_1$—$C_4$-Alkoxycarbonyl sowie durch Oxo oder Hydroxy, Alkenylenamino durch $C_1$—$C_4$-Alkoxycarbonyl und gegebenenfalls zusätzlich durch Hydroxy C-substituiert und (Aza)alkylenamino durch $C_1$—$C_4$-Alkyl C-oder N'- und/oder durch $C_1$—$C_7$-Alkanoyl N'-substituiert sein kann, N-(Carbamyl-$C_1$—$C_4$-alkyl)carbamyl-$C_1$—$C_4$-alkyl, N-(N'-$C_1$—$C_7$- oder N',N'-Di-$C_1$—$C_4$-alkylcarbamyl-$C_2$—$C_4$-alkyl)carbamyl-$C_1$—$C_4$-alkyl, 3-N-$C_3$—$C_8$-Cycloalkyl- oder N,N-Di-$C_3$—$C_8$-cyclo-alkylcarbamyl-$C_1$—$C_4$-alkyl, N-$C_7$—$C_9$-Bicycloalkylcarbamyl-$C_1$—$C_4$-alkyl, N-$C_8$—$C_{12}$-Tricycloalkylcarbamyl-$C_1$—$C_4$-alkyl, unsubstituiertes oder im Phenylteil durch $C_1$—$C_4$-Alkyl, $C_1$—$C_4$-Alkoxy, Halogen und/oder Trifluormethyl substituiertes N-Mono- oder N,N-Di(phenyl-$C_1$—$C_4$-alkyl)carbamyl-$C_1$—$C_4$-alkyl, N,N-Alkylencarbamyl-$C_1$—$C_4$-alkyl, N,N-Alkenylcarbonyl-$C_1$—$C_4$-alkyl, N,N-(Aza)alkylencarbamyl-$C_1$—$C_4$-alkyl oder N,N-(Oxa-oder Thia)alkylencarbamyl-$C_1$—$C_4$-alkyl bedeuten, wobei der Alkylenamino-, Alenylenamino-, (Aza)alkylenamino-, (Oxa)alkylenamino- bzw. (Thia)alkylenaminorest einschliesslich der Aminogruppe 4 bis 8 Ringglieder enthält und Alkylenamino durch $C_1$—$C_4$-Alkoxycarbonyl sowie durch Oxo oder Hydroxy, Alkenylenamino durch $C_1$—$C_4$-Alkoxycarbonyl und gegebenenfalls zusätzlich durch Hydroxy C-substituiert und (Aza)alkylenamino durch $C_1$—$C_4$-Alkyl C- oder N'-und/oder durch $C_1$—$C_7$-Alkanoyl N'-substituiert sein kann, und $R_3$, $R_4$, $R_5$ und $R_6$ jeweils Wasserstoff oder $C_1$—$C_7$-Alkyl bedeuten oder $R_3$ und $R_4$ und/oder $R_5$ und $R_6$ gemeinsam einen $C_4$—$C_8$-Alkylenrest darstellen und $R_7$, $R_8$, $R_9$ und $R_{10}$ jeweils für Wasserstoff stehen oder $R_7$ gemeinsam mit $R_8$ und/oder $R_9$ gemeinsam mit $R_{10}$ jeweils eine zusätzliche Bindung bedeuten, und ihrer Salze dadurch gekennzeichnet, dass man

a) eine Verbindung der Formel

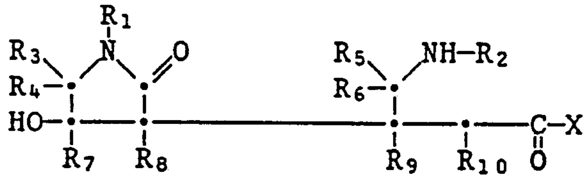

(II)

worin X gegebenenfalls verestertes oder verethertes Hydroxy bedeutet, oder ein Salz davon cyclisiert oder

b) in einer Verbindung der Formel

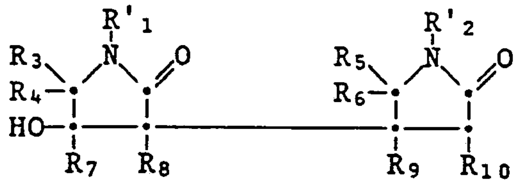

(III)

worin $R'_1$ eine in einen Rest $R_1$ überführbare Gruppe und $R'_2$ einen Rest $R_2$ oder eine in diesen überführbare Gruppe bedeutet, oder in einem Salz davon $R'_1$ in eine Gruppe $R_1$ und gegebenenfalls einem in $R_2$ überführen Rest $R'_2$ in eine Gruppe $R_2$ überführt oder

c) in einer Verbindung der Formel

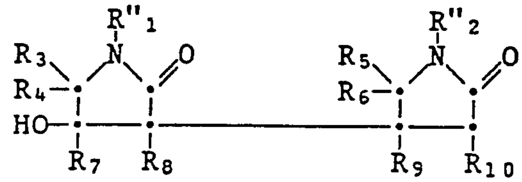

(IV)

worin $R''_1$ Wasserstoff und $R''_2$ oder einem Rest $R_2$ bedeutet, oder in einem Salz davon das Wasserstoffatom $R''_1$ durch einem Rest $R_1$ und gegebenenfalls das Wasserstoffatom $R''_2$ durch einen Rest $R_2$ ersetzt oder

d) eine Verbindung der Formel

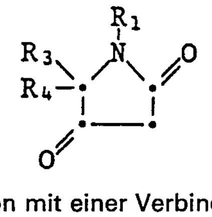

(V)

oder ein Tautomeres und/oder Salz davon mit einer Verbindung der Formel

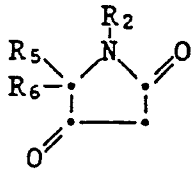

(VI)

oder einem Tautomeren davon kondensiert und jeweils gewünschtenfalls eine verfahrensegemäss erhältliche Verbindung in eine andere Verbindung der Formel I Überführt, ein verfahrensgemäss erhältliches Isomerengemisch in die Isomeren auftrennt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1 zur Herstellung von Verbindungen der Formel 1, worin $R_1$ und $R_2$ unabhängig voneinander Carboxy-$C_1$—$C_4$-alkyl, Carbamyl-$C_1$—$C_4$-alkyl, N-Mono- oder N,N-Di-$C_1$—$C_4$-alkyl-, N-mono- oder N,N-Di-(ω-hydroxy-$C_2$—$C_4$-alkyl)carbamyl-$C_1$—$C_4$-alkyl, N,N-(ω-Amino-$C_2$—$C_4$-alkyl)carbamyl-$C_1$—$C_4$-alkyl, N-[ω-(N',N'-Di-$C_1$—$C_4$-alkamino)-$C_2$—$C_4$-alkyl]carbamyl-$C_1$—$C_4$-alkyl, N-(ω-Alkylenamino-$C_2$—$C_4$-alkyl)carbamyl-$C_1$—$C_4$-alkyl mit 4 bis 7 Ringgliedern, N-[ω-(3-Azaalkylenamino)-$C_2$—$C_4$-alkyl]carbamyl-$C_1$—$C_4$-alkyl, N-[ω-(3-Oxaalkylenamino)-$C_2$—$C_4$-alkyl]carbamyl-$C_1$—$C_4$-alkyl mit 4 bis 7 Ringgliedern, N-(Phenyl-$C_1$—$C_4$-alkyl)-carbamyl-$C_1$—$C_4$-alkyl, wobei der Phenylteil durch $C_1$—$C_4$-Alkyl, $C_1$—$C_4$-Alkoxy, Halogen der Atomnummer bis und mit 35 und/oder Trifluormethyl mono- oder disubstituiert sein kann, N-(α-Carbamyl-$C_1$—$C_4$-alkyl)carbamyl-$C_1$—$C_4$-alkyl, N,N-Alkylencarbamyl-$C_1$—$C_4$-alkyl mit 4 bis 7 Ringgliedern, N,N-(Azaalkylen)carbamyl-$C_1$—$C_4$-alkyl mit 4 bis 7 Ringgliedern N,N-(3-Oxaalkylen)carbamyl-$C_1$—$C_4$-alkyl mit 4 bis 7 Ringgliedern oder N,N-(3-Thiaalkylen)carbamyl-$C_1$—$C_4$-alkyl mit 4 bis 7 Ringgliedern bedeutet, $R_3$, $R_4$, $R_5$ und $R_6$ für Wasserstoff stehen oder $R_3$ und $R_5$ gleiche $C_1$—$C_4$-Alkylreste und $R_4$ und $R_6$ Wasserstoff oder gleiche $C_1$—$C_4$-Alkylreste darstellen oder $R_3$ und $R_4$ sowie $R_5$ und $R_6$ gemeinsam jeweils gleiche $C_2$—$C_6$-Alkylenreste bedeuten und $R_7$ gemeinsam mit $R_8$ und/oder $R_9$ gemeinsam mit $R_{10}$ jeweils eine zusätzliche Bindung bedeuten, und ihre Salze

3. Verfahren gemäss Anspruch 1 zur Herstellung von Verbindungen der Formel I, worin $R_1$ und $R_2$ gleich sind und Carboxy-$C_1$—$C_4$-alkyl, Carbamyl-$C_1$—$C_4$-alkyl, N-Mono-$C_1$—$C_7$- oder N,N-Di-$C_1$—$C_4$-alkylcarbamyl-$C_1$—$C_4$-alkyl, N-(ω-Di-$C_1$—$C_4$-alkylamino-$C_2$—$C_4$-alkyl)carbamyl-$C_1$—$C_4$-alkyl, N-(ω-Hydroxy-$C_2$—$C_4$-alkyl)carbamyl-$C_1$—$C_4$-alkyl, N-(ω-$C_1$—$C_4$-Alkoxy-$C_2$—$C_4$-alkyl)carbamyl-$C_1$—$C_4$-alkyl, N-$C_5$—$C_6$-Cycloalkyl-, N-$C_8$—$C_9$-Bicycloalkyl- oder N-$C_8$—$C_{10}$-Tricycloalkylcarbamyl-$C_1$—$C_4$-alkyl, 5- oder 6-gliedriges, gegebenenfalls durch $C_1$—$C_4$-Alkoxycarbonyl, 3- und Oxo- oder Hydroxy 4-substituiertes N,N-Alkylenaminocarbonyl-$C_1$—$C_4$-alkyl, 5- oder 6-gliedriges, durch $C_1$—$C_4$-Alkoxycarbonyl, 3-substituiertes und gegebenenfalls zusätzlich durch Hydroxy 4-substituiertes Alkenylenaminocarbonyl-$C_1$—$C_4$-alkyl, 5- bis 6-gliedriges, gegebenenfalls durch $C_1$—$C_4$-Alkyl oder $C_1$—$C_4$-Alkanoyl N'-substituiertes N,N-(Aza)alkylenaminocarbonyl-$C_1$—$C_4$-alkyl, 5- bis 6-gliedriges N,N-(Oxa)alkylenaminocarbonyl-$C_1$—$C_4$-alkyl, oder im Phenylteil gegebenenfalls durch $C_1$—$C_4$-Alkoxy mono- oder disubstituiertes N-Phenyl-$C_1$—$C_4$-alkylcarbamyl-$C_1$—$C_4$-alkyl bedeuten, $R_3$, $R_4$, $R_5$ und $R_6$ Wasserstoff bedeuten und $R_7$, $R_8$, $R_9$ und $R_{10}$ jeweils für Wasserstoff stehen oder $R_7$ gemeinsam mit $R_8$ sowie $R_9$ gemeinsam mit $R_{10}$ je eine zusätzliche Bindung darstellen.

4. Verfahren gemäss Anspruch 1 zur Herstellung von Verbindungen der Formel I, worin $R_1$ und $R_2$ gleiche Carboxy-$C_1$—$C_4$-alkyl- bzw. Carbamyl-$C_1$—$C_4$-alkylreste oder durch ω-N,N-Di-$C_1$—$C_4$-alkylamino-$C_2$—$C_4$-alkyl N-mono-substituiertes, durch ω-hydroxy-$C_2$—$C_4$-alkyl, N-mono- oder N,N-disubstituierte oder

durch 4-bis 6-gleidriges (3-$C_1$—$C_4$-Alkyl-3-aza)alkylen, N,N-disubstituierte Carbamylmethylreste darstellen und $R_3$, $R_4$, $R_5$ und $R_6$ für Wasserstoff stehen, und deren Salze.

5. Verfahren gemäss Anspruch 1 zur Herstellung von Verbindungen, erhältlich gemäss Anspruch 1 oder 2, worin $R_1$ und $R_2$ gleich sind, $R_3$, $R_4$, $R_5$ und $R_6$ für Wasserstoff stehen und $R_7$ gemeinsam mit $R_8$ und $R_9$ gemeinsam mit $R_{10}$ je eine zusätzliche Bindung darstellen.

6. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäurediamid oder eines Salzes davon.

7. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-(2-phenylethyl)amid oder eines Salz davon.

8. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-(carbamylmethyl)amid oder ein Salzes davon.

9. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N,N-[1,5-(2-methoxycarbonyl)pent-2-enylen]amid

10. Verfahren gemäss Anspruch 1 zur Herstellung von 1,1',5,5'-Tetrahydro-4-hydroxy-2,2'-dioxo-[3,4'-bi-2H-pyrrol]-1,1'-di(3-propionsäure)diamid oder eines Salzes davon,

11. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäure oder eines Salzes davon.

12. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N,(2-diisopropylaminoethyl)amid oder eines Salzes davon.

13. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredipiperidid oder eines Salzes davon.

14. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-cyclohexylamid oder eines Salzes davon.

15. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredimorpholid oder eines Salzes davon.

16. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-ethylamid oder eines Salzes davon.

17. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-[2-(3,4-dimethoxyphenyl)ethyl]amid oder eines Salzes davon.

18. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-(2,2-dimethylpropyl)amid oder eines Salzes davon.

19. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-(3-methoxypropyl)amid oder eines Salzes davon.

20. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-methyl-N-(2-hydroxyethyl)-amid oder eines Salzes davon.

21. Verfahren gemäss Anspruch 1 zur Herstellung von 1,1',5,5'-Tetrahydro-4-hydroxy-2,2'-dioxo-[3,4'-bi-2H-pyrrol]-1,1'-di(3-propionsäure) oder eines Salzes davon.

22. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi-N-adamantylamid oder eines Salzes davon.

23. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi(4-acetyl)piperazid oder eines Salzes davon.

24. Verfahren gemäss Anspruch 1 zur Herstellung von Octahydro-4-hydroxy-2,2'-dioxo[3,4'-bi-2H-pyrrol]-1,1'-diessigsäure oder eines Salzes davon.

25. Verfahren gemäss Anspruch 1 zur Herstellung von 1,1',5,5'-Tetrahydro-4-hydroxy-2,2'-dioxo-[3,4'-bi-2H-pyrrol]-1,1'-diessigsäuredi(4-methyl)piperazid, oder eines Salzes davon.

26. Verfahren gemäss Anspruch 1 zur Herstellung von Octahydro-4-hydroxy-2,2'-dioxo[3,4'-bi-2H-pyrrol]-1,1'-diessigsäurediamid oder eines Salzes davon.

27. Verfahren gemäss Anspruch 1 zur Herstellung von Verbindungen, erhältlich gemäss einem der Ansprüche 1—26 in Form des Natrium-, Kalium-, Ammonium-, Diethylammonium-, Bis(2-hydroxyethyl)ammonium-, Tris(2-hydroxyethyl)ammonium-, Tris(hydroxymethyl)methyl-ammonium- oder N,N-Dimethyl-N-(2-hydroxyethyl)-ammoniumsalzes

28. Verfahren zur Herstellung pharmazeutischer Präparate, dadurch gekennzeichnet, dass man eine Verbindung, erhältlich gemäss einem der Ansprüche 1—27 in freier Form oder in Form eines pharmazeutisch verwendbaren Salzes mit üblichen pharmazeutischen Hilfsstoffen vermischt.

**Revendications pour les Etats contractants: BE CH DE FR GB IT LI LU NL SE**

1. Nouvelles tétrahydro-, hexahydro- ou octahydro- [3,4'-bi-2H-pyrrol]-2,2'-diones substituées de formule

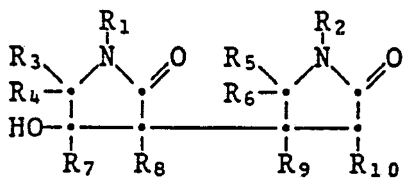

(I),

où $R_1$ et $R_2$ représentent indépendamment l'un de l'autre un carboxyalkyle en $C_1$—$C_4$, un carbamylalkyle en $C_1$—$C_4$, un N-alkyle en $C_1$—$C_7$ ou un N,N-dialkyle en -$C_1$—$C_4$ carbamylalkyle en $C_1$—$C_4$, le ou les groupe(s) hydroxy, alcoxy, amino, alylamino, dialkylamino, alkylène ou alkénylène ou aza-, oxa- ou thiaalkylène-amino en une position plus éloignée que la position α par rapport à l'atome d'azote du carbamyle portant le N-mono- ou le N,N-Di(hydroxyalkyle en $C_2$—$C_4$carbamylalkyle en $C_1$—$C_4$, un N-alcoxy en $C_1$—$C_4$ alkyle en $C_2$—$C_4$)carbamylalkyle en $C_1$—$C_4$, un N-mono- ou un N-N-di(aminoalkyle en $C_2$—$C_4$) carbamylalkyle en $C_1$—$C_4$, un N-(alkyle en $C_1$—$C_7$ aminoalkyle en $C_1$—$C_4$)- ou un N-(dialkyle $C_1$—$C_4$ aminoalkyle en $C_2$—$C_4$) carbamylalkyle en $C_1$—$C_4$, un N-(alkylèneaminoalkyle en $C_2$—$C_4$) carbamylalkyle en $C_1$—$C_4$, un N-(alkénylèneaminoalkyle en $C_2$—$C_4$) carbamylalkyle en $C_1$—$C_4$, un N-[(aza)alkylèneaminoalkyle en $C_2$—$C_4$] carbamylalkyle en $C_1$—$C_4$ un N-[(0xa)-alkylèneaminoalkyle en $C_2$—$C_4$] carbamylalkyle en $C_1$—$C_4$ ou un N-[(thia)alkylèneaminoalkyle en $C_2$—$C_4$]carbamylalkyle en $C_1$—$C_4$, les restes alkylèneamino, alkénylène-amino, (aza)alkylèneamino, (oxa)alkylèneamino ou (thia)alkylèneamino incluant le groupe amino contenant 4 à 8 termes cycliques et l'alkylèneamino pouvant être substitué en C par un alcoxy en $C_1$—$C_4$ carbonyle ainsi que par l'oxo ou l'hydroxy, l'alkénylèneamino pouvant être substitué par un alcoxy en $C_1$—$C_4$ carbonyle et éventuellement en plus par l'hydroxy et l'(aza)alkylèneamino pouvant être substitué en C ou en N' par un alkyle en $C_1$—$C_4$ et ou pouvant être substitué en N' par un alcanoyle en $C_1$—$C_7$, un N-(carbamylalkyle en $C_1$—$C_4$) carbamylalkyle en $C_1$—$C_4$, un N-(N'-alkyle en $C_1$—$C_7$ ou N',N'-dialkyle en $C_1$—$C_4$ carbamylalkyle en $C_2$—$C_4$ carbamylalkyle en $C_1$—$C_4$, un 3-N-cycloalkyle en $C_4$—$C_8$ ou le dicycloalkyle en $C_3$—$C_8$ carbamylalkyle en $C_1$—$C_4$, un N-bicycloalkyle en $C_7$—$C_9$ carbamylalkyle en $C_1$—$C_4$, un N-tricyclo-alkyle en $C_8$—$C_{12}$ carbamylalkyle en $C_1$—$C_4$, un N-mono- ou N,N-di(phénylalkyle en $C_1$—$C_4$) carbamylalkyle en $C_1$—$C_4$ non substitué ou substitué dans la partie phényle par un alkyle en $C_1$—$C_4$, par un alcoxy en $C_1$—$C_4$, par un halogène et/ou par le trifluorométhyle, un N,N-alkylènecarbamylalkyle en $C_1$—$C_4$, un N,N-alkénylènecarbamylalkyle en $C_1$—$C_4$, un N,N-(aza)alkylènecarbamylalkyle en $C_1$—$C_4$ ou un N,N-(oxa- ou thia)alkylènecarbamylalkyle en $C_1$—$C_4$, les restes alkylèneamino, alkénylèneamino, (aza)alkylèneamino, (oxa)alkylèneamino ou (thia)alkylèneamino incluant le groupe amino contenant 4 à 8 termes cycliques et l'alkylèneamino pouvant être substitué en C par un alcoxy en $C_1$—$C_4$ carbonyle ainsi que par l'oxo ou l'hydroxy, l'alkénylèneamino pouvant être substitué en C par un alcoxy en $C_1$—$C_4$ carbonyle et éventuellement en plus par l'hydroxy et l'(aza)alkylèneamino pouvant être substitué en C ou en N' par un alkyle en $C_1$—$C_4$ et/ou pouvant être substitué en N' par un alcanoyle en $C_1$—$C_7$ et $R_3$, $R_4$, $R_5$ et $R_6$ représentent l'hydrogène ou un alkyle en $C_1$—$C_7$ ou $R_3$ et $R_4$ et/ou $R_5$ et $R_6$ représentent ensemble un reste alkylène en $C_4$—$C_8$ et $R_7$, $R_8$, $R_9$ et $R_{10}$ représentent l'hydrogène ou $R_7$ ensemble avec $R_8$ et/ou $R_9$ ensemble avec $R_{10}$ représentent une liaison supplémentaire et leurs sels, en particulier leurs sels pharmaceutiques utilisables.

2. Composés de formule I selon la revendication 1 où $R_1$ et $R_2$ représentent indépendamment l'un de l'autre un carboxyalkyle en $C_1$—$C_4$, carbamylalkyle en $C_1$—$C_4$, un N-mono- ou un N,N-dialkyle en $C_1$—$C_4$ carbamylalkyle en $C_1$—$C_4$, un N-mono- ou un N,N-di(ω-hydroxyalkyle en $C_2$—$C_4$) carbamylalkyle en $C_1$—$C_4$, un N,N-(ω-aminoalkyle en $C_2$—$C_4$ carbamylalkyle en $C_1$—$C_4$, un N-[ω(N',N'-dialkyle en $C_1$—$C_4$ amino)alkyle en $C_2$—$C_4$] carbamylalkyle en $C_1$—$C_4$, un N-(ω-alkylèneaminoalkyle en $C_2$—$C_4$)carbamylalkyle en $C_1$—$C_4$ ayant 4 à 7 termes cycliques, un N-[ω-(3-azaalkylèneamino)alkyle en $C_2$—$C_4$] carbamylalkyle en $C_1$—$C_4$ ayant 4 à 7 termes cycliques, un N-[ω-(3-oxaalkylèneamino)-alkyle en $C_2$—$C_4$]carbamylalkyle en $C_1$—$C_4$ ayant 4 à 7 termes cycliques, un N-(phénylalkyle en $C_1$—$C_4$)-carbamylalkyle en $C_1$—$C_4$, la partie phényle pouvant être mono- ou disubstituée par un alkyle en $C_1$—$C_4$, par un alcoxy en $C_1$—$C_4$, par un halogène de numéro atomique allant jusqu'à 35 et/ou par le trifluorométhyle, un N-(α-carbamylalkyle en $C_1$—$C_4$)carbamylalkyle en $C_1$—$C_4$, un N,N-Alkylènecarbamylalkyle en $C_1$—$C_4$ ayant 4 à 7 termes cycliques, un N,N-(azaalkylène)carbamylalkyle en $C_1$—$C_4$ ayant 4 à 7 termes cycliques, un N,N-(3-oxaalkylène)carbamylalkyle en $C_1$—$C_4$ ayant 4 à 7 termes cycliques, un N,N-(3-oxaalkylène)carbamylalkyle en $C_1$—$C_4$ ayant 4 à 7 termes cycliques ou un N,N-(3-thiaalkylène)carbamylalkyle en $C_1$—$C_4$ ayant 4 à 7 termes cycliques, $R_3$, $R_4$, $R_5$ et $R_6$ représentent l'hydrogène ou $R_3$ et $R_5$ représentent des restes alkyle en $C_1$—$C_4$ identiques et $R_4$ et $R_6$ représentent l'hydrogène ou des restes alkyles en $C_1$—$C_4$ identiques ou $R_3$ et $R_4$ ainsi que $R_5$ et $R_6$ représentent ensemble des restes alkylènes en $C_2$—$C_6$ identiques et $R_7$ ensemble avec $R_8$ et/ou $R_9$ ensemble avec $R_{10}$ représentent une liaison supplémentaires et leurs sels.

3. Composés de formule I selon la revendication 1 où $R_1$ et $R_2$ représentent un carboxyalkyle en $C_1$—$C_4$, un carbamylalkyle en $C_1$—$C_4$, un N-monoalkyle en $C_1$—$C_7$ ou un N,N-dialkyle en $C_1$—$C_4$ carbamylalkyle en $C_1$—$C_4$, un N-(ω-dialkyle en $C_1$—$C_4$ aminoalkyle en $C_2$—$C_4$-alkyl)carbamylalkyle en $C_1$—$C_4$, un N-(ω-hydroxy-alkyle en $C_2$—$C_4$)carbamylalkyle en $C_1$—$C_4$, un N-(ω-alcoxy en $C_1$—$C_4$ alkyle en $C_2$—$C_4$)carbamylalkyle en $C_1$—$C_4$, un N-cycloalkyle en $C_5$—$C_6$)carbamylalkyle en $C_1$—$C_4$, un N-bicycloalkyle en $C_8$—$C_9$ carbamylalkyle $C_1$—$C_4$ ou un N-tricycloalkyle en $C_8$—$C_{10}$ carbamylalkyle en $C_1$—$C_4$, un N,N-alkylèneaminocarbonylalkyle en $C_1$—$C_4$ ayant 5- ou 6 chaînons éventuellement substitué en 3 par un alcoxy en $C_1$—$C_4$ carbonyle et éventuellement en 4 par l'oxo ou par l'hydroxy, alkénylèneaminocarbonylalkyle en $C_1$—$C_4$ ayant 5 ou 6 chaînons substitué en 3 par un alcoxy en $C_1$—$C_4$ carbonyle tel que le méthoxy- ou l'éthoxycarbonyle et éventuellement substitué en plus en 4 par l'hydroxy, un N,N-(aza)alkylèneaminocarbonylalkyle en $C_1$—$C_4$ ayant 5 ou 6 chaînons éventuellement substitué en N' par un alkyle en $C_1$—$C_4$ ou un alcanoyle en $C_1$—$C_4$, un N,N-(oxa)alkylèneaminocarbonylalkyle en $C_1$—$C_4$ ayant 5 ou 6 chaînons ou un N-phényl alkyle en $C_1$—$C_4$ carbamylalkyle en $C_1$—$C_4$ éventuellement mono- ou disubstitué dans la partie phényle par un alcoxy en $C_1$—$C_4$, $R_3$, $R_4$, $R_5$ et $R_6$ represéntent l-hydrogène et $R_7$, $R_8$, $R_9$ et $R_{10}$ représentent l'hydrogène ou $R_7$

ensemble avec $R_8$ ainsi que $R_9$ ensemble avec $R_{10}$ représentent une liaison supplémentaire.

4. Composés de formule I selon la revendication 2 où $R_1$ et $R_2$ représentent des restes carboxyalkyle en $C_1$—$C_4$ ou carbamylalkyle en $C_1$—$C_4$ identiques où des restes carbamyléthyle N-monosubstitués par un ω-N,N-dialkyle en $C_1$—$C_4$ aminoalkyle en $C_2$—$C_4$, N-mono- ou N,N-disubstitués par un ω-hydroxyalkyle en $C_2$—$C_4$ ou N,N-disubstitués par un (3 alkyle en $C_1$—$C_4$ 3-aza) alkylène ayant 4 à 6 chaînons et où $R_3$, $R_4$, $R_5$ et $R_6$ représentent l'hydrogène et leurs sels.

5. Composés selon l'une des revendications 1 et 2 où $R_1$ et $R_2$ sont identiques $R_3$, $R_4$, $R_5$ et $R_6$ représentent l'hydrogène et $R_7$ ensemble avec $R_8$ et $R_9$ ensemble avec $R_{10}$ représentent une liaison supplémentaire.

6. Le diamide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

7. Le di-N-(2-phényléthyl) amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

8. Le di-N--(carbamylméthyl)amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

9. Le di-N,N-[1,5-(2-méthoxycarbonyl)pent-2-énylèn] amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

10. Le diamide de l'acide 1,1',5,5'-tétrahydro-4-hydroxy-2,2'-dioxo-[3,4'-bi-2H-pyrrol]-1,1'-di(3-propionique) selon la revendication 1 ou l'un de ses sels.

11. L'acide 1,1',5,5'-tétrahydro-4-hydroxy-2,2'-dioxo-[3,4'-bi-2H-pyrrol]-1,1'-diacétique selon la revendication 1 ou l'un de ses sels.

12. Le di-N-(2-diisopropylaminoéthyl)amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

13. Le dipipéridide de l'acide 1,1',5,5'-tétrahydro-4-hydroxy-2,2'-dioxo-[3,4'-bi-2H-pyrrol]-1,1'-diacétique selon la revendication 1 ou l'une de ses sels.

14. Le di-N-cyclohexylamide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

15. Le dimorpholide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

16. Le di-N-éthylamide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

17. Le di-N-[2-(3,4-diméthoxyphényl)éthyl]amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

18. Le di-N-(2,2-diméthylpropyl)amide de l'acide 1,1',5,5'-tétrahydro-4-hydroxy-2,2'-dioxo-[3,4'-bis-2H-pyrrol]-1,1'-diacétique selon la revendication 1 ou l'un de ses sels.

19. Le di-N-(3-méthoxypropyl)amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

20. Le di-N-méthyl-N-(2-hydroxyéthyl) amide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

21. L'acide 1,1',5,5'-tétrahydro-4-hydroxy-2,2'-dioxo-[3,4'-bi-2H-pyrrol]-1,1'-di(3-propionique) selon la revendication 1 ou l'un de ses sels.

22. Le di-N-adamantylamide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

23. Le di (4-acétyl)pipérazide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

24. L'acide octahydro-4-hydroxy-2,2'-dioxo-[3,4'-bi-2H-pyrrol]-1,1'-diacétique selon la revendication 1 ou l'un de ses sels.

25. Le di(4-méthyl)pipérazide de l'acide 1,1',5,5'-tétrahydro-4-hydroxy-2,2'-dioxo-[3,4'-bi-2H-pyrrol]-1,1'-diacétique selon la revendication 1 ou l'un de ses sels.

26. Le diamide de l'acide octahydro-4-hydroxy-2,2'-dioxo[3,4'-bi-2H-pyrrol]-1,1'-diacétique selon la revendication 1 ou l'un de ses sels.

27. Le sel de sodium, de potassium, d'ammonium, de diéthylammonium, de bis(2-hydroxyéthyl)ammonium, de tris(2-hydroxyéthyl)ammonium), de tris(hydroxyméthyl) méthyl-ammonium ou de N,N-diméthyl-N-(2-hydroxyéthyl)-ammonium d'un composé selon l'une des revendications 1 à 26.

28. Un composé selon l'une des revendications 1 à 27 sous forme libre ou sous forme d'un sel pharmaceutiquement utilisable destiné à être utilisé dans un procédé de traitement thérapeutique de l'organisme humain ou animal.

29. Préparation pharmaceutiques contenant un composé selon l'une revendications 1 à 27 sous forme libre ou sous forme d'un sel pharmaceutiquement utilisable à côte des adjuvants pharmaceutiques usuels.

30. Procédé pour la preparation de nouvelles tétrahydro-, hexahydro- ou octahydro-[3,4'-bi-2H-pyrrol]-2,2'-diones selon la revendication 1 et de leurs sels, caractérisé

a) en ce que l'on cyclise un composé de formule

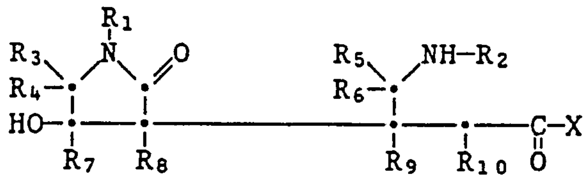

(II)

ou X représente un hydroxy éventuellement estérifie ou éthérifié ou l'une de ses sels ou

b) en ce que, dans un composé de formule

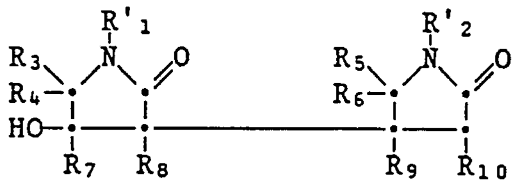

(III)

où $R'_1$représente un groupe transformable en un reste $R_2$ et $R'_2$ représente un reste $R_2$ ou un groupe transformable en ce reste ou dans l'un de ses sels, on transforme $R'_1$ ou un groupe $R_1$ et éventuellement un reste $R'_2$ transformable en $R_2$ en un groupe $R_2$ ou

c) en ce que, dans un composé de formule

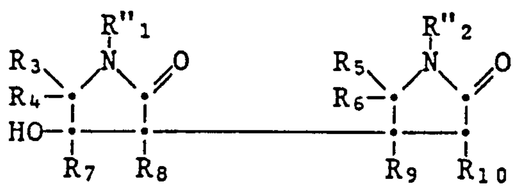

(IV)

où $R''_1$ représente l'hydrogène et $R''_2$ représente l'hydrogène ou un reste $R_2$ ou dans l'un de ses sels, on remplace l'atome d'hydrogène $R''_1$ par un reste $R_1$ et éventuellement l'atome d'hydrogène $R''_2$ par un reste $R_2$ ou

d) en ce que l'on condense un composé de formule

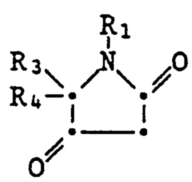

(V)

ou l'un de ses tautomères et/ou l'un de ses sels avec un composé de formule

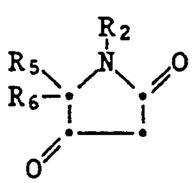

(VI)

ou avec l'un de ses tautomères et, si désiré, en ce que l'on transforme un composé obtenu conformément au procédé en un autre composé de formule I, en ce que l'on sépare le mélange d'isomères obtenu conformément au procédé en isomères et/ou en ce que l'on transforme le composé libre obtenu conformément au procédé en un sel ou le sel obtenu conformément au procédé en un composé libre ou en un autre sel.

31. Utilisation d'un composé selon l'une des revendications 1 à 28 pour la préparation d'un nootrope.

**Revendications pour les Etat contractants: AT**

1. Procédé pour la préparation de nouvelles tétrahydro-, hexahydro- ou octahydro-[3,4'-bi-2H-pyrrol]-2,2'-diones substitutées de formule

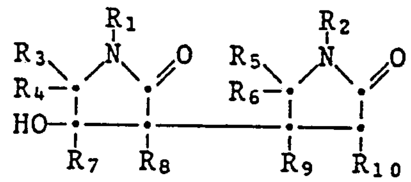

(I),

où $R_1$ et $R_2$ représentent indépendamment l'un de l'autre un carboxyalkyle en $C_1$—$C_4$, un carbamylalkyle en $C_1$—$C_4$, un N-alkyle en $C_1$—$C_7$ ou un N,N-dialkyle en -$C_1$—$C_4$ carbamylalkyle en $C_1$—$C_4$, le ou les groupe(s) hydroxy, alcoxy, amino, alkylamino, dialkylamino, alkylène ou alkénylène ou aza-, oxa- ou thiaalkylène-amino en une position plus éloignée que la position α par rapport à l'atome d'azote du carbamyle portant le N-mono- ou le N,N-di(hydroxyalkyle en $C_2$—$C_4$ carbamylalkyle en $C_1$—$C_4$, N-alcoxy en $C_1$—$C_4$ alkyle en $C_2$—$C_4$)carbamylalkyle en $C_1$—$C_4$, un N-mono- ou un N-N-di(aminoalkyle en $C_2$—$C_4$) carbamylalkyle en $C_1$—$C_4$, un N-(alkyle en $C_1$—$C_7$ aminoalkyle en $C_1$—$C_4$)- ou un N-(dialkyle en $C_1$—$C_4$ aminoalkyle en $C_2$—$C_4$) carbamylalkyle en $C_1$—$C_4$, un N-(alkylèneaminoalkyle en $C_2$—$C_4$) carbamylalkyle en $C_1$—$C_4$, un N-(alkénylèneaminoalkyle en $C_2$—$C_4$) carbamylalkyle en $C_1$—$C_4$, un N-[(aza)alkylèneaminoalkyle en $C_2$—$C_4$]carbamylalkyle en $C_1$—$C_4$, un N-[(oxa)alkylèneaminoalkyle en $C_2$—$C_4$] carbamylalkyle en $C_1$—$C_4$ ou un N-[(thia)alkylèneaminoalkyle en $C_2$—$C_4$]carbamylalkyle en $C_1$—$C_4$, les restes alkylèneamino, alkénylène-amino, (aza)alkylèneamino, (oxa)alkylèneamino ou (thia)alkylèneamino incluant le groupe amino contenant 4 à 8 termes cycliques et l'alkylèneamino pouvant être substitué en C par un alcoxy en $C_1$—$C_4$ carbonyle ainsi que par l'oxo ou l-hydroxy, l'alkénylèneamino pouvant être substitué par un alcoxy en $C_1$—$C_4$ carbonyle et éventuellement en plus par l'hydroxy et l'(aza)alkylène amino pouvant être substitué en C ou en N' par un alkyle en $C_1$—$C_4$ et ou pouvant être substitué en N' par un alcanoyle en $C_1$—$C_7$, un N-(carbamylalkyle en $C_1$—$C_4$) carbamylalkyle en $C_1$—$C_4$, un N-(N'-alkyle en $C_1$—$C_7$ ou N',N'-dialkyle en $C_1$—$C_4$ carbamylalkyle en $C_2$—$C_4$ carbamylalkyle en $C_1$—$C_4$, un 3-N-cycloalkyle en $C_3$—$C_8$ ou le dicycloalkyle en $C_3$—$C_8$ carbamylalkyle en $C_1$—$C_4$, un N-bicycloalkyle en $C_7$—$C_9$ carbamylalkyle en $C_1$—$C_4$, un N-tricyclo-alkyle en $C_8$—$C_{12}$ carbamylalkyle en $C_1$—$C_4$, un N-mono- ou N,N-di(phénylalkyle en $C_1$—$C_4$) carbamylalkyle en $C_1$—$C_4$ non substitué ou substitué dans la partie phényle par un alkyle en $C_1$—$C_4$, par un alcoxy en $C_1$—$C_4$, par un halogène et/ou par le trifluorométhyle, un N,N-alkylènecarbamylalkyle en $C_1$—$C_4$, un N,N-alkénylènecarbamylalkyle en $C_1$—$C_4$, un N,N-(aza)alkylènecarbamylalkyle en $C_1$—$C_4$ ou un N,N-(oxa- ou thia)alkylènecarbamylalkyle en $C_1$—$C_4$, les restes alkyleneamino, alkénylèneamino, (aza)alkylèneamino, (oxa)alkylèneamino ou (thia)alkylèneamino incluant le groupe amino contenant 4 à 8 termes cycliques et l'alkylèneamino pouvant être substitué en C par un alcoxy en $C_1$—$C_4$ carbonyle ainsi que par l'oxo ou l'hydroxy, l'alkénylèneamino pouvant être substitué en C par un alcoxy en $C_1$—$C_4$ carbonyle et éventuellement en plus par l'hydroxy et l'(aza)alkylèneamino pouvant être substitué en C ou en N' par un alkyle en $C_1$—$C_4$ et/ou pouvant être substitué en N' par un alcanoyle en $C_1$—$C_7$ et $R_3$, $R_4$, $R_5$ et $R_6$ représentent l'hydrogène ou un alkyle en $C_1$—$C_7$ ou $R_3$ et $R_4$ et/ou $R_5$ et $R_6$ représentent ensemble un reste alkylène en $C_4$—$C_8$ et $R_7$, $R_8$, $R_9$ et $R_{10}$ représentent l'hydrogène ou $R_7$ ensemble avec $R_8$ et/ou $R_9$ ensemble avec $R_{10}$ représentent une liaison supplémentaire et de leurs sels, caractérisé .

a) en ce que l'on cyclise un composé de formule

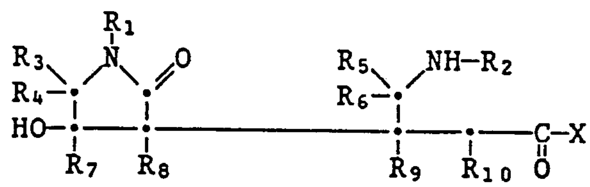

(II)

ou X représente un hydroxy éventuellement estérifié ou éthérifié ou l'un de ses sels ou

b) en ce que, dans un composé de formule

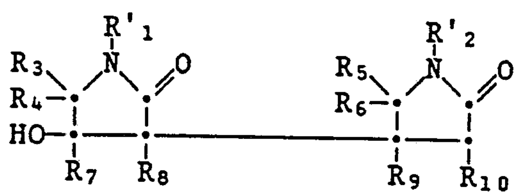

(III)

où $R'_1$ représente un groupe transformable en un reste $R_2$ et $R'_2$ représente un reste $R_2$ ou un groupe

transformable en ce reste ou dans l'un de ses sels, on transforme $R'_1$ ou un groupe $R_1$ et éventuellement un reste $R'_2$ transformable en $R_2$ en un groupe $R_2$ ou

c) en ce que, dans un composé de formule

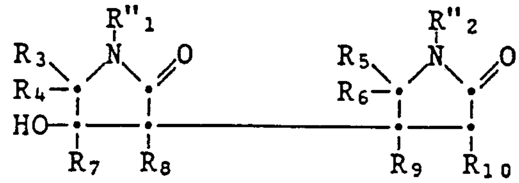

(IV)

où $R''_1$ représente l'hydrogène et $R''_2$ représente l'hydrogène ou un reste $R_2$ ou dans l'un de ses sels, on remplace l'atome d'hydrogène $R''_1$ par un reste $R_1$ et éventuellement l'atome d'hydrogène $R''_2$ par un reste $R_2$ ou

d) en ce que l'on condense un composé de formule

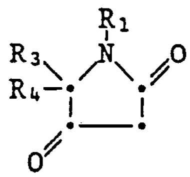

(V)

ou l'un de ses tautomères et/ou l'un de ses sels avec un composé de formule

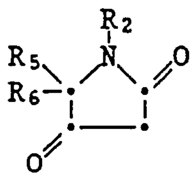

(VI)

ou avec l'un de ses tautomères et, si désiré, en ce que l'on transforme un composé obtenu conformément au procédé en un autre composé de formule I, en ce que l'on sépare le mélange d'isomères obtenu conformément au procédé en isomères et/ou en ce que l'on transforme le composé libre obtenu conformément au procédé en un sel ou obtenue conformément au procédé en un composé libre ou en un autre sel.

2. Procédé selon la revendication 1 pour la préparation des composés de formule I où $R_1$ et $R_2$ représentent indépendamment l'un de l'autre un carboxyalkyle en $C_1$—$C_4$, un carbamylalkyle en $C_1$—$C_4$, un N-mono- ou un N,N-dialkyle en $C_1$—$C_4$ carbamylalkyle en $C_1$—$C_4$, un N-mono- ou un N,N-di(ω-hydroxyalkyle en $C_2$—$C_4$)carbamylalkyle en $C_1$—$C_4$, un N,N-(ω-aminoalkyle en $C_2$—$C_4$)carbamylalkyle en $C_1$—$C_4$, un N-[ω-(N',N'-dialkyle en $C_1$—$C_4$ amino)alkyle en $C_2$—$C_4$]carbamylalkyle en $C_1$—$C_4$, un N-(ω-alkylèneaminoalkyle en $C_2$—$C_4$)carbamylalkyle en $C_1$—$C_4$ ayant 4 à 7 termes cycliques, un N-[ω-(3-azaalkylèneamino)alkyle en $C_2$—$C_4$]carbamylalkyle en $C_1$—$C_4$, un N-[ω-(3-oxaalkylèneamino)-alkyle en $C_2$—$C_4$]carbamylalkyle en $C_1$—$C_4$ ayant 4 à 7 termes cycliques, un N-(phénylalkyle en $C_1$—$C_4$)carbamyl-alkyle en $C_1$—$C_4$, la partie phényle pouvant être mono- ou disubstituée par un alkyle en $C_1$—$C_4$, par un alcoxy en $C_1$—$C_4$, par un halogène de numéro atomique allant jusqu'à 35 et/ou par le trifluorométhyle, un N-(α-carbamylalkyle en $C_1$—$C_4$)carbamylalkyle en $C_1$—$C_4$, un N,N-alkylènecarbamylalkyle en $C_1$—$C_4$ ayant 4 à 7 termes cycliques, un N,N-(azaalkylène)carbamylalkyle en $C_1$—$C_4$ ayant 4 à 7 termes cycliques, un N,N-(3-oxaalkylène)carbamylalkyle en $C_1$—$C_4$ ayant 4 à 7 termes cycliques, un N,N-(3-oxaalkylène)carbamyl-alkyle en $C_1$—$C_4$ ayant 4 à 7 termes cycliques ou un N,N-(3-thiaalkylène)carbamylalkyle en $C_1$—$C_4$ ayant 4 à 7 termes cycliques, $R_3$, $R_4$, $R_5$ et $R_6$ représentent l'hydrogène ou $R_3$ et $R_5$ représentent des restes alkyle en $C_1$—$C_4$ identiques et $R_4$ et $R_6$ représentent l'hydrogène ou des restes alkyles en $C_1$—$C_4$ identiques ou $R_3$ et $R_4$ ainsi que $R_5$ et $R_6$ représentent ensemble des restes alkylènes en $C_2$—$C_6$ identiques et $R_7$ ensemble avec $R_8$ et/ou $R_9$ ensemble avec $R_{10}$ représentent une liaison supplémentaire.

3. Procédé selon la revendication 1 pour la préparation des composés de formule I où $R_1$ et $R_2$ sont identiques et représentent un carboxyalkyle en $C_1$—$C_4$, un carbamyalkyle en $C_1$—$C_4$, un N-monoalkyle en $C_1$—$C_7$ ou un N,N-dialkyle en $C_1$—$C_4$ carbamylalkyle en $C_1$—$C_4$, un N-(ω-dialkyle en $C_1$—$C_4$ aminoalkyle en $C_2$—$C_4$)carbamylalkyle en $C_1$—$C_4$, un N-(ω-hydroxyalkyle en $C_2$—$C_4$)carbamylalkyle en $C_1$—$C_4$, un N-(ω-alcoxy en $C_1$—$C_4$ alkyle en $C_2$—$C_4$)carbamylalkyle en $C_1$—$C_4$, un N-cycloalkyle en $C_5$—$C_6$ carbamylalkyle en $C_1$—$C_4$, un N-bicycloalkyle en $C_8$—$C_9$ carbamylalkyle en $C_1$—$C_4$ ou un N-tricycloalkyle en $C_8$—$C_{10}$ carbamyl-alkyle en $C_1$—$C_4$, un N,N-alkylèneaminocarbonylalkyle en $C_1$—$C_4$ ayant 5- ou 6 chaînons éventuellement substitué en 3 par un alcoxy en $C_1$—$C_4$ carbonyle et éventuellement en 4 par l'oxo ou par l'hydroxy, un alkénylèneaminocarbonylalkyle en $C_1$—$C_4$ ayant 5 ou 6 chaînons substitué en 3 par un alcoxy en $C_1$—$C_4$ carbonyle tel que le méthoxy- ou l'éthoxycarbonyle et éventuellement substitué en plus en 4 par l'hydroxy,

un N,N-(aza)alkylèneaminocarbonylalkyle en $C_1$—$C_4$ ayant 5 ou 6 chaînons éventuellement substitué en N' par un alkyle en $C_1$—$C_4$ ou un alcanoyle en $C_1$—$C_4$, un N,N-(oxa)alkylèneaminocarbonylalkyle en $C_1$—$C_4$ ayant 5 ou 6 chaînons ou un N-phénylalkyle en $C_1$—$C_4$ carbamylalkyle en $C_1$—$C_4$ éventuellement mono- ou disubstitué dans la partie phényle par un alcoxy en $C_1$—$C_4$, $R_3$, $R_4$, $R_5$ et $R_6$ représentent l'hydrogène et $R_7$, $R_8$, $R_9$ et $R_{10}$ représentent l'hydrogène ou $R_7$ ensemble avec $R_8$ ainsi que $R_9$ ensemble avec $R_{10}$ représentent une liaison supplémentaire.

4. Procédé selon la revendication 1 pour la préparation des composés de formule I où $R_1$ et $R_2$ représentent des restes carboxyalkyle en $C_1$—$C_4$ ou carbamylalkyle e $C_1$—$C_4$ identiques ou des restes carbamyléthyle N-monosubstitués par un ω-N,N-dialkyle en $C_1$—$C_4$ aminoalkyle en $C_2$—$C_4$, N-mono- ou N,N-disubstitués par un ω-hydroxyalkyle en $C_2$—$C_4$ ou N,N-disubstitués par un (3 alkyle en $C_1$—$C_4$ 3-aza) alkylène ayant 4 à 6 chaînons et où $R_3$, $R_4$, $R_5$ et $R_6$ représentent l'hydrogène et de leurs sels.

5. Procédé selon la revendication 1 pour la préparation des composés obtenus selon la revendication 1 ou 2 où $R_1$ et $R_2$ sont identiques $R_3$, $R_4$, $R_5$ et $R_6$ représentent l'hydrogène et $R_7$ ensemble avec $R_8$ et $R_9$ ensemble avec $R_{10}$ représentent une liaison supplémentaire.

6. Procédé selon la revendication 1 pour la préparation du diamide de l'acide 1,1',5,5'-tétrahydro-4-hydroxy-2,2'-dioxo-[3,4'-bi-2H-pyrrol]-1,1'-diacétique ou de l'un de ses sels.

7. Procédé selon la revendication 1 pour la préparation du di-N-(2-phényléthyl)amide de l'acide 1,1',5,5'-tétrahydro-4-hydroxy-2,2'-dioxo-[3,4'-bi-2H-pyrrol]-1,1'-diacétique ou de l'un de ses sels.

8. Procédé selon la revendication 1 pour la préparation du di-N-(carbamylméthyl)amide de l'acide 1,1',5,5'-tétrahydro-4-hydroxy-2,2'-dioxo-[3,4'-bi-2H-pyrrol]-1,1'-diacétique ou de l'un de ses sels.

9. Procédé selon la revendication 1 pour la préparation du di-N,N-[1,5-(2-méthoxycarbonyl)pent-2-énylèn] amide de l'acide 1,1',5,5'-tétrahydro-4-hydroxy-2,2'-dioxo-[3,4'-bi-2H-pyrrol]-1,1'-diacétique ou de l'un de ses sels.

10. Procédé selon la revendication 1 pour la préparation du diamide de l'acide 1,1',5,5'-tétrahydro-4-hydroxy-2,2'-dioxo-[3,4'-bi-2H-pyrrol]-1,1'-di(3-propionique) ou de l'un de ses sels.

11. Procédé selon la revendication 1 pour la préparation de l'acide 1,1',5,5'-tétrahydro-4-hydroxy-2,2'-dioxo-[3,4'-bi-2H-pyrrol]-1,1'-diacétique ou de l'un de ses sels.

12. Procédé selon la revendication 1 pour la préparation du di-N-(2-diisopropylaminoéthyl)amide de l'acide 1,1',5,5'-tétrahydro-4-hydroxy-2,2'-dioxo-[3,4'-bi-2H-pyrrol]-1,1'-diacétique ou de l'un de ses sels.

13. Procédé selon la revendication 1 pour la préparation du dipipéridide de l'acide 1,1',5,5'-tétrahydro-4-hydroxy-2,2'-dioxo-[3,4'-bi-2H-pyrrol]-1,1'-diacétique ou de l'un de ses sels.

14. Procédé selon la revendication 1 pour la préparation du di-N-cyclohexylamide de l'acide 1,1',5,5'-tétrahydro-4-hydroxy-2,2'-dioxo-[3,4'-bi-2H-pyrrol]-1,1'-diacétique ou de l'un de ses sels.

15. Procédé selon la revendication 1 pour la préparation du dimorpholide de l'acide 1,1',5,5'-tétrahydro-4-hydroxy-2,2'-dioxo-[3,4'-bi-2H-pyrrol]-1,1'-diacétique ou de l'un de ses sels.

16. Procédé selon la revendication 1 pour la préparation du di-N-éthylamide de l'acide 1,1',5,5'-tétrahydro-4-hydroxy-2,2'-dioxo-[3-,4'-bi-2H-pyrrol]-1,1'-diacétique ou de l'un de ses sels.

17. Procédé selon la revendication 1 pour la préparation du di-N-[2-(3,4-diméthoxyphényl)éthyl]amide de l'acide 1,1',5,5'-tétrahydro-4-hydroxy-2,2'-dioxo-[3,4'-bi-2H-pyrrol]-1,1'-diacétique ou de l'un de ses sels.

18. Procédé selon la revendication 1 pour la préparation du di-N-(2,2-diméthylpropyl)amide de l'acide 1,1',5,5'-tétrahydro-4-hydroxy-2,2'-dioxo-[3,4'-bi-2H-pyrrol]-1,1'-diacétique ou de l'un de ses sels.

19. Procédé selon la revendication 1 pour la préparation du di-N-(3-méthoxypropyl)amide de l'acide 1,1',5,5'-tétrahydro-4-hydroxy-2,2'-dioxo-[3,4'-bi-2H-pyrrol]-1,1'-diacétique ou de l'un de ses sels.

20. Procédé selon la revendication 1 pour la préparation du di-N-méthyl-N-(2-hydroxyéthyl) amide de l'acide 1,1',5,5'-tétrahydro-4-hydroxy-2,2'-dioxo-[3,4'-bi-2H-pyrrol]-1,1'-diacétique ou de l'un de ses sels.

21. Procédé selon la revendication 1 pour la préparation de l'acide 1,1',5,5'-tétrahydro-4-hydroxy-2,2'-dioxo-[3,4'-bi-2H-pyrrol]-1,1'-di(3-propionique) ou de l'un de ses sels.

22. Procédé selon la revendication 1 pour la préparation du di-N-adamantylamide de l'acide 1,1',5,5'-tétrahydro-4-hydroxy-2,2'-dioxo-[3,4'-bi-2H-pyrrol]-1,1'-diacétique ou de l'un de ses sels.

23. Procédé selon la revendication 1 pour la préparation du di (4-acétyl)pipérazide de l'acide 1,1',5,5'-tétrahydro-4-hydroxy-2,2'-dioxo-[3,4'-bi-2H-pyrrol]-1,1'-diacétique ou de l'un de ses sels.

24. Procédé selon la revendication 1 pour la préparation de l'acide octahydro-4-hydroxy-2,2'-dioxo[3,4'-bi-2H-pyrrol]-1,1'-diacétique ou de l'un de ses sels.

25. Procédé selon la revendication 1 pour la préparation du di(4-méthyl)pipérazide de l'acide 1,1',5,5'-tétrahydro-4-hydroxy-2,2'-dioxo-[3,4'-bi-2H-pyrrol]-1,1'-diacétique ou de l'un de ses sels.

26. Procédé selon la revendication 1 pour la préparation du diamide de l'acide octahydro-4-hydroxy-2,2'-dioxo[3,4'-bi-2H-pyrrol]-1,1'-diacétique ou de l'un de ses sels.

27. Procédé selon la revendication 1 pour la préparation des composés obtenus selon l'une des revendications 1 à 26 sous la forme d'un sel de sodium, de potassium, d'ammonium, de diéthylammonium, de bis(2-hydroxyéthyl)ammonium, de tris(2-hydroxyéthyl)ammonium), de tris(hydroxyméthyl)méthyl-ammonium ou de N,N-diméthyl-N-(2-hydroxyéthyl)-ammonium.

28. Procédé pour la préparation de préparations pharmaceutiques, caractérise en ce que l'on mélange un composé obtenu selon l'une des revendications 1 à 27 sous forme libre ou sous forme d'un sel pharmaceutiquement utilisable avec des adjuvants pharmaceutiques usuels.

**Claims for the Contracting States: BE CH DE FR GB IT LI LU NL SE**

1. Novel substituted tetrahydro-, hexahydro- and octahydro-[3,4'-bi-2H-pyrrol]-2,2'-diones of the formula

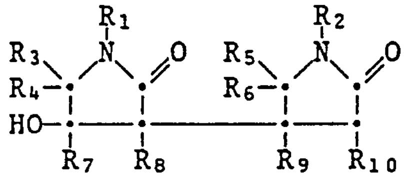

(I),

wherein each of $R_1$ and $R_2$ independently of the other is carboxy-$C_1$—$C_4$alkyl, carbamoyl-$C_1$—$C_4$alkyl, N-$C_1$—$C_7$- or N,N-di-$C_1$—$C_4$-alkylcarbamoyl-$C_1$—$C_4$alkyl, or one of the following radicals carrying the hydroxy, alkoxy, amino, alkylamino, dialkylamino, alkylene or alkenylene, or aza-, oxa- or thia-alkyleneamino group(s) in a position higher than the α-position with respect to the carbamoyl nitrogen atom: N-mono or N,N-di(hydroxy-$C_2$—$C_4$-alkyl)carbamoyl-$C_1$—$C_4$alkyl, N-($C_1$—$C_4$alkoxy-$C_2$—$C_4$alkyl)carbamoyl-$C_1$—$C_4$alkyl, N-mono- or N,N-di-(amino-$C_2$—$C_4$alkyl)-carbamoyl-$C_1$—$C_4$alkyl, N-($C_1$—$C_7$-alkylamino-$C_2$—$C_4$alkyl)- or N-(di-$C_2$—$C_4$alkylamino-$C_2$—$C_4$-alkyl)carbamoyl-$C_1$—$C_4$alkyl, N-(alkyleneamino-$C_2$—$C_4$alkyl)carbamoyl-$C_1$—$C_4$alkyl, N-(alkenyleneamino-$C_2$—$C_4$alkyl)carbamoyl-$C_1$—$C_4$alkyl, N-[(aza)alkyleneamino-$C_2$—$C_4$-alkyl]-carbamoyl-$C_1$—$C_4$alkyl, N-[(oxa)-alkyleneamino-$C_2$—$C_4$alkyl]-carbamoyl-$C_1$—$C_4$alkyl and N-[(thia)alkyleneamino-$C_2$—$C_4$alkyl]-carbamoyl-$C_1$—$C_4$alkyl, wherein the alkyleneamino, alkenyleneamino, (aza)alkyleneamino-, (oxa)alkyleneamino or (thia)alkyleneamino radical contains, including the amino group from 4 to 8 ring members and alkyleneamino may be C-substituted by $C_1$—$C_4$alkoxycarbonyl and by oxo or hydroxy, alkenyleneamino may be C-substituted by $C_1$—$C_4$alkoxycarbonyl and optionally additionally by hydroxy, and (aza)alkyleneamino may be C- or N'-substituted by $C_1$—$C_4$-alkyl and/or N'-substituted by $C_1$—$C_7$alkanoyl; or is N-(carbamoyl-$C_1$—$C_4$alkyl)carbamoyl-$C_1$—$C_4$alkyl, N-(N'-$C_1$—$C_7$- or N',N'-di-$C_1$—$C_4$-alkylcarbamoyl-$C_2$—$C_4$alkyl)carbamoyl-$C_1$—$C_4$alkyl, 3-N-$C_3$—$C_8$cycloalkyl- or N,N-di-$C_3$—$C_8$cycloalkylcarbamoyl-$C_1$—$C_4$alkyl, N-$C_7$—$C_9$bicycloalkylcarbamoyl-$C_1$—$C_4$alkyl, N-$C_8$—$C_{12}$tricycloalkylcarbamoyl-$C_1$—$C_4$alkyl, N-mono- or N,N-di-(phenyl-$C_1$—$C_4$alkyl)carbamoyl-$C_1$—$C_4$alkyl which is unsubstituted or substituted in the phenyl moiety by $C_1$—$C_4$alkyl, $C_1$—$C_4$alkoxy, halogen and/or by trifluoromethyl, or is N,N-alkylenecarbamoyl-$C_1$—$C_4$alkyl, N,N-alkenylenecarbamoyl-$C_1$—$C_4$alkyl, N,N-(aza)alkylenecarbamoyl-$C_1$—$C_4$alkyl or N,N-(oxa or thia-)alkylenecarbamoyl-$C_1$—$C_4$alkyl, wherein the alkyleneamino, alkenyleneamino, (aza)alkyleneamino, (oxa)alkyleneamino or (thia)alkyleneamino radical contains, including the amino group, from 4 to 8 ring members and alkyleneamino may be C-substituted by $C_1$—$C_4$alkoxycarbonyl and by oxo or hydroxy, and alkenyleneamino may be C-substituted by $C_1$—$C_4$alkoxycarbonyl and optionally additionally by hydroxy, and (aza)alkyleneamino may be C- or N'-substituted by $C_1$—$C_4$alkyl and/or N'-substituted by $C_1$—$C_7$alkanoyl, and each of $R_3$, $R_4$, $R_5$ and $R_6$ is hydrogen or $C_1$—$C_7$alkyl, or $R_3$ and $R_4$ and/or $R_5$ and $R_6$ taken together are a $C_4$—$C_8$alkylene radical, and each of $R_7$, $R_8$, $R_9$ and $R_{10}$ is hydrogen, or $R_7$ together with $R_8$ and/or $R_9$ together with $R_{10}$ are in each case an additional bond, and their salts, especially their pharmaceutically acceptable salts.

2. Compounds of formula I according to claim 1, wherein each of $R_1$ and $R_2$ independently of the other is carboxy-$C_1$—$C_4$alkyl, carbamoyl-$C_1$—$C_4$alkyl, N-mono- or N,N-di-$C_1$—$C_4$-alkylcarbamoyl-$C_1$—$C_4$alkyl, N-mono- or N,N-di(ω-hydroxy-$C_2$—$C_4$alkyl)carbamoyl-$C_1$—$C_4$alkyl, N,N-(ω-amino-$C_2$—$C_4$alkyl)carbamoyl-$C_1$—$C_4$alkyl, N-[ω-(N',N'-Di-$C_1$—$C_4$alkylamino)-$C_2$—$C_4$alkyl]carbamoyl-$C_1$—$C_4$alkyl, N-(ω-alkyleneamino-$C_2$—$C_4$alkyl)carbamoyl-$C_1$—$C_4$alkyl having from 4 to 7 ring members, N-[ω-(3-azaalkyleneamino)-$C_2$—$C_4$alkyl]carbamoyl-$C_1$—$C_4$alkyl having from 4 to 7 ring members, N-[ω-(3-oxaalkylenamino)-$C_2$—$C_4$alkyl]carbamoyl-$C_1$—$C_4$-alkyl having from 4 to 7 ring members, N-(phenyl-$C_1$—$C_4$alkyl)carbamoyl-$C_1$—$C_4$alkyl, wherein the phenyl moiety may be mono- or di-substituted by $C_1$—$C_4$alkyl, $C_1$—$C_4$alkoxy, halogen having an atomic number of up to and including 35 and/or by trifluoromethyl N-(α-carbamoyl-$C_1$—$C_4$alkyl)carbamoyl-$C_1$—$C_4$-alkyl, N,N-alkylenecarbamoyl-$C_1$—$C_4$alkyl having from 4 to 7 ring members, N,N-(azaalkylene)carbamoyl-$C_1$—$C_4$alkyl having from 4 to 7 ring members, N,N-(3-oxaalkylene)carbamoyl-$C_1$—$C_4$alkyl having from 4 to 7 ring members or N,N-(3-thiaalkylene)carbamoyl-$C_1$—$C_4$alkyl having from 4 to 7 members, $R_3$, $R_4$, $R_5$ and $R_6$ are hydrogen or $R_3$ and $R_5$ are identical $C_1$—$C_4$alkyl radicals and $R_4$ and $R_6$ are hydrogen or identical $C_1$—$C_4$alkyl radicals, or $R_3$ and $R_4$ and also $R_5$ and $R_6$ taken together are in each case identical $C_2$—$C_6$alkylene radicals, and $R_7$ together with $R_8$ and/or $R_9$ together with $R_{10}$ are in each case an additional bond, and their salts.

3. Compounds of formula I according to claim 1, wherein $R_1$ and $R_2$ are identical and are carboxy-$C_1$—$C_4$alkyl, carbamoyl-$C_1$—$C_4$alkyl, N-mono-$C_1$—$C_7$- or N,N-di-$C_1$—$C_4$-alkylcarbamoyl-$C_1$—$C_4$alkyl, N-(ω-di-$C_1$—$C_4$alkylamino-$C_2$—$C_4$alkyl)carbamoyl-$C_1$—$C_4$alkyl, N-(ω-hydroxy-$C_2$—$C_4$alkyl)carbamoyl-$C_1$—$C_4$alkyl, N-(ω-$C_1$—$C_4$alkoxy-$C_2$—$C_4$alkyl)carbamoyl-$C_1$—$C_4$alkyl, N-$C_5$—$C_6$cycloalkyl-, N-$C_8$—$C_9$-bicycloalkyl- or N-$C_8$—$C_{10}$-tricycloalkyl-carbamoyl-$C_1$—$C_4$alkyl, 5- or 6-membered N,N-alkyleneaminocarbonyl-$C_1$—$C_4$alkyl optionally 3-substituted by $C_1$—$C_4$alkoxycarbonyl, and 4-substituted by oxo or hydroxy, 5- or 6-membered alkenyleneaminocarbonyl-$C_1$—$C_4$alkyl 3-substituted by $C_1$—$C_4$alkoxycarbonyl and optionally additionally 4-substituted by hydroxy, 5- or 6-membered N,N-(aza)alkyleneaminocarbonyl-$C_1$—$C_4$alkyl optionally N'-

substituted by $C_1$—$C_4$alkyl or by $C_1$—$C_4$alkanoyl, 5- or 6-membered N,N-(oxa)alkyleneaminocarbonyl-$C_1$—$C_4$-alkyl, or N-phenyl-$C_1$—$C_4$alkylcarbamoyl-$C_1$—$C_4$alkyl optionally mono- or di-substituted in the phenyl moiety by $C_1$—$C_4$alkoxy, $R_3$, $R_4$, $R_5$ and $R_6$ are hydrogen, and each of $R_7$, $R_8$, $R_9$ and $R_{10}$ is hydrogen, or $R_7$ together with $R_8$ and also $R_9$ together with $R_{10}$ are in each case an additional bond, and salts thereof.

4. Compounds of formula I according to claim 2, wherein $R_1$ and $R_2$ are identical carboxy-$C_1$—$C_4$alkyl or carbamoyl-$C_1$—$C_4$alkyl radicals, or carbamoylmethyl radicals N-monosubstituted by ω-N,N-di-$C_1$—$C_4$alkyl-amino-$C_2$—$C_4$alkyl N-mono- or N,N-di-substituted by ω-hydroxy-$C_2$—$C_4$alkyl, or N,N-disubstituted by 4- to 6-membered (3-$C_1$—$C_4$-alkyl-3-aza)alkylene, and $R_3$, $R_4$, $R_5$ and $R_6$ are hydrogen, and salts thereof.

5. Compounds according to one of claims 1 and 2, wherein $R_1$ and $R_2$ are identical, $R_3$, $R_4$, $R_5$ and $R_6$ are hydrogen, and $R_7$ together with $R_8$ and $R_9$ together with $R_{10}$ are in each case an additional bond.

6. 1,1',5,5'-tetrahydro-4-hydroxy-2,2'-dioxo-[3,4'-bi-2H-pyrrole]-1,1'-diacetic acid diamide according to claim 1 or a salt thereof.

7. 1,1',5,5'-tetrahydro-4-hydroxy-2,2'-dioxo-[3,4'-bi-2H-pyrrole]-1,1'-diacetic acid di-N-(2-phenylethyl)-amide according to claim 1 or a salt thereof.

8. 1,1',5,5'-tetrahydro-4-hydroxy-2,2'-dioxo-[3,4'-bi-2H-pyrrole]-1,1'-diacetic acid di-N-(carbamoylmethyl)amide according to claim 1 or a salt thereof.

9. 1,1',5,5'-tetrahydro-4-hydroxy-2,2'-dioxo-[3,4'-bi-2H-pyrrole]-1,1'-diacetic acid di-N,N-[1,5-(2-methoxycarbonyl)pent-2-enylene]amide according to claim 1 or a salt thereof.

10. 1,1',5,5'-tetrahydro-4-hydroxy-2,2'-dioxo-[3,4'-bi-2H-pyrrole]-1,1'-di(3-propionic acid) diamide according to claim 1 or a salt thereof.

11. 1,1',5,5'-tetrahydro-4-hydroxy-2,2'-dioxo-[3,4'-bi-2H-pyrrole]-1,1'-diacetic acid diamide according to claim 1 or a salt thereof.

12. 1,1',5,5'-tetrahydro-4-hydroxy-2,2'-dioxo-[3,4'-bi-2H-pyrrole]-1,1'-diacetic acid di-N,(2-diisopropylaminoethyl) amide according to claim 1 or a salt thereof.

13. 1,1',5,5'-tetrahydro-4-hydroxy-2,2'-dioxo-[3,4'-bi-2H-pyrrole]-1,1'-diacetic acid according to claim 1 or a salt thereof.

14. 1,1',5,5'-tetrahydro-4-hydroxy-2,2'-dioxo-[3,4'-bi-2H-pyrrole]-1,1'-diacetic acid di-N-cyclohexylamide according to claim 1 or a salt therof.

15. 1,1',5,5'-tetrahydro-4-hydroxy-2,2'-dioxo-[3,4'-bi-2H-pyrrole]-1,1'-diacetic acid dimorpholide according to claim 1 or a salt thereof.

16. 1,1',5,5'-tetrahydro-4-hydroxy-2,2'-dioxo-[3,4'-bi-2H-pyrrole]-1,1'-diacetic acid di-N-ethylamide according to claim 1 or a salt thereof.

17. 1,1',5,5'-tetrahydro-4-hydroxy-2,2'-dioxo-[3,4'-bi-2H-pyrrole]-1,1'-diacetic acid di-N-[2-(3,4-dimethoxyphenyl)ethyl]amide according to claim 1 or a salt thereof.

18. 1,1',5,5'-tetrahydro-4-hydroxy-2,2'-dioxo-[3,4'-bi-2H-pyrrole]-1,1'-diacetic acid di-N-(2,2-dimethylpropyl) amide according to claim 1 or a salt thereof.

19. 1,1',5,5'-tetrahydro-4-hydroxy-2,2'-dioxo-[3,4'-bi-2H-pyrrole]-1,1'-diacetic acid di-N-(3-methoxypropyl)amide according to claim 1 or a salt thereof.

20. 1,1',5,5'-tetrahydro-4-hydroxy-2,2'-dioxo-[3,4'-bi-2H-pyrrole]-1,1'-diacetic acid di-N-methyl-N-(2-hydroxyethyl)-amide according to claim 1 or a salt thereof.

21. 1,1',5,5'-tetrahydro-4-hydroxy-2,2'-dioxo-[3,4'-bi-2H-pyrrole]-1,1'-di-(3-propionic acid) according to claim 1 or a salt thereof.

22. 1,1',5,5'-tetrahydro-4-hydroxy-2,2'-dioxo-[3,4'-bi-2H-pyrrole]-1,1'-diacetic acid di-N-adamantylamide according to claim 1 or a salt thereof.

23. 1,1',5,5'-tetrahydro-4-hydroxy-2,2'-dioxo-[3,4'-bi-2H-pyrrole]-1,1'-diacetic acid di(4-acetyl)-piperazide according to claim 1 or a salt thereof.

24. Octahydro-4-hydroxy-2,2'-dioxo-[3,4'-bi-2H-pyrrole]-1,1'-diacetic acid according to claim 1 or a salt thereof.

25. 1,1',5,5'-tetrahydro-4-hydroxy-2,2'-dioxo-[3,4'-bi-2H-pyrrole]-1,1'-diacetic acid di(4-methyl)piperazide according to claim 1 or a salt thereof.

26. Octahydro-4-hydroxy-2,2'-dioxo-[3,4'-bi-2H-pyrrole]-1,1'-diacetic acid diamide according to claim 1 or a salt thereof.

27. The sodium, potassium, ammonium, diethylammonium, bis(2-hydroxyethyl)ammonium, tris-[2-hydroxyethyl)ammonium, tris-(hydroxymethylammonium or N,N-dimethyl-N-(2-hydroxyethyl) ammonium salt of a compound according to one of the claims 1 to 26.

28. A compound according to any one of claims 1 to 27 in free form or in the form of a pharmaceutically acceptable salt for use in a method for the therapeutic treatment of the human or animal organism.

29. Pharmaceutical preparations containing a compound according to any one of claims 1 to 27 in free form or in the form of a pharmaceutically acceptable salt, together with customary pharmaceutical adjuncts.

30. A process for the preparation of novel tetrahydro-, hexahydro- and octahydro-[3,4'-bi-2H-pyrrole]-2,2'-diones according to claim 1 and salts thereof, characterised in that

a) a compound of the formula

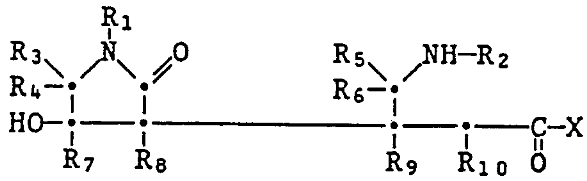

(II)

wherein X is optionally esterified or etherified hydroxy, or a salt thereof, is cyclised, or

b) in a compound of the formula

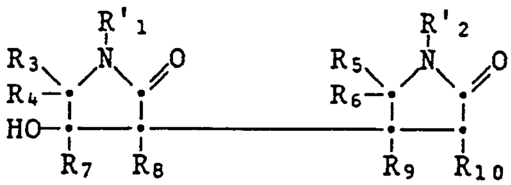

(III)

wherein $R'_1$ is a group that can be converted into a radical $R_1$ and $R'_2$ is a radical $R_2$ or a group that can be converted into the radical $R_2$, or in a salt thereof, $R'_1$ is converted into a group $R_1$ and, optionally, a radical $R'_2$ that can be converted into $R_2$ is converted into a group $R_2$, or

c) in a compound of the formula

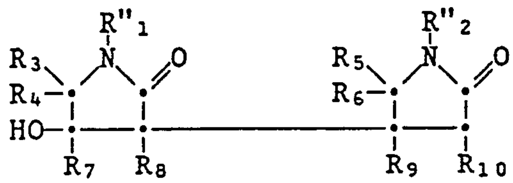

(IV)

wherein $R''_1$ is hydrogen and $R''_2$ is hydrogen or a radical $R_2$, or in a salt thereof, the hydrogen atoms $R''_1$ is replaced by a radical $R_1$ and, optionally, the hydrogen atoms $R''_2$ is replaced by a radical $R_2$, or

d) a compound of the formula

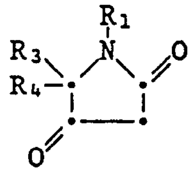

(V)

or a tautomer and/or salt thereof is condensed with a compound of the formula

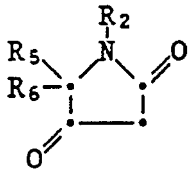

(VI)

or with a tautomer thereof, and, if desired, a compound obtainable in accordance with the process is converted into a different compound of formula I, an isomeric mixture obtainable in accordance with the process is separated into the isomers and/or a free compound obtainable in accordance with the process is converted into a salt or a salt obtainable in accordance with the process is converted into the free compound or into a different salt.

31. The use of a compound according to any one of claims 1 to 28 for the preparation of a nootropic.

**Claims for the Contracting State: AT**

1. A process for the preparation of novel substituted tetrahydro-, hexahydro- and octahydro-[3,4′-bi-2H-pyrrole]-2,2′-diones of the formula

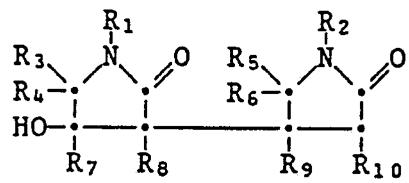

(I),

wherein each of $R_1$ and $R_2$ independently of the other is carboxy-$C_1$—$C_4$alkyl, carbamoyl-$C_1$—$C_4$alkyl, N-$C_1$—$C_7$- or N,N-di-$C_1$—$C_4$-alkylcarbamoyl-$C_1$—$C_4$alkyl, or one of the following radicals carrying the hydroxy, alkoxy, amino, alkylamino, dialkylamino, alkylene or alkenylene, or aza-, oxa- or thia-alkyleneamino group(s) in a position higher than the α-position with respect to the carbamoyl nitrogen atom: N-mono or N,N-di(hydroxy-$C_2$—$C_4$alkyl)carbamoyl-$C_1$—$C_4$alkyl, N-($C_1$—$C_4$alkoxy-$C_2$—$C_4$alkyl)carbamoyl-$C_1$—$C_4$alkyl, N-mono or N,N-di(amino-$C_2$—$C_4$alkyl)carbamoyl-$C_1$—$C_4$alkyl, N-($C_1$—$C_7$-alkylamino-$C_2$—$C_4$alkyl)- or N-(di-$C_1$—$C_4$alkylamino - $C_2$—$C_4$alkyl) - carbamoyl - $C_1$—$C_4$alkyl, N - (alkyleneamino - $C_2$—$C_4$alkyl)carbamoyl-$C_1$—$C_4$alkyl, N-(alkenyleneamino-$C_2$—$C_4$-alkyl)carbamoyl-$C_1$—$C_4$-alkyl, N-[(aza)alkyleneamino-$C_2$—$C_4$alkyl]-carbamoyl-$C_1$—$C_4$alkyl, N-[(oxa)-alkyleneamino-$C_2$—$C_4$alkyl]carbamoyl-$C_1$—$C_4$alkyl and N-[(thia)alkylene-amino-$C_2$—$C_4$alkyl]carbamoyl-$C_1$—$C_4$alkyl, wherein the alkyleneamino, alkenyleneamino-, (aza)alkyleneamino, (oxa)alkyleneamino or (thia)alkyleneamino radical contains, including the amino group from 4 to 8 ring members and alkyleneamino may be C-substituted by $C_1$—$C_4$alkoxycarbonyl and by oxo or hydroxy, alkenyleneamino may be C-substituted by $C_1$—$C_4$alkoxycarbonyl and optionally additionally by hydroxy, and (aza)alkyleneamino may be C- or N′-substituted by $C_1$—$C_4$alkyl and/or N′substituted by $C_1$—$C_7$alkanoyl; or is N-(carbamoyl-$C_1$—$C_4$alkyl)carbamoyl-$C_1$—$C_4$alkyl, N-(N′-$C_1$—$C_7$- or N′,N′-di-$C_1$—$C_4$-alkylcarbamoyl-$C_2$—$C_4$alkyl)carbamoyl-$C_1$—$C_4$alkyl, 3-N-$C_3$—$C_8$cycloalkyl- or N,N-Di-$C_3$—$C_8$cycloalkylcarbamoyl-$C_1$—$C_4$alkyl, N-$C_7$—$C_9$bicycloalkylcarbamoyl-$C_1$—$C_4$alkyl, N-$C_8$—$C_{12}$tricycloalkylcarbamoyl-$C_1$—$C_4$alkyl, N-mono or N,N-di-(phenyl-$C_1$—$C_4$alkyl)carbamoyl-$C_1$—$C_4$alkyl which is unsubstituted or substituted in the phenyl moiety by $C_1$—$C_4$alkyl, $C_1$—$C_4$alkoxy, halogen and/or by trifluoromethyl, or is N,N-alkylene-carbamoyl-$C_1$—$C_4$alkyl, N,N-alkenylenecarbamoyl-$C_1$—$C_4$alkyl, N,N-(aza)alkylenecarbamoyl-$C_1$—$C_4$alkyl or N,N-(oxa or thia-)alkylenecarbamoyl-$C_1$—$C_4$alkyl, wherein the alkyleneamino, alkenyleneamino, (aza)alkyleneamino, (oxa)alkyleneamino or (thia)alkyleneamino radical contains, including the amino group, from 4 to 8 ring members and alkyleneamino may be C-substituted by $C_1$—$C_4$alkoxycarbonyl and by oxo or hydroxy, and alkenyleneamino may be C-substituted by $C_1$—$C_4$alkoxycarbonyl and optionally additionally by hydroxy, and (aza)alkyleneamino may be C- or N′-substituted by $C_1$—$C_4$alkyl and/or N′-substituted by $C_1$—$C_7$alkanoyl, and each of $R_3$, $R_4$, $R_5$ and $R_6$ is hydrogen or $C_1$—$C_7$alkyl, or $R_3$ and $R_4$ and/or $R_5$ and $R_6$ taken together are a $C_4$—$C_8$alkylene radical, and each of $R_7$, $R_8$, $R_9$ and $R_{10}$ is hydrogen, or $R_7$ together with $R_8$ and/or $R_9$ together with $R_{10}$ are in each case an additional bond, and their salts, characterised in that

a) a compound of the formula

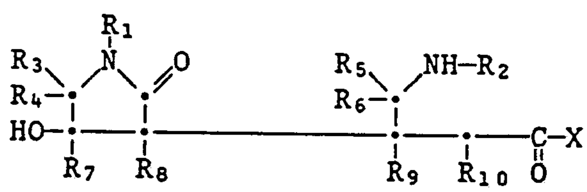

(II)

wherein X is optionally esterified or etherified hydroxy, or a salt thereof, is cyclised, or

b) in a compound of the formula

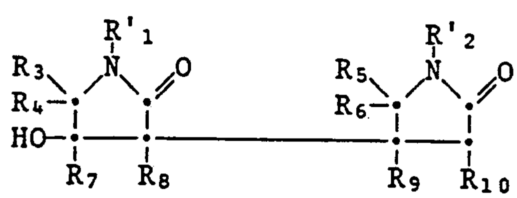

(III)

wherein $R'_1$ is a group that can be converted into a radical $R_1$ and $R'_2$ is a radical $R_2$ or a group that can be converted into the radical $R_2$, or in a salt thereof, $R'_1$ is converted into a group $R_1$ and, optionally, a radical $R'_2$ that can be converted into $R_2$ is converted into a group $R_2$, or

c) in a compound of the formula

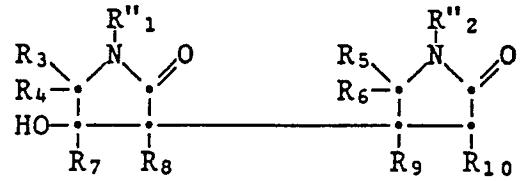

(IV)

wherein $R''_1$ is hydrogen and $R''_2$ is hydrogen or a radical $R_2$, or in a salt thereof, the hydrogen atoms $R''_1$ is replaced by a radical $R_1$ and, optionally, the hydrogen atom $R''_2$ is replaced by a radical $R_2$, or

d) a compound of the formula

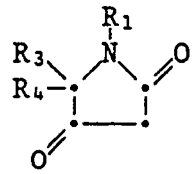

(V)

or a tautomer and/or salt thereof is condensed with a compound of the formula

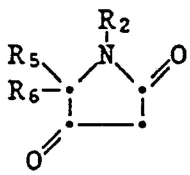

(VI)

or with a tautomer thereof, and, if desired, a compound obtainable in accordance with the process is converted into a different compound of formula I, an isomeric mixture obtainable in accordance with the process is separated into the isomers and/or a free compound obtainable in accordance with the process is converted into a salt or a salt obtainable in accordance with the process is converted into the free compound or into a different salt.

2. A process according to claim 1 for the preparation of compounds of formula I wherein each of $R_1$ and $R_2$ independently of the other is carboxy-$C_1$—$C_4$alkyl, carbamoyl-$C_1$—$C_4$alkyl, N-mono- or N,N-di-$C_1$—$C_4$-alkylcarbamoyl-$C_1$—$C_4$alkyl, N-mono- or N,N-di-(ω-hydroxy-$C_2$—$C_4$alkyl)carbamoyl-$C_1$—$C_4$alkyl, or N,N-(ω-amino-$C_2$—$C_4$alkyl)carbamoyl-$C_1$—$C_4$alkyl, N-[ω-(N',N'-Di-$C_1$—$C_4$alkylamino)-$C_2$—$C_4$-alkyl]carbamoyl-$C_1$—$C_4$alkyl, N-(ω-alkyleneamino-$C_2$—$C_4$alkyl)carbamoyl-$C_1$—$C_4$alkyl having from 4 to 7 ring members, N-[ω - (3 - azaalkylenamino) - $C_2$—$C_4$alkyl]carbamoyl-$C_1$—$C_4$alkyl N-[ω-(3-oxaalkyleneamino)-$C_2$—$C_4$alkyl]-carbamoyl-$C_1$—$C_4$alkyl having from 4 to 7 ring members, N-(phenyl-$C_1$—$C_4$alkyl)carbamoyl-$C_1$—$C_4$alkyl, wherein the phenyl moiety may be mono- or di-substituted by $C_1$—$C_4$alkyl, $C_1$—$C_4$alkoxy, halogen having an atomic number of up to and including 35 and/or by trifluormethyl N-(α-carbamyl-$C_1$—$C_4$alkyl)carbamoyl-$C_1$—$C_4$alkyl, N,N-alkylenecarbamoyl-$C_1$—$C_4$alkyl having from 4 to 7 ring members, N,N-(aza-alkylene)carbamoyl-$C_1$—$C_4$alkyl having from 4 to 7 ring members, N,N-(3-oxaalkylene)carbamoyl-$C_1$—$C_4$alkyl having 4 to 7 ring members or N,N-(3-thiaalkylene)carbamoyl-$C_1$—$C_4$alkyl having from 4 to 7 members, $R_3$, $R_4$, $R_5$ and $R_6$ are hydrogen or $R_3$ and $R_5$ are identical $C_1$—$C_4$alkyl radicals and $R_4$ and $R_6$ are hydrogen or identical $C_1$—$C_4$alkyl radicals, or $R_3$ and $R_4$ and also $R_5$ and $R_6$ taken together are in each case identical $C_2$—$C_6$alkylene radicals, and $R_7$ together with $R_8$ and/or $R_9$ together with $R_{10}$ are in each case an additional bond, and their salts.

3. A process according to claim 1 for the preparation of compounds of formula I wherein $R_1$ and $R_2$ are identical and are carboxy-$C_1$—$C_4$alkyl, carbamoyl-$C_1$—$C_4$alkyl, N-mono-$C_1$—$C_7$- or N,N-di-$C_1$—$C_4$-alkylcarbamoyl-$C_1$—$C_4$alkyl, N-(ω-di-$C_1$—$C_4$alkylamino-$C_2$—$C_4$-alkyl)carbamoyl-$C_1$—$C_4$alkyl, N-(ω-hydroxy-$C_2$—$C_4$alkyl)carbamoyl - $C_1$—$C_4$alkyl, N - (ω - $C_1$—$C_4$ - alkoxy - $C_2$—$C_4$alkyl)carbamoyl - $C_1$—$C_4$alkyl, N-$C_5$—$C_6$cycloalkyl-, N-$C_8$—$C_9$bicycloalkyl- or N-$C_8$—$C_{10}$-tricycloalkylcarbamoyl-$C_1$—$C_4$alkyl, 5- or 6-membered N,N-alkyleneaminocarbonoyl-$C_1$—$C_4$alkyl optionally 3-substituted by $C_1$—$C_4$alkoxycarbonyl, and 4-substituted by oxo or hydroxy, 5- or 6-membered alkenyleneaminocarbonyl-$C_1$—$C_4$alkyl 3-substituted by $C_1$—$C_4$alkoxycarbonyl and optionally additionally 4-substituted by hydroxy, 5- or 6-membered N,N-(aza)alkylenaminocarbonyl-$C_1$—$C_4$alkyl optionally N'-substituted by $C_1$—$C_4$ alkyl or by $C_1$—$C_4$alkanoyl, 5- or 6-membered N,N-(oxa)alkyleneaminocarbonyl-$C_1$—$C_4$-alkyl, or N-phenyl-$C_1$—$C_4$alkylcarbamoyl-$C_1$—$C_4$alkyl optionally mono- or di-substituted in the phenyl moiety by $C_1$—$C_4$alkoxy, $R_3$, $R_4$, $R_5$ and $R_6$ are hydrogen, and each of $R_7$, $R_8$, $R_9$ and $R_{10}$ is hydrogen, or $R_7$ together with $R_8$ and also $R_9$ together with $R_{10}$ are in each case an additional bond, and salts thereof.

4. A process according to claim 1 for the preparation of compounds of formula I wherein $R_1$ and $R_2$ are identical carboxy-$C_1$—$C_4$alkyl or carbamoyl-$C_1$—$C_4$alkyl radicals, or carbamoylmethyl radicals N-monosubstituted by ω-N,N-di-$C_1$—$C_4$alkylamino-$C_2$—$C_4$alkyl N-mono- or N,N-di-substituted by ω-hydroxy-$C_2$—$C_4$alkyl, or N,N-disubstituted by 4- to 6-membered (3-$C_1$—$C_4$alkyl-3-aza)alkylene, and $R_3$, $R_4$, $R_5$ and $R_6$ are hydrogen, and salts thereof.

5. A process according to claim 1 for the preparation of compounds obtainable in accordance with claim 1 or 2, wherein $R_1$ and $R_2$ are identical, $R_3$, $R_4$, $R_5$ and $R_6$ are hydrogen, and $R_7$ together with $R_8$ and $R_9$ together with $R_{10}$ are in each case an additional bond.

6. A process according to claim 1 for the preparation of 1,1′,5,5′-tetrahydro-4-hydroxy-2,2′-dioxo-[3,4′-bi-2H-pyrrole]-1,1′-diacetic acid diamide or a salt thereof.

7. A process according to claim 1 for the preparation of 1,1′,5,5′-tetrahydro-4-hydroxy-2,2′-dioxo-[3,4′-bi-2H-pyrrole]-1,1′-diacetic acid di-N-(2-phenylethyl)amide or a salt thereof.

8. A process according to claim 1 for the preparation of 1,1′,5,5′-tetrahydro-4-hydroxy-2,2′-dioxo-[3,4′-bi-2H-pyrrole]-1,1′-diacetic acid di-N-(carbamoylmethyl)amide or a salt thereof.

9. A process according to claim 1 for the preparation of 1,1′,5,5′-tetrahydro-4-hydroxy-2,2′-dioxo-[3,4′-bi-2H-pyrrole]-1,1′-diacetic acid di-N,N-[1,5-(2-methoxycarbonyl)pent-2-enylene]amide or a salt thereof.

10. A process according to claim 1 for the preparation of 1,1′,5,5′-tetrahydro-4-hydroxy-2,2′-dioxo-[3,4′-bi-2H-pyrrole]-1,1′-di(3-propionic acid) diamide or a salt thereof.

11. A process according to claim 1 for the preparation of 1,1′,5,5′-tetrahydro-4-hydroxy-2,2′-dioxo-[3,4′-bi-2H-pyrrole]-1,1′-diacetic acid or a salt thereof.

12. A process according to claim 1 for the preparation of 1,1′,5,5′-tetrahydro-4-hydroxy-2,2′-dioxo-[3,4′-bi-2H-pyrrole]-1,1′-diacetic acid di-N,(2-diisopropylaminoethyl)amide or a salt thereof.

13. A process according to claim 1 for the preparation of 1,1′,5,5′-tetrahydro-4-hydroxy-2,2′-dioxo-[3,4′-bi-2H-pyrrole]-1,1′-diacetic acid dipiperidide or a salt thereof.

14. A process according to claim 1 for the preparation of 1,1′,5,5′-tetrahydro-4-hydroxy-2,2′-dioxo-[3,4′-bi-2H-pyrrole]-1,1′-diacetic acid di-N-cyclohexylamide or a salt thereof.

15. A process according to claim 1 for the preparation of 1,1′,5,5′-tetrahydro-4-hydroxy-2,2′-dioxo-[3,4′-bi-2H-pyrrole]-1,1′-diacetic acid dimorpholide or a salt thereof.

16. A process according to claim 1 for the preparation of 1,1′,5,5′-tetrahydro-4-hydroxy-2,2′-dioxo-[3,4′-bi-2H-pyrrole]-1,1′-diacetic acid di-N-ethylamide or a salt thereof.

17. A process according to claim 1 for the preparation of 1,1′,5,5′-tetrahydro-4-hydroxy-2,2′-dioxo-[3,4′-bi-2H-pyrrole]-1,1′-diacetic acid di-N-[2-(3,4-dimethoxyphenyl)ethyl]amide or a salt thereof.

18. A process according to claim 1 for the preparation of 1,1′,5,5′-tetrahydro-4-hydroxy-2,2′-dioxo-[3,4′-bi-2H-pyrrole]-1,1′-diacetic acid di-N-(2,2-dimethylpropyl) amide or a salt thereof.

19. A process according to claim 1 for the preparation of 1,1′,5,5′-tetrahydro-4-hydroxy-2,2′-dioxo-[3,4′-bi-2H-pyrrol]-1,1′-diacetic acid di-N-(3-methoxypropyl)amide or a salt thereof.

20. A process according to claim 1 for the preparation of 1,1′,5,5′-tetrahydro-4-hydroxy-2,2′-dioxo-[3,4′-bi-2H-pyrrole]-1,1′-diacetic acid di-N-methyl-N-(2-hydroxyethyl)-amide or a salt thereof.

21. A process according to claim 1 for the preparation of 1,1′,5,5′-tetrahydro-4-hydroxy-2,2′-dioxo-[3,4′-bi-2H-pyrrole]-1,1′-di-(3-propionic acid) or a salt thereof.

22. A process according to claim 1 for the preparation of 1,1′,5,5′-tetrahydro-4-hydroxy-2,2′-dioxo-[3,4′-bi-2H-pyrrole]-1,1′-diacetic acid di-N-adamantylamide or a salt thereof.

23. A process according to claim 1 for the preparation of 1,1′,5,5′-tetrahydro-4-hydroxy-2,2′-dioxo-[3,4′-bi-2H-pyrrole]-1,1′-diacetic acid di(4-acetyl)piperazide or a salt thereof.

24. A process according to claim 1 for the preparation of octahydro-4-hydroxy-2,2′-dioxo[3,4′-bi-2H-pyrrole]-1,1′-diacetic acid or a salt thereof.

25. A process according to claim 1 for the preparation of 1,1′,5,5′-tetrahydro-4-hydroxy-2,2′-dioxo-[3,4′-bi-2H-pyrrole]-1,1′-diacetic acid di(4-methyl)piperazide or a salt thereof.

26. A process according to claim 1 for the preparation of octahydro-4-hydroxy-2,2′-dioxo[3,4′-bi-2H-pyrrole]-1,1′-diacetic acid diamide or a salt thereof.

27. A process according to claim 1 for the preparation of compounds obtainable in accordance with any one of claims 1 to 26 in the form of the sodium, potassium, ammonium, diethylammonium, bis-(2-hydroxyethyl)ammonium, tris-[2-hydroxyethyl)ammonium, tris-(hydroxymethyl)methylammonium or N,N-dimethyl-N-(2-hydroxyethyl)ammonium salt.

28. A process for the preparation of pharmaceutical preparations, characterised in that a compound obtainable in accordance with any one of claims 1 to 27 in free form or in the form of a pharmaceutically acceptable salt is mixed with customary pharmaceutical adjuncts.